# EUROPÄISCHE PATENTANMELDUNG

(11) **EP 2 628 792 A1**
(43) Veröffentlichungstag der Anmeldung: **21.08.2013**
(21) Anmeldenummer: 12156052.8
(22) Anmeldetag: 17.02.2012
(51) Int. Cl.: C12N 1/20, C12P 13/12, C12P 13/22

(54) **Zelle mit verringerter ppGppase-Aktivität**

(71) Anmelder: Evonik Industries AG, 45128 Essen (DE)
(72) Erfinder: Schneider, Frank, 33790 Halle (DE); Gerth, Caroline, 33813 Oerlinghausen (DE)

(57) **Zusammenfassung**

Die vorliegende Erfindung betrifft eine Zelle, die ihrem Wildtyp gegenüber derartig genetisch verändert ist, dass sie eine relativ zu ihrem Wildtyp verringerte ppGppase-Aktvität aufweist und bevorzugt eine essentielle Aminosäure, noch bevorzugter eine von Serin abgeleitete essentielle Aminosäure, am bevorzugtesten Methionin oder Tryptophan, ein Futtermitteladditiv umfassend eine solche Zelle, ein Verfahren zur Herstellung einer essentiellen Aminosäure, bevorzugter einer von Serin abgeleiteten essentiellen Aminosäure, am bevorzugtesten Methionin oder Tryptophan, überproduzierenden Zelle, umfassend Herstellen einer Zelle mit einem Knock out eines für eine ppGppase kodierenden Gens, sowie ein Verfahren zur Herstellung einer essentiellen Aminosäure, bevorzugter einer von Serin abgeleiteten essentiellen Aminosäure, am bevorzugtesten Methionin oder Tryptophan, umfassend die Schritte a) Kultivieren der Zelle nach dem ersten Aspekt der vorliegenden Erfindung oder einer seiner Ausführungsformen, und b) optional: Aufreinigen der Aminosäure.

SEQUENCE LISTING

<110>  Evonik Industries AG

<120>  Zelle mit verringerter ppGppase-Aktivität


<130>  201100331

<160>  19

<170>  PatentIn version 3.5

<210>  1
<211>  2109
<212>  DNA
<213>  Escherichia coli str. K12 substr. MG1655


<220>
<221>  CDS
<222>  (1)..(2106)
<223>  spoT(b3650, ECK3640)

       Accession NP_418107; bifunctional (p)ppGpp synthetase II/
       guanosine-3',5'-bis pyrophosphate 3'-pyrophosphohydrolase
       [Escherichia coli str. K-12 substr. MG1655].

<400>  1
ttg tat ctg ttt gaa agc ctg aat caa ctg att caa acc tac ctg ccg        48
Leu Tyr Leu Phe Glu Ser Leu Asn Gln Leu Ile Gln Thr Tyr Leu Pro
1               5                   10                  15

gaa gac caa atc aag cgt ctg cgg cag gcg tat ctc gtt gca cgt gat        96
Glu Asp Gln Ile Lys Arg Leu Arg Gln Ala Tyr Leu Val Ala Arg Asp
            20                  25                  30

gct cac gag ggg caa aca cgt tca agc ggt gaa ccc tat atc acg cac        144
Ala His Glu Gly Gln Thr Arg Ser Ser Gly Glu Pro Tyr Ile Thr His
        35                  40                  45

ccg gta gcg gtt gcc tgc att ctg gcc gag atg aaa ctc gac tat gaa        192
Pro Val Ala Val Ala Cys Ile Leu Ala Glu Met Lys Leu Asp Tyr Glu
    50                  55                  60

acg ctg atg gcg gcg ctg ctg cat gac gtg att gaa gat act ccc gcc        240
Thr Leu Met Ala Ala Leu Leu His Asp Val Ile Glu Asp Thr Pro Ala
65                  70                  75                  80

acc tac cag gat atg gaa cag ctt ttt ggt aaa agc gtc gcc gag ctg        288
Thr Tyr Gln Asp Met Glu Gln Leu Phe Gly Lys Ser Val Ala Glu Leu
                85                  90                  95

gta gag ggg gtg tcg aaa ctt gat aaa ctc aag ttc cgc gat aag aaa        336
Val Glu Gly Val Ser Lys Leu Asp Lys Leu Lys Phe Arg Asp Lys Lys
            100                 105                 110

gag gcg cag gcc gaa aac ttt cgc aag atg att atg gcg atg gtg cag        384
Glu Ala Gln Ala Glu Asn Phe Arg Lys Met Ile Met Ala Met Val Gln
        115                 120                 125

gat atc cgc gtc atc ctc atc aaa ctt gcc gac cgt acc cac aac atg        432
Asp Ile Arg Val Ile Leu Ile Lys Leu Ala Asp Arg Thr His Asn Met
    130                 135                 140

cgc acg ctg ggc tca ctt cgc ccg gac aaa cgt cgc cgc atc gcc cgt 480
Arg Thr Leu Gly Ser Leu Arg Pro Asp Lys Arg Arg Arg Ile Ala Arg
145                 150                 155                 160

gaa act ctc gaa att tat agc ccg ctg gcg cac cgt tta ggt atc cac 528
Glu Thr Leu Glu Ile Tyr Ser Pro Leu Ala His Arg Leu Gly Ile His
                165                 170                 175

cac att aaa acc gaa ctc gaa gag ctg ggt ttt gag gcg ctg tat ccc 576
His Ile Lys Thr Glu Leu Glu Glu Leu Gly Phe Glu Ala Leu Tyr Pro
            180                 185                 190

aac cgt tat cgc gta atc aaa gaa gtg gtg aaa gcc gcg cgc ggc aac 624
Asn Arg Tyr Arg Val Ile Lys Glu Val Val Lys Ala Ala Arg Gly Asn
        195                 200                 205

cgt aaa gag atg atc cag aag att ctt tct gaa atc gaa ggg cgt ttg 672
Arg Lys Glu Met Ile Gln Lys Ile Leu Ser Glu Ile Glu Gly Arg Leu
    210                 215                 220

cag gaa gcg gga ata ccg tgc cgc gtc agt ggt cgc gag aag cat ctt 720
Gln Glu Ala Gly Ile Pro Cys Arg Val Ser Gly Arg Glu Lys His Leu
225                 230                 235                 240

tat tcg att tac tgc aaa atg gtg ctc aaa gag cag cgt ttt cac tcg 768
Tyr Ser Ile Tyr Cys Lys Met Val Leu Lys Glu Gln Arg Phe His Ser
                245                 250                 255

atc atg gac atc tac gct ttc cgc gtg atc gtc aat gat tct gac acc 816
Ile Met Asp Ile Tyr Ala Phe Arg Val Ile Val Asn Asp Ser Asp Thr
            260                 265                 270

tgt tat cgc gtg ctg ggc cag atg cac agc ctg tac aag ccg cgt ccg 864
Cys Tyr Arg Val Leu Gly Gln Met His Ser Leu Tyr Lys Pro Arg Pro
        275                 280                 285

ggc cgc gtg aaa gac tat atc gcc att cca aaa gcg aac ggc tat cag 912
Gly Arg Val Lys Asp Tyr Ile Ala Ile Pro Lys Ala Asn Gly Tyr Gln
    290                 295                 300

tct ttg cac acc tcg atg atc ggc ccg cac ggt gtg ccg gtt gag gtc 960
Ser Leu His Thr Ser Met Ile Gly Pro His Gly Val Pro Val Glu Val
305                 310                 315                 320

cag atc cgt acc gaa gat atg gac cag atg gcg gag atg ggt gtt gcc 1008
Gln Ile Arg Thr Glu Asp Met Asp Gln Met Ala Glu Met Gly Val Ala
                325                 330                 335

gcg cac tgg gct tat aaa gag cac ggc gaa acc agt act acc gca caa 1056
Ala His Trp Ala Tyr Lys Glu His Gly Glu Thr Ser Thr Thr Ala Gln
            340                 345                 350

atc cgc gcc cag cgc tgg atg caa agc ctg ctg gag ctg caa cag agc 1104
Ile Arg Ala Gln Arg Trp Met Gln Ser Leu Leu Glu Leu Gln Gln Ser
        355                 360                 365

gcc ggt agt tcg ttt gaa ttt atc gag agc gtt aaa tcc gat ctc ttc 1152
Ala Gly Ser Ser Phe Glu Phe Ile Glu Ser Val Lys Ser Asp Leu Phe
    370                 375                 380

ccg gat gag att tac gtt ttc aca ccg gaa ggg cgc att gtc gag ctg 1200
Pro Asp Glu Ile Tyr Val Phe Thr Pro Glu Gly Arg Ile Val Glu Leu
385                 390                 395                 400

cct gcc ggt gca acg ccc gtc gac ttc gct tat gca gtg cat acc gat 1248

Pro Ala Gly Ala Thr Pro Val Asp Phe Ala Tyr Ala Val His Thr Asp
                405                 410                 415

atc ggt cat gcc tgc gtg ggc gca cgc gtt gac cgc cag cct tac ccg 1296
Ile Gly His Ala Cys Val Gly Ala Arg Val Asp Arg Gln Pro Tyr Pro
            420                 425                 430

ctg tcg cag ccg ctt acc agc ggt caa acc gtt gaa atc att acc gct 1344
Leu Ser Gln Pro Leu Thr Ser Gly Gln Thr Val Glu Ile Ile Thr Ala
        435                 440                 445

ccg ggc gct cgc ccg aat gcc gct tgg ctg aac ttt gtc gtt agc tcg 1392
Pro Gly Ala Arg Pro Asn Ala Ala Trp Leu Asn Phe Val Val Ser Ser
    450                 455                 460

aaa gcg cgc gcc aaa att cgt cag ttg ctg aaa aac ctc aag cgt gat 1440
Lys Ala Arg Ala Lys Ile Arg Gln Leu Leu Lys Asn Leu Lys Arg Asp
465                 470                 475                 480

gat tct gta agc ctg ggc cgt cgt ctg ctc aac cat gct ttg ggt ggt 1488
Asp Ser Val Ser Leu Gly Arg Arg Leu Leu Asn His Ala Leu Gly Gly
                485                 490                 495

agc cgt aag ctg aat gaa atc ccg cag gaa aat att cag cgc gag ctg 1536
Ser Arg Lys Leu Asn Glu Ile Pro Gln Glu Asn Ile Gln Arg Glu Leu
            500                 505                 510

gat cgc atg aag ctg gca acg ctt gac gat ctg ctg gca gaa atc gga 1584
Asp Arg Met Lys Leu Ala Thr Leu Asp Asp Leu Leu Ala Glu Ile Gly
        515                 520                 525

ctt ggt aac gca atg agc gtg gtg gtc gcg aaa aat ctg caa cat ggg 1632
Leu Gly Asn Ala Met Ser Val Val Val Ala Lys Asn Leu Gln His Gly
    530                 535                 540

gac gcc tcc att cca ccg gca acc caa agc cac gga cat ctg ccc att 1680
Asp Ala Ser Ile Pro Pro Ala Thr Gln Ser His Gly His Leu Pro Ile
545                 550                 555                 560

aaa ggt gcc gat ggc gtg ctg atc acc ttt gcg aaa tgc tgc cgc cct 1728
Lys Gly Ala Asp Gly Val Leu Ile Thr Phe Ala Lys Cys Cys Arg Pro
                565                 570                 575

att cct ggc gac ccg att atc gcc cac gtc agc ccc ggt aaa ggt ctg 1776
Ile Pro Gly Asp Pro Ile Ile Ala His Val Ser Pro Gly Lys Gly Leu
            580                 585                 590

gtg atc cac cat gaa tcc tgc cgt aat atc cgt ggc tac cag aaa gag 1824
Val Ile His His Glu Ser Cys Arg Asn Ile Arg Gly Tyr Gln Lys Glu
        595                 600                 605

cca gag aag ttt atg gct gtg gaa tgg gat aaa gag acg gcg cag gag 1872
Pro Glu Lys Phe Met Ala Val Glu Trp Asp Lys Glu Thr Ala Gln Glu
    610                 615                 620

ttc atc acc gaa atc aag gtg gag atg ttc aat cat cag ggt gcg ctg 1920
Phe Ile Thr Glu Ile Lys Val Glu Met Phe Asn His Gln Gly Ala Leu
625                 630                 635                 640

gca aac ctg acg gcg gca att aac acc acg act tcg aat att caa agt 1968
Ala Asn Leu Thr Ala Ala Ile Asn Thr Thr Thr Ser Asn Ile Gln Ser
                645                 650                 655

ttg aat acg gaa gag aaa gat ggt cgc gtc tac agc gcc ttt att cgt 2016
Leu Asn Thr Glu Glu Lys Asp Gly Arg Val Tyr Ser Ala Phe Ile Arg

660                 665                 670

ctg acc gct cgt gac cgt gtg cat ctg gcg aat atc atg cgc aaa atc        2064
Leu Thr Ala Arg Asp Arg Val His Leu Ala Asn Ile Met Arg Lys Ile
        675                 680                 685

cgc gtg atg cca gac gtg att aaa gtc acc cga aac cga aat taa            2109
Arg Val Met Pro Asp Val Ile Lys Val Thr Arg Asn Arg Asn
    690                 695                 700

<210>  2
<211>  702
<212>  PRT
<213>  Escherichia coli str. K12 substr. MG1655

<400>  2

Leu Tyr Leu Phe Glu Ser Leu Asn Gln Leu Ile Gln Thr Tyr Leu Pro
1               5                   10                  15

Glu Asp Gln Ile Lys Arg Leu Arg Gln Ala Tyr Leu Val Ala Arg Asp
            20                  25                  30

Ala His Glu Gly Gln Thr Arg Ser Ser Gly Glu Pro Tyr Ile Thr His
        35                  40                  45

Pro Val Ala Val Ala Cys Ile Leu Ala Glu Met Lys Leu Asp Tyr Glu
    50                  55                  60

Thr Leu Met Ala Ala Leu Leu His Asp Val Ile Glu Asp Thr Pro Ala
65                  70                  75                  80

Thr Tyr Gln Asp Met Glu Gln Leu Phe Gly Lys Ser Val Ala Glu Leu
                85                  90                  95

Val Glu Gly Val Ser Lys Leu Asp Lys Leu Lys Phe Arg Asp Lys Lys
            100                 105                 110

Glu Ala Gln Ala Glu Asn Phe Arg Lys Met Ile Met Ala Met Val Gln
        115                 120                 125

Asp Ile Arg Val Ile Leu Ile Lys Leu Ala Asp Arg Thr His Asn Met
    130                 135                 140

Arg Thr Leu Gly Ser Leu Arg Pro Asp Lys Arg Arg Arg Ile Ala Arg
145                 150                 155                 160

Glu Thr Leu Glu Ile Tyr Ser Pro Leu Ala His Arg Leu Gly Ile His
                165                 170                 175

His Ile Lys Thr Glu Leu Glu Glu Leu Gly Phe Glu Ala Leu Tyr Pro
            180                 185                 190

Asn Arg Tyr Arg Val Ile Lys Glu Val Val Lys Ala Ala Arg Gly Asn
        195                 200                 205

Arg Lys Glu Met Ile Gln Lys Ile Leu Ser Glu Ile Glu Gly Arg Leu
    210                 215                 220

Gln Glu Ala Gly Ile Pro Cys Arg Val Ser Gly Arg Glu Lys His Leu
225                 230                 235                 240

Tyr Ser Ile Tyr Cys Lys Met Val Leu Lys Glu Gln Arg Phe His Ser
                245                 250                 255

Ile Met Asp Ile Tyr Ala Phe Arg Val Ile Val Asn Asp Ser Asp Thr
            260                 265                 270

Cys Tyr Arg Val Leu Gly Gln Met His Ser Leu Tyr Lys Pro Arg Pro
        275                 280                 285

Gly Arg Val Lys Asp Tyr Ile Ala Ile Pro Lys Ala Asn Gly Tyr Gln
    290                 295                 300

Ser Leu His Thr Ser Met Ile Gly Pro His Gly Val Pro Val Glu Val
305                 310                 315                 320

Gln Ile Arg Thr Glu Asp Met Asp Gln Met Ala Glu Met Gly Val Ala
                325                 330                 335

Ala His Trp Ala Tyr Lys Glu His Gly Glu Thr Ser Thr Thr Ala Gln
            340                 345                 350

Ile Arg Ala Gln Arg Trp Met Gln Ser Leu Leu Glu Leu Gln Gln Ser
        355                 360                 365

Ala Gly Ser Ser Phe Glu Phe Ile Glu Ser Val Lys Ser Asp Leu Phe
    370                 375                 380

Pro Asp Glu Ile Tyr Val Phe Thr Pro Glu Gly Arg Ile Val Glu Leu
385                 390                 395                 400

Pro Ala Gly Ala Thr Pro Val Asp Phe Ala Tyr Ala Val His Thr Asp
                405                 410                 415

Ile Gly His Ala Cys Val Gly Ala Arg Val Asp Arg Gln Pro Tyr Pro
            420                 425                 430

Leu Ser Gln Pro Leu Thr Ser Gly Gln Thr Val Glu Ile Ile Thr Ala
        435                 440                 445

Pro Gly Ala Arg Pro Asn Ala Ala Trp Leu Asn Phe Val Val Ser Ser
450                 455                 460

Lys Ala Arg Ala Lys Ile Arg Gln Leu Leu Lys Asn Leu Lys Arg Asp
465                 470                 475                 480

Asp Ser Val Ser Leu Gly Arg Arg Leu Leu Asn His Ala Leu Gly Gly
                485                 490                 495

Ser Arg Lys Leu Asn Glu Ile Pro Gln Glu Asn Ile Gln Arg Glu Leu
            500                 505                 510

Asp Arg Met Lys Leu Ala Thr Leu Asp Asp Leu Leu Ala Glu Ile Gly
        515                 520                 525

Leu Gly Asn Ala Met Ser Val Val Val Ala Lys Asn Leu Gln His Gly
    530                 535                 540

Asp Ala Ser Ile Pro Pro Ala Thr Gln Ser His Gly His Leu Pro Ile
545                 550                 555                 560

Lys Gly Ala Asp Gly Val Leu Ile Thr Phe Ala Lys Cys Cys Arg Pro
                565                 570                 575

Ile Pro Gly Asp Pro Ile Ile Ala His Val Ser Pro Gly Lys Gly Leu
            580                 585                 590

Val Ile His His Glu Ser Cys Arg Asn Ile Arg Gly Tyr Gln Lys Glu
        595                 600                 605

Pro Glu Lys Phe Met Ala Val Glu Trp Asp Lys Glu Thr Ala Gln Glu
    610                 615                 620

Phe Ile Thr Glu Ile Lys Val Glu Met Phe Asn His Gln Gly Ala Leu
625                 630                 635                 640

Ala Asn Leu Thr Ala Ala Ile Asn Thr Thr Thr Ser Asn Ile Gln Ser
                645                 650                 655

Leu Asn Thr Glu Glu Lys Asp Gly Arg Val Tyr Ser Ala Phe Ile Arg
            660                 665                 670

Leu Thr Ala Arg Asp Arg Val His Leu Ala Asn Ile Met Arg Lys Ile
        675                 680                 685

Arg Val Met Pro Asp Val Ile Lys Val Thr Arg Asn Arg Asn
    690                 695                 700

<210>  3

<211>  2112
<212>  DNA
<213>  Salmonella enterica subsp. enterica serovar Typhimurium str. LT2

<220>
<221>  CDS
<222>  (1)..(2109)
<223>  spoT_St (Accession  NC_003197 REGION: 3934070..3936181; locus
       tag: STM3742)

<400>  3
ttg tat ctg ttt gaa agc ctg aat caa ctg att caa acc tac ctg ccg        48
Leu Tyr Leu Phe Glu Ser Leu Asn Gln Leu Ile Gln Thr Tyr Leu Pro
1               5                   10                  15

gaa gac cag att aag cgt ctt cgg cag gcg tat ctc gtt gca cgt gac        96
Glu Asp Gln Ile Lys Arg Leu Arg Gln Ala Tyr Leu Val Ala Arg Asp
            20                  25                  30

gct cac gag ggc cag aca cgt tca agc ggt gaa ccc tat atc acg cac       144
Ala His Glu Gly Gln Thr Arg Ser Ser Gly Glu Pro Tyr Ile Thr His
        35                  40                  45

ccg gtg gcg gtg gcc tgt att ctg gcc gag atg aaa ctc gac tac gaa       192
Pro Val Ala Val Ala Cys Ile Leu Ala Glu Met Lys Leu Asp Tyr Glu
    50                  55                  60

acg ctg atg gcc gct ctg ctg cat gac gtg att gaa gat acc ccc gcc       240
Thr Leu Met Ala Ala Leu Leu His Asp Val Ile Glu Asp Thr Pro Ala
65                  70                  75                  80

acc tat cag gac atg gaa cag ctt ttc ggt aaa agc gtt gcc gag ctg       288
Thr Tyr Gln Asp Met Glu Gln Leu Phe Gly Lys Ser Val Ala Glu Leu
                85                  90                  95

gta gag ggg gtg tcg aaa ctt gat aag ctc aag ttt cgc gat aag aaa       336
Val Glu Gly Val Ser Lys Leu Asp Lys Leu Lys Phe Arg Asp Lys Lys
            100                 105                 110

gag gcg cag gcc gaa aac ttt cgc aaa atg att atg gcg atg gtg cag       384
Glu Ala Gln Ala Glu Asn Phe Arg Lys Met Ile Met Ala Met Val Gln
        115                 120                 125

gat atc cgc gtc atc ctc att aag ctt gct gac cgt acc cat aac atg       432
Asp Ile Arg Val Ile Leu Ile Lys Leu Ala Asp Arg Thr His Asn Met
    130                 135                 140

cgc acg ctg ggc tcg tta cgc ccg gat aaa cgt cgt cgt att gcc cgt       480
Arg Thr Leu Gly Ser Leu Arg Pro Asp Lys Arg Arg Arg Ile Ala Arg
145                 150                 155                 160

gaa acg ctg gaa atc tac agt cct ctg gcg cac cgt tta ggt att cat       528
Glu Thr Leu Glu Ile Tyr Ser Pro Leu Ala His Arg Leu Gly Ile His
                165                 170                 175

cac atc aaa acc gag ctg gaa gag ctg ggt ttt gaa gcg ctg tat ccc       576
His Ile Lys Thr Glu Leu Glu Glu Leu Gly Phe Glu Ala Leu Tyr Pro
            180                 185                 190

aat cgt tac cgc gtc atc aaa gaa gtg gta aaa gcg gcg cgc ggc aac       624
Asn Arg Tyr Arg Val Ile Lys Glu Val Val Lys Ala Ala Arg Gly Asn
        195                 200                 205

cgt aag gag atg atc caa aaa atc ctc tct gaa atc gaa gga cgt ttg       672

Arg Lys Glu Met Ile Gln Lys Ile Leu Ser Glu Ile Glu Gly Arg Leu
    210                 215                 220

caa gag gcg gga att ccg tgt cgc gtt agc ggt cgc gaa aaa cat ctt      720
Gln Glu Ala Gly Ile Pro Cys Arg Val Ser Gly Arg Glu Lys His Leu
225                 230                 235                 240

tac tcg atc tac tgc aaa atg gtg ctc aaa gag cag cgt ttt cac tcg      768
Tyr Ser Ile Tyr Cys Lys Met Val Leu Lys Glu Gln Arg Phe His Ser
                245                 250                 255

atc atg gac att tac gct ttc cgc gtc atc gtt cat gac tcc gat acc      816
Ile Met Asp Ile Tyr Ala Phe Arg Val Ile Val His Asp Ser Asp Thr
            260                 265                 270

tgc tat cgc gta ctc ggc cag atg cac agt ctc tat aag ccg cgt ccg      864
Cys Tyr Arg Val Leu Gly Gln Met His Ser Leu Tyr Lys Pro Arg Pro
        275                 280                 285

gga cgg gtg aaa gac tat att gcc att ccc aaa gcg aac ggc tat cag      912
Gly Arg Val Lys Asp Tyr Ile Ala Ile Pro Lys Ala Asn Gly Tyr Gln
    290                 295                 300

tct ttg cac acc tca atg atc ggc ccg cac ggc gtt cct gtt gaa gtc      960
Ser Leu His Thr Ser Met Ile Gly Pro His Gly Val Pro Val Glu Val
305                 310                 315                 320

cag atc cgt acc gaa gat atg gat cag atg gcg gaa atg ggg gtc gcg     1008
Gln Ile Arg Thr Glu Asp Met Asp Gln Met Ala Glu Met Gly Val Ala
                325                 330                 335

gcg cac tgg gcg tat aaa gaa cac ggt gag acc agc acc acg gcg cag     1056
Ala His Trp Ala Tyr Lys Glu His Gly Glu Thr Ser Thr Thr Ala Gln
            340                 345                 350

atc cgc gcc cag cgc tgg atg cag agc ctg ctg gag cta caa cag agc     1104
Ile Arg Ala Gln Arg Trp Met Gln Ser Leu Leu Glu Leu Gln Gln Ser
        355                 360                 365

gcc ggt agt tcg ttt gaa ttt atc gaa agc gta aaa tcc gat ctc ttc     1152
Ala Gly Ser Ser Phe Glu Phe Ile Glu Ser Val Lys Ser Asp Leu Phe
    370                 375                 380

ccg gat gag att tac gtt ttc acc ccg gaa ggg cgc att gtc gaa ctg     1200
Pro Asp Glu Ile Tyr Val Phe Thr Pro Glu Gly Arg Ile Val Glu Leu
385                 390                 395                 400

ccc gct ggc gct acg ccg gtg gat ttt gcc tat gca gtg cat acc gac     1248
Pro Ala Gly Ala Thr Pro Val Asp Phe Ala Tyr Ala Val His Thr Asp
                405                 410                 415

atc ggc cac gcc tgc gtc ggc gcg cgt gtc gac cgc cag cct tat ccg     1296
Ile Gly His Ala Cys Val Gly Ala Arg Val Asp Arg Gln Pro Tyr Pro
            420                 425                 430

ctg tcg cag ccg ctt agc agc ggt cag acc gtc gaa att att acc gcg     1344
Leu Ser Gln Pro Leu Ser Ser Gly Gln Thr Val Glu Ile Ile Thr Ala
        435                 440                 445

ccg ggc gcg cgt ccc aac gcc gcc tgg ctg aac ttt gtc gtc agc tct     1392
Pro Gly Ala Arg Pro Asn Ala Ala Trp Leu Asn Phe Val Val Ser Ser
    450                 455                 460

aaa gcg cgc gct aaa att cgt cag ttg ctg aaa aac ctc aaa cgt gat     1440
Lys Ala Arg Ala Lys Ile Arg Gln Leu Leu Lys Asn Leu Lys Arg Asp

465                 470                 475                 480

gac tcc gta agc ctg ggc cgt cgt ctg ctt aac cat gcc tta ggc ggt      1488
Asp Ser Val Ser Leu Gly Arg Arg Leu Leu Asn His Ala Leu Gly Gly
                485                 490                 495

agt cgt aag ctg gcg gaa att ccg cag gaa aat att cag cgc gaa ttg      1536
Ser Arg Lys Leu Ala Glu Ile Pro Gln Glu Asn Ile Gln Arg Glu Leu
            500                 505                 510

gat cgt atg aag ctg gca acg ctt gac gat ctg ctg gcg gaa atc ggt      1584
Asp Arg Met Lys Leu Ala Thr Leu Asp Asp Leu Leu Ala Glu Ile Gly
        515                 520                 525

ctc ggc aac gcg atg agc gta gtg gtc gcg aaa aat ctg cag caa ggc      1632
Leu Gly Asn Ala Met Ser Val Val Val Ala Lys Asn Leu Gln Gln Gly
    530                 535                 540

gaa gcc gtg gtg ccg acc gtt gcg caa tcg aat cac ggc cac ctg ccg      1680
Glu Ala Val Val Pro Thr Val Ala Gln Ser Asn His Gly His Leu Pro
545                 550                 555                 560

att aaa ggc gcg gat ggc gtg ctt atc acc ttt gcg aag tgt tgt cgt      1728
Ile Lys Gly Ala Asp Gly Val Leu Ile Thr Phe Ala Lys Cys Cys Arg
                565                 570                 575

ccg atc cca ggc gac ccg atc atc gct cac gtc agc cca ggt aaa gga      1776
Pro Ile Pro Gly Asp Pro Ile Ile Ala His Val Ser Pro Gly Lys Gly
            580                 585                 590

ctg gtg atc cac cac gaa tcc tgc cgt aat atc cgt gga tac cag aaa      1824
Leu Val Ile His His Glu Ser Cys Arg Asn Ile Arg Gly Tyr Gln Lys
        595                 600                 605

gag cca gag aaa ttt atg gcg gtc gaa tgg gac aaa gag acg gag cag      1872
Glu Pro Glu Lys Phe Met Ala Val Glu Trp Asp Lys Glu Thr Glu Gln
    610                 615                 620

gaa ttc att acc gaa atc aag gtg gaa atg ttt aac cat cag ggc gcg      1920
Glu Phe Ile Thr Glu Ile Lys Val Glu Met Phe Asn His Gln Gly Ala
625                 630                 635                 640

ctg gct aac ctg acg gcg gcg att aat acc acc acc tcc aat att caa      1968
Leu Ala Asn Leu Thr Ala Ala Ile Asn Thr Thr Thr Ser Asn Ile Gln
                645                 650                 655

agc ctg aat act gaa gag aaa gat ggt cgc gtc tat agt acc ttt att      2016
Ser Leu Asn Thr Glu Glu Lys Asp Gly Arg Val Tyr Ser Thr Phe Ile
            660                 665                 670

cgc ctt acc gca cgc gat cgc gta cat ctg gcg aat atc atg cgc aaa      2064
Arg Leu Thr Ala Arg Asp Arg Val His Leu Ala Asn Ile Met Arg Lys
        675                 680                 685

atc cgc gtg atg cca gac gtc att aaa gtc acc cgt aac cga aac tag      2112
Ile Arg Val Met Pro Asp Val Ile Lys Val Thr Arg Asn Arg Asn
    690                 695                 700


<210>  4
<211>  703
<212>  PRT
<213>  Salmonella enterica subsp. enterica serovar Typhimurium str. LT2

<400>  4

Leu Tyr Leu Phe Glu Ser Leu Asn Gln Leu Ile Gln Thr Tyr Leu Pro
1               5                   10                  15

Glu Asp Gln Ile Lys Arg Leu Arg Gln Ala Tyr Leu Val Ala Arg Asp
            20                  25                  30

Ala His Glu Gly Gln Thr Arg Ser Ser Gly Glu Pro Tyr Ile Thr His
        35                  40                  45

Pro Val Ala Val Ala Cys Ile Leu Ala Glu Met Lys Leu Asp Tyr Glu
    50                  55                  60

Thr Leu Met Ala Ala Leu Leu His Asp Val Ile Glu Asp Thr Pro Ala
65                  70                  75                  80

Thr Tyr Gln Asp Met Glu Gln Leu Phe Gly Lys Ser Val Ala Glu Leu
                85                  90                  95

Val Glu Gly Val Ser Lys Leu Asp Lys Leu Lys Phe Arg Asp Lys Lys
            100                 105                 110

Glu Ala Gln Ala Glu Asn Phe Arg Lys Met Ile Met Ala Met Val Gln
        115                 120                 125

Asp Ile Arg Val Ile Leu Ile Lys Leu Ala Asp Arg Thr His Asn Met
    130                 135                 140

Arg Thr Leu Gly Ser Leu Arg Pro Asp Lys Arg Arg Arg Ile Ala Arg
145                 150                 155                 160

Glu Thr Leu Glu Ile Tyr Ser Pro Leu Ala His Arg Leu Gly Ile His
                165                 170                 175

His Ile Lys Thr Glu Leu Glu Glu Leu Gly Phe Glu Ala Leu Tyr Pro
            180                 185                 190

Asn Arg Tyr Arg Val Ile Lys Glu Val Val Lys Ala Ala Arg Gly Asn
        195                 200                 205

Arg Lys Glu Met Ile Gln Lys Ile Leu Ser Glu Ile Glu Gly Arg Leu
    210                 215                 220

Gln Glu Ala Gly Ile Pro Cys Arg Val Ser Gly Arg Glu Lys His Leu
225                 230                 235                 240

Tyr Ser Ile Tyr Cys Lys Met Val Leu Lys Glu Gln Arg Phe His Ser
                245                 250                 255

Ile Met Asp Ile Tyr Ala Phe Arg Val Ile Val His Asp Ser Asp Thr
            260                 265                 270

Cys Tyr Arg Val Leu Gly Gln Met His Ser Leu Tyr Lys Pro Arg Pro
        275                 280                 285

Gly Arg Val Lys Asp Tyr Ile Ala Ile Pro Lys Ala Asn Gly Tyr Gln
    290                 295                 300

Ser Leu His Thr Ser Met Ile Gly Pro His Gly Val Pro Val Glu Val
305                 310                 315                 320

Gln Ile Arg Thr Glu Asp Met Asp Gln Met Ala Glu Met Gly Val Ala
                325                 330                 335

Ala His Trp Ala Tyr Lys Glu His Gly Glu Thr Ser Thr Thr Ala Gln
            340                 345                 350

Ile Arg Ala Gln Arg Trp Met Gln Ser Leu Leu Glu Leu Gln Gln Ser
        355                 360                 365

Ala Gly Ser Ser Phe Glu Phe Ile Glu Ser Val Lys Ser Asp Leu Phe
    370                 375                 380

Pro Asp Glu Ile Tyr Val Phe Thr Pro Glu Gly Arg Ile Val Glu Leu
385                 390                 395                 400

Pro Ala Gly Ala Thr Pro Val Asp Phe Ala Tyr Ala Val His Thr Asp
                405                 410                 415

Ile Gly His Ala Cys Val Gly Ala Arg Val Asp Arg Gln Pro Tyr Pro
            420                 425                 430

Leu Ser Gln Pro Leu Ser Ser Gly Gln Thr Val Glu Ile Ile Thr Ala
        435                 440                 445

Pro Gly Ala Arg Pro Asn Ala Ala Trp Leu Asn Phe Val Val Ser Ser
    450                 455                 460

Lys Ala Arg Ala Lys Ile Arg Gln Leu Leu Lys Asn Leu Lys Arg Asp
465                 470                 475                 480

Asp Ser Val Ser Leu Gly Arg Arg Leu Leu Asn His Ala Leu Gly Gly
                485                 490                 495

Ser Arg Lys Leu Ala Glu Ile Pro Gln Glu Asn Ile Gln Arg Glu Leu
            500                 505                 510

Asp Arg Met Lys Leu Ala Thr Leu Asp Asp Leu Leu Ala Glu Ile Gly

515                 520                 525

Leu Gly Asn Ala Met Ser Val Val Val Ala Lys Asn Leu Gln Gln Gly
    530                 535                 540

Glu Ala Val Val Pro Thr Val Ala Gln Ser Asn His Gly His Leu Pro
545                 550                 555                 560

Ile Lys Gly Ala Asp Gly Val Leu Ile Thr Phe Ala Lys Cys Cys Arg
                565                 570                 575

Pro Ile Pro Gly Asp Pro Ile Ile Ala His Val Ser Pro Gly Lys Gly
            580                 585                 590

Leu Val Ile His His Glu Ser Cys Arg Asn Ile Arg Gly Tyr Gln Lys
        595                 600                 605

Glu Pro Glu Lys Phe Met Ala Val Glu Trp Asp Lys Glu Thr Glu Gln
    610                 615                 620

Glu Phe Ile Thr Glu Ile Lys Val Glu Met Phe Asn His Gln Gly Ala
625                 630                 635                 640

Leu Ala Asn Leu Thr Ala Ala Ile Asn Thr Thr Thr Ser Asn Ile Gln
                645                 650                 655

Ser Leu Asn Thr Glu Glu Lys Asp Gly Arg Val Tyr Ser Thr Phe Ile
            660                 665                 670

Arg Leu Thr Ala Arg Asp Arg Val His Leu Ala Asn Ile Met Arg Lys
        675                 680                 685

Ile Arg Val Met Pro Asp Val Ile Lys Val Thr Arg Asn Arg Asn
    690                 695                 700

<210>  5
<211>  2112
<212>  DNA
<213>  Serratia proteamaculans 568

<220>
<221>  CDS
<222>  (1)..(2109)
<223>  locus tag Spro_4869

<400>  5
ttg tac ctg ttt gaa agc ctg aat ctg ctg att caa cgt tac ctg cct      48
Leu Tyr Leu Phe Glu Ser Leu Asn Leu Leu Ile Gln Arg Tyr Leu Pro
1               5                   10                  15

gag gag cag att aag cgc ctc aaa cag gca tac ctc gtt gca cgt gat      96
Glu Glu Gln Ile Lys Arg Leu Lys Gln Ala Tyr Leu Val Ala Arg Asp

20                  25                  30

gct cac gag gga cag aca cgc tcc agc ggt gag ccc tac att act cac          144
Ala His Glu Gly Gln Thr Arg Ser Ser Gly Glu Pro Tyr Ile Thr His
        35                  40                  45

ccg gtt gcc gtg gcc tgc att ctg gcg gaa atg cgt ctc gat cat gag          192
Pro Val Ala Val Ala Cys Ile Leu Ala Glu Met Arg Leu Asp His Glu
    50                  55                  60

acg ctg atg gca gca ctg ctg cac gac gtc atc gaa gat acc ccg gcc          240
Thr Leu Met Ala Ala Leu Leu His Asp Val Ile Glu Asp Thr Pro Ala
65                  70                  75                  80

acc tac cag gat atg gaa cag cta ttc ggc aaa agc gtt gcc gaa ctg          288
Thr Tyr Gln Asp Met Glu Gln Leu Phe Gly Lys Ser Val Ala Glu Leu
                85                  90                  95

gtt gaa ggc gta tcc aag ctc gac aag ctg aag ttc cag gat aaa aaa          336
Val Glu Gly Val Ser Lys Leu Asp Lys Leu Lys Phe Gln Asp Lys Lys
            100                 105                 110

gaa gct cag gcg gaa aac ttc cgc aag atg atc atg gcg atg gtg cag          384
Glu Ala Gln Ala Glu Asn Phe Arg Lys Met Ile Met Ala Met Val Gln
        115                 120                 125

gat atc cgc gtc gtg ctg atc aag ctg gcc gac cgt acg cac aac atg          432
Asp Ile Arg Val Val Leu Ile Lys Leu Ala Asp Arg Thr His Asn Met
    130                 135                 140

cgc act ctc ggt tcg ctg cgt cct gac aaa cgg cgg cgt att gca cgt          480
Arg Thr Leu Gly Ser Leu Arg Pro Asp Lys Arg Arg Arg Ile Ala Arg
145                 150                 155                 160

gaa acc ctg gaa atc tac agc ccc ctg gcc cat cgc ctg ggt att cac          528
Glu Thr Leu Glu Ile Tyr Ser Pro Leu Ala His Arg Leu Gly Ile His
                165                 170                 175

cac ctg aaa acc gag ctg gaa gag ctg ggt ttt gag gcg ttg tac ccg          576
His Leu Lys Thr Glu Leu Glu Glu Leu Gly Phe Glu Ala Leu Tyr Pro
            180                 185                 190

aac cgc tat cgc gta atc aaa gaa gtg gtg aaa gcc gca cgc ggt aac          624
Asn Arg Tyr Arg Val Ile Lys Glu Val Val Lys Ala Ala Arg Gly Asn
        195                 200                 205

cgt aaa gag atg atc cag aag att ctc tcc gag atc gaa ggg cga ctg          672
Arg Lys Glu Met Ile Gln Lys Ile Leu Ser Glu Ile Glu Gly Arg Leu
    210                 215                 220

acc gag gcc ggc att ccc tgc cgc gtc agc gga cgg gaa aaa cat ctg          720
Thr Glu Ala Gly Ile Pro Cys Arg Val Ser Gly Arg Glu Lys His Leu
225                 230                 235                 240

tat tcc atc tac ctc aag atg cac ctg aaa gaa cag cgt ttc cat tcg          768
Tyr Ser Ile Tyr Leu Lys Met His Leu Lys Glu Gln Arg Phe His Ser
                245                 250                 255

att atg gat atc tac gcg ttc cgg gtg atc gtg aag gaa gtc gac acc          816
Ile Met Asp Ile Tyr Ala Phe Arg Val Ile Val Lys Glu Val Asp Thr
            260                 265                 270

tgc tac cgc gtg ctg ggc cag gct cac agc ctg tac aaa ccg cgt ccg          864
Cys Tyr Arg Val Leu Gly Gln Ala His Ser Leu Tyr Lys Pro Arg Pro
        275                 280                 285

ggc agg gtc aaa gac tat atc gcc att ccc aag gcc aac ggc tat caa 912
Gly Arg Val Lys Asp Tyr Ile Ala Ile Pro Lys Ala Asn Gly Tyr Gln
    290                 295                 300

tcg ctg cac acc tca ctg atc ggc cca cat ggc gta ccg gtt gaa gtg 960
Ser Leu His Thr Ser Leu Ile Gly Pro His Gly Val Pro Val Glu Val
305                 310                 315                 320

cag atc cgt acc gaa gat atg gat cag atg gcc gaa atg ggg gtc gcc 1008
Gln Ile Arg Thr Glu Asp Met Asp Gln Met Ala Glu Met Gly Val Ala
                325                 330                 335

gcg cac tgg gct tat aaa gaa aaa gaa cag ggc gaa acc ggc acc acc 1056
Ala His Trp Ala Tyr Lys Glu Lys Glu Gln Gly Glu Thr Gly Thr Thr
            340                 345                 350

gca caa atc cgc gct cag cgg tgg atg cag agc ttg ctg gag ctg caa 1104
Ala Gln Ile Arg Ala Gln Arg Trp Met Gln Ser Leu Leu Glu Leu Gln
        355                 360                 365

caa agc gcc ggc agt tcg ttt gaa ttt atc gag agc gta aaa tcc gat 1152
Gln Ser Ala Gly Ser Ser Phe Glu Phe Ile Glu Ser Val Lys Ser Asp
    370                 375                 380

ctg ttc ccg gat gag att tac gtt ttc acc ccg gaa ggc cgc att gtt 1200
Leu Phe Pro Asp Glu Ile Tyr Val Phe Thr Pro Glu Gly Arg Ile Val
385                 390                 395                 400

gaa ttg cct gcg ggc gca acg ccg gtc gac ttc gcc tac gcg gtg cac 1248
Glu Leu Pro Ala Gly Ala Thr Pro Val Asp Phe Ala Tyr Ala Val His
                405                 410                 415

acc gat atc ggc cac gcc tgc gtt ggc gca cgt gtt gat cgc cag cca 1296
Thr Asp Ile Gly His Ala Cys Val Gly Ala Arg Val Asp Arg Gln Pro
            420                 425                 430

tac ccg ctg tca cag tcc ttg acc agc ggg caa acg gtc gaa atc atc 1344
Tyr Pro Leu Ser Gln Ser Leu Thr Ser Gly Gln Thr Val Glu Ile Ile
        435                 440                 445

acg gct cct ggt gca cgg cct aac gcc gcc tgg ctg aac ttt gtc gtc 1392
Thr Ala Pro Gly Ala Arg Pro Asn Ala Ala Trp Leu Asn Phe Val Val
    450                 455                 460

agc tca aaa gcg cgc gca aaa atc cgc caa atg ctg aaa aac ctc aag 1440
Ser Ser Lys Ala Arg Ala Lys Ile Arg Gln Met Leu Lys Asn Leu Lys
465                 470                 475                 480

cgc gat gat tca gtc ggc ctc ggc cgc cgc ttg tta aat cat gca ttg 1488
Arg Asp Asp Ser Val Gly Leu Gly Arg Arg Leu Leu Asn His Ala Leu
                485                 490                 495

ggc ggc agt cgc aaa ctg gca gag gtt ccg gca gaa aat atc caa cac 1536
Gly Gly Ser Arg Lys Leu Ala Glu Val Pro Ala Glu Asn Ile Gln His
            500                 505                 510

gag ttg gat cgc atg aaa ctg gcg acg ctg gac gac ctg ttg gct gaa 1584
Glu Leu Asp Arg Met Lys Leu Ala Thr Leu Asp Asp Leu Leu Ala Glu
        515                 520                 525

atc ggc ttg ggc aat gcc atg agc gtg gtg gta gcg aaa aac ctg caa 1632
Ile Gly Leu Gly Asn Ala Met Ser Val Val Val Ala Lys Asn Leu Gln
    530                 535                 540

ggt gac cag tct aat ctg ggg gcc tcc tct ggg gtt cgt aat ctg gcg 1680
Gly Asp Gln Ser Asn Leu Gly Ala Ser Ser Gly Val Arg Asn Leu Ala
545                 550                 555                 560

atc aag ggc tca gac ggc gtg ctg atc acc ttc gct aaa tgc tgc cga 1728
Ile Lys Gly Ser Asp Gly Val Leu Ile Thr Phe Ala Lys Cys Cys Arg
                565                 570                 575

cct att cca ggc gat ccg atc atc gcc cac gtc agc cca ggc aaa ggc 1776
Pro Ile Pro Gly Asp Pro Ile Ile Ala His Val Ser Pro Gly Lys Gly
            580                 585                 590

ctg gtg atc cac cat gag tcc tgc cga aat atc cgc ggc tac cag aaa 1824
Leu Val Ile His His Glu Ser Cys Arg Asn Ile Arg Gly Tyr Gln Lys
        595                 600                 605

gaa cct gaa aaa ttc atg gcg gta gag tgg gat caa gac atc gaa cag 1872
Glu Pro Glu Lys Phe Met Ala Val Glu Trp Asp Gln Asp Ile Glu Gln
    610                 615                 620

gaa ttc atc gcc gag atc aaa gtg gac atg ttt aat cat cag ggg gca 1920
Glu Phe Ile Ala Glu Ile Lys Val Asp Met Phe Asn His Gln Gly Ala
625                 630                 635                 640

ctg gcc aac ctg acg gca gcc atc aat gcg gct gag tcc aat att caa 1968
Leu Ala Asn Leu Thr Ala Ala Ile Asn Ala Ala Glu Ser Asn Ile Gln
                645                 650                 655

agc ctg aat acc gaa gaa aaa gac ggc cgg gtt tat agt gca ttt atc 2016
Ser Leu Asn Thr Glu Glu Lys Asp Gly Arg Val Tyr Ser Ala Phe Ile
            660                 665                 670

cgt ttg acc acc cgc gat cgc atc cat ctg gca aat att atg cgt aaa 2064
Arg Leu Thr Thr Arg Asp Arg Ile His Leu Ala Asn Ile Met Arg Lys
        675                 680                 685

atc cgt atc atg ccg gat gtg att aaa gtt aac cgt aac cga aat tag 2112
Ile Arg Ile Met Pro Asp Val Ile Lys Val Asn Arg Asn Arg Asn
    690                 695                 700

<210> 6
<211> 703
<212> PRT
<213> Serratia proteamaculans 568

<400> 6

Leu Tyr Leu Phe Glu Ser Leu Asn Leu Leu Ile Gln Arg Tyr Leu Pro
1               5                   10                  15

Glu Glu Gln Ile Lys Arg Leu Lys Gln Ala Tyr Leu Val Ala Arg Asp
            20                  25                  30

Ala His Glu Gly Gln Thr Arg Ser Ser Gly Glu Pro Tyr Ile Thr His
        35                  40                  45

Pro Val Ala Val Ala Cys Ile Leu Ala Glu Met Arg Leu Asp His Glu
    50                  55                  60

Thr Leu Met Ala Ala Leu Leu His Asp Val Ile Glu Asp Thr Pro Ala

65                  70                  75                  80

Thr Tyr Gln Asp Met Glu Gln Leu Phe Gly Lys Ser Val Ala Glu Leu
                85                  90                  95

Val Glu Gly Val Ser Lys Leu Asp Lys Leu Lys Phe Gln Asp Lys Lys
            100                 105                 110

Glu Ala Gln Ala Glu Asn Phe Arg Lys Met Ile Met Ala Met Val Gln
        115                 120                 125

Asp Ile Arg Val Val Leu Ile Lys Leu Ala Asp Arg Thr His Asn Met
    130                 135                 140

Arg Thr Leu Gly Ser Leu Arg Pro Asp Lys Arg Arg Arg Ile Ala Arg
145                 150                 155                 160

Glu Thr Leu Glu Ile Tyr Ser Pro Leu Ala His Arg Leu Gly Ile His
                165                 170                 175

His Leu Lys Thr Glu Leu Glu Glu Leu Gly Phe Glu Ala Leu Tyr Pro
            180                 185                 190

Asn Arg Tyr Arg Val Ile Lys Glu Val Val Lys Ala Ala Arg Gly Asn
        195                 200                 205

Arg Lys Glu Met Ile Gln Lys Ile Leu Ser Glu Ile Glu Gly Arg Leu
    210                 215                 220

Thr Glu Ala Gly Ile Pro Cys Arg Val Ser Gly Arg Glu Lys His Leu
225                 230                 235                 240

Tyr Ser Ile Tyr Leu Lys Met His Leu Lys Glu Gln Arg Phe His Ser
                245                 250                 255

Ile Met Asp Ile Tyr Ala Phe Arg Val Ile Val Lys Glu Val Asp Thr
            260                 265                 270

Cys Tyr Arg Val Leu Gly Gln Ala His Ser Leu Tyr Lys Pro Arg Pro
        275                 280                 285

Gly Arg Val Lys Asp Tyr Ile Ala Ile Pro Lys Ala Asn Gly Tyr Gln
    290                 295                 300

Ser Leu His Thr Ser Leu Ile Gly Pro His Gly Val Pro Val Glu Val
305                 310                 315                 320

Gln Ile Arg Thr Glu Asp Met Asp Gln Met Ala Glu Met Gly Val Ala
                325                 330                 335

Ala His Trp Ala Tyr Lys Glu Lys Glu Gln Gly Glu Thr Gly Thr Thr
            340                 345                 350

Ala Gln Ile Arg Ala Gln Arg Trp Met Gln Ser Leu Leu Glu Leu Gln
        355                 360                 365

Gln Ser Ala Gly Ser Ser Phe Glu Phe Ile Glu Ser Val Lys Ser Asp
    370                 375                 380

Leu Phe Pro Asp Glu Ile Tyr Val Phe Thr Pro Glu Gly Arg Ile Val
385                 390                 395                 400

Glu Leu Pro Ala Gly Ala Thr Pro Val Asp Phe Ala Tyr Ala Val His
                405                 410                 415

Thr Asp Ile Gly His Ala Cys Val Gly Ala Arg Val Asp Arg Gln Pro
            420                 425                 430

Tyr Pro Leu Ser Gln Ser Leu Thr Ser Gly Gln Thr Val Glu Ile Ile
        435                 440                 445

Thr Ala Pro Gly Ala Arg Pro Asn Ala Ala Trp Leu Asn Phe Val Val
    450                 455                 460

Ser Ser Lys Ala Arg Ala Lys Ile Arg Gln Met Leu Lys Asn Leu Lys
465                 470                 475                 480

Arg Asp Asp Ser Val Gly Leu Gly Arg Arg Leu Leu Asn His Ala Leu
                485                 490                 495

Gly Gly Ser Arg Lys Leu Ala Glu Val Pro Ala Glu Asn Ile Gln His
            500                 505                 510

Glu Leu Asp Arg Met Lys Leu Ala Thr Leu Asp Asp Leu Leu Ala Glu
        515                 520                 525

Ile Gly Leu Gly Asn Ala Met Ser Val Val Val Ala Lys Asn Leu Gln
    530                 535                 540

Gly Asp Gln Ser Asn Leu Gly Ala Ser Ser Gly Val Arg Asn Leu Ala
545                 550                 555                 560

Ile Lys Gly Ser Asp Gly Val Leu Ile Thr Phe Ala Lys Cys Cys Arg
                565                 570                 575

Pro Ile Pro Gly Asp Pro Ile Ile Ala His Val Ser Pro Gly Lys Gly
            580                 585                 590

Leu Val Ile His His Glu Ser Cys Arg Asn Ile Arg Gly Tyr Gln Lys
        595                 600                 605

Glu Pro Glu Lys Phe Met Ala Val Glu Trp Asp Gln Asp Ile Glu Gln
    610                 615                 620

Glu Phe Ile Ala Glu Ile Lys Val Asp Met Phe Asn His Gln Gly Ala
625                 630                 635                 640

Leu Ala Asn Leu Thr Ala Ala Ile Asn Ala Ala Glu Ser Asn Ile Gln
                645                 650                 655

Ser Leu Asn Thr Glu Glu Lys Asp Gly Arg Val Tyr Ser Ala Phe Ile
            660                 665                 670

Arg Leu Thr Thr Arg Asp Arg Ile His Leu Ala Asn Ile Met Arg Lys
        675                 680                 685

Ile Arg Ile Met Pro Asp Val Ile Lys Val Asn Arg Asn Arg Asn
    690                 695                 700

<210>  7
<211>  2115
<212>  DNA
<213>  Escherichia coli str. K12 substr. MG442

<220>
<221>  CDS
<222>  (1)..(2112)
<223>  spoT_allel

<220>
<221>  mutation
<222>  (253)..(258)
<223>  Insertion of catgat causes an HD-Insertion after amino acid
       position 84 in the SpoT_allel protein

<220>
<221>  mutation
<222>  (526)..(526)
<223>  SNP c520t  (position referred to spoT_MG1655) causes the amino
       acid exchange G174C in the SpoT_allel protein (position referred
       to SpoT_MG1655)

<220>
<221>  mutation
<222>  (1591)..(1591)
<223>  SNP c1585t (position referred to spoT_MG1655) causes the amino
       acid exchange L529F in the SpoT_allel protein (position referred
       to SpoT_MG1655)

<400>  7
ttg tat ctg ttt gaa agc ctg aat caa ctg att caa acc tac ctg ccg        48
Leu Tyr Leu Phe Glu Ser Leu Asn Gln Leu Ile Gln Thr Tyr Leu Pro
1               5                   10                  15

gaa gac caa atc aag cgt ctg cgg cag gcg tat ctc gtt gca cgt gat          96
Glu Asp Gln Ile Lys Arg Leu Arg Gln Ala Tyr Leu Val Ala Arg Asp
            20                  25                  30

gct cac gag ggg caa aca cgt tca agc ggt gaa ccc tat atc acg cac         144
Ala His Glu Gly Gln Thr Arg Ser Ser Gly Glu Pro Tyr Ile Thr His
        35                  40                  45

ccg gta gcg gtt gcc tgc att ctg gcc gag atg aaa ctc gac tat gaa         192
Pro Val Ala Val Ala Cys Ile Leu Ala Glu Met Lys Leu Asp Tyr Glu
    50                  55                  60

acg ctg atg gcg gcg ctg ctg cat gac gtg att gaa gat act ccc gcc         240
Thr Leu Met Ala Ala Leu Leu His Asp Val Ile Glu Asp Thr Pro Ala
65                  70                  75                  80

acc tac cag gat cat gat atg gaa cag ctt ttt ggt aaa agc gtc gcc         288
Thr Tyr Gln Asp His Asp Met Glu Gln Leu Phe Gly Lys Ser Val Ala
                85                  90                  95

gag ctg gta gag ggg gtg tcg aaa ctt gat aaa ctc aag ttc cgc gat         336
Glu Leu Val Glu Gly Val Ser Lys Leu Asp Lys Leu Lys Phe Arg Asp
            100                 105                 110

aag aaa gag gcg cag gcc gaa aac ttt cgc aag atg att atg gcg atg         384
Lys Lys Glu Ala Gln Ala Glu Asn Phe Arg Lys Met Ile Met Ala Met
        115                 120                 125

gtg cag gat atc cgc gtc atc ctc atc aaa ctt gcc gac cgt acc cac         432
Val Gln Asp Ile Arg Val Ile Leu Ile Lys Leu Ala Asp Arg Thr His
    130                 135                 140

aac atg cgc acg ctg ggc tca ctt cgc ccg gac aaa cgt cgc cgc atc         480
Asn Met Arg Thr Leu Gly Ser Leu Arg Pro Asp Lys Arg Arg Arg Ile
145                 150                 155                 160

gcc cgt gaa act ctc gaa att tat agc ccg ctg gcg cac cgt tta tgt         528
Ala Arg Glu Thr Leu Glu Ile Tyr Ser Pro Leu Ala His Arg Leu Cys
                165                 170                 175

atc cac cac att aaa acc gaa ctc gaa gag ctg ggt ttt gag gcg ctg         576
Ile His His Ile Lys Thr Glu Leu Glu Glu Leu Gly Phe Glu Ala Leu
            180                 185                 190

tat ccc aac cgt tat cgc gta atc aaa gaa gtg gtg aaa gcc gcg cgc         624
Tyr Pro Asn Arg Tyr Arg Val Ile Lys Glu Val Val Lys Ala Ala Arg
        195                 200                 205

ggc aac cgt aaa gag atg atc cag aag att ctt tct gaa atc gaa ggg         672
Gly Asn Arg Lys Glu Met Ile Gln Lys Ile Leu Ser Glu Ile Glu Gly
    210                 215                 220

cgt ttg cag gaa gcg gga ata ccg tgc cgc gtc agt ggt cgc gag aag         720
Arg Leu Gln Glu Ala Gly Ile Pro Cys Arg Val Ser Gly Arg Glu Lys
225                 230                 235                 240

cat ctt tat tcg att tac tgc aaa atg gtg ctc aaa gag cag cgt ttt         768
His Leu Tyr Ser Ile Tyr Cys Lys Met Val Leu Lys Glu Gln Arg Phe
                245                 250                 255

cac tcg atc atg gac atc tac gct ttc cgc gtg atc gtc aat gat tct         816
His Ser Ile Met Asp Ile Tyr Ala Phe Arg Val Ile Val Asn Asp Ser
            260                 265                 270

gac acc tgt tat cgc gtg ctg ggc cag atg cac agc ctg tac aag ccg         864

Asp Thr Cys Tyr Arg Val Leu Gly Gln Met His Ser Leu Tyr Lys Pro
        275                 280                 285

cgt ccg ggc cgc gtg aaa gac tat atc gcc att cca aaa gcg aac ggc         912
Arg Pro Gly Arg Val Lys Asp Tyr Ile Ala Ile Pro Lys Ala Asn Gly
    290                 295                 300

tat cag tct ttg cac acc tcg atg atc ggc ccg cac ggt gtg ccg gtt         960
Tyr Gln Ser Leu His Thr Ser Met Ile Gly Pro His Gly Val Pro Val
305                 310                 315                 320

gag gtc cag atc cgt acc gaa gat atg gac cag atg gcg gag atg ggt        1008
Glu Val Gln Ile Arg Thr Glu Asp Met Asp Gln Met Ala Glu Met Gly
                325                 330                 335

gtt gcc gcg cac tgg gct tat aaa gag cac ggc gaa acc agt act acc        1056
Val Ala Ala His Trp Ala Tyr Lys Glu His Gly Glu Thr Ser Thr Thr
            340                 345                 350

gca caa atc cgc gcc cag cgc tgg atg caa agc ctg ctg gag ctg caa        1104
Ala Gln Ile Arg Ala Gln Arg Trp Met Gln Ser Leu Leu Glu Leu Gln
        355                 360                 365

cag agc gcc ggt agt tcg ttt gaa ttt atc gag agc gtt aaa tcc gat        1152
Gln Ser Ala Gly Ser Ser Phe Glu Phe Ile Glu Ser Val Lys Ser Asp
    370                 375                 380

ctc ttc ccg gat gag att tac gtt ttc aca ccg gaa ggg cgc att gtc        1200
Leu Phe Pro Asp Glu Ile Tyr Val Phe Thr Pro Glu Gly Arg Ile Val
385                 390                 395                 400

gag ctg cct gcc ggt gca acg ccc gtc gac ttc gct tat gca gtg cat        1248
Glu Leu Pro Ala Gly Ala Thr Pro Val Asp Phe Ala Tyr Ala Val His
                405                 410                 415

acc gat atc ggt cat gcc tgc gtg ggc gca cgc gtt gac cgc cag cct        1296
Thr Asp Ile Gly His Ala Cys Val Gly Ala Arg Val Asp Arg Gln Pro
            420                 425                 430

tac ccg ctg tcg cag ccg ctt acc agc ggt caa acc gtt gaa atc att        1344
Tyr Pro Leu Ser Gln Pro Leu Thr Ser Gly Gln Thr Val Glu Ile Ile
        435                 440                 445

acc gct ccg ggc gct cgc ccg aat gcc gct tgg ctg aac ttt gtc gtt        1392
Thr Ala Pro Gly Ala Arg Pro Asn Ala Ala Trp Leu Asn Phe Val Val
    450                 455                 460

agc tcg aaa gcg cgc gcc aaa att cgt cag ttg ctg aaa aac ctc aag        1440
Ser Ser Lys Ala Arg Ala Lys Ile Arg Gln Leu Leu Lys Asn Leu Lys
465                 470                 475                 480

cgt gat gat tct gta agc ctg ggc cgt cgt ctg ctc aac cat gct ttg        1488
Arg Asp Asp Ser Val Ser Leu Gly Arg Arg Leu Leu Asn His Ala Leu
                485                 490                 495

ggt ggt agc cgt aag ctg aat gaa atc ccg cag gaa aat att cag cgc        1536
Gly Gly Ser Arg Lys Leu Asn Glu Ile Pro Gln Glu Asn Ile Gln Arg
            500                 505                 510

gag ctg gat cgc atg aag ctg gca acg ctt gac gat ctg ctg gca gaa        1584
Glu Leu Asp Arg Met Lys Leu Ala Thr Leu Asp Asp Leu Leu Ala Glu
        515                 520                 525

atc gga ttt ggt aac gca atg agc gtg gtg gtc gcg aaa aat ctg caa        1632
Ile Gly Phe Gly Asn Ala Met Ser Val Val Val Ala Lys Asn Leu Gln

530                 535                 540

cat ggg gac gcc tcc att cca ccg gca acc caa agc cac gga cat ctg         1680
His Gly Asp Ala Ser Ile Pro Pro Ala Thr Gln Ser His Gly His Leu
545                 550                 555                 560

ccc att aaa ggt gcc gat ggc gtg ctg atc acc ttt gcg aaa tgc tgc         1728
Pro Ile Lys Gly Ala Asp Gly Val Leu Ile Thr Phe Ala Lys Cys Cys
                565                 570                 575

cgc cct att cct ggc gac ccg att atc gcc cac gtc agc ccc ggt aaa         1776
Arg Pro Ile Pro Gly Asp Pro Ile Ile Ala His Val Ser Pro Gly Lys
            580                 585                 590

ggt ctg gtg atc cac cat gaa tcc tgc cgt aat atc cgt ggc tac cag         1824
Gly Leu Val Ile His His Glu Ser Cys Arg Asn Ile Arg Gly Tyr Gln
        595                 600                 605

aaa gag cca gag aag ttt atg gct gtg gaa tgg gat aaa gag acg gcg         1872
Lys Glu Pro Glu Lys Phe Met Ala Val Glu Trp Asp Lys Glu Thr Ala
    610                 615                 620

cag gag ttc atc acc gaa atc aag gtg gag atg ttc aat cat cag ggt         1920
Gln Glu Phe Ile Thr Glu Ile Lys Val Glu Met Phe Asn His Gln Gly
625                 630                 635                 640

gcg ctg gca aac ctg acg gcg gca att aac acc acg act tcg aat att         1968
Ala Leu Ala Asn Leu Thr Ala Ala Ile Asn Thr Thr Thr Ser Asn Ile
                645                 650                 655

caa agt ttg aat acg gaa gag aaa gat ggt cgc gtc tac agc gcc ttt         2016
Gln Ser Leu Asn Thr Glu Glu Lys Asp Gly Arg Val Tyr Ser Ala Phe
            660                 665                 670

att cgt ctg acc gct cgt gac cgt gtg cat ctg gcg aat atc atg cgc         2064
Ile Arg Leu Thr Ala Arg Asp Arg Val His Leu Ala Asn Ile Met Arg
        675                 680                 685

aaa atc cgc gtg atg cca gac gtg att aaa gtc acc cga aac cga aat         2112
Lys Ile Arg Val Met Pro Asp Val Ile Lys Val Thr Arg Asn Arg Asn
    690                 695                 700

taa                                                                     2115

<210>  8
<211>  704
<212>  PRT
<213>  Escherichia coli str. K12 substr. MG442

<400>  8

Leu Tyr Leu Phe Glu Ser Leu Asn Gln Leu Ile Gln Thr Tyr Leu Pro
1               5                   10                  15

Glu Asp Gln Ile Lys Arg Leu Arg Gln Ala Tyr Leu Val Ala Arg Asp
            20                  25                  30

Ala His Glu Gly Gln Thr Arg Ser Ser Gly Glu Pro Tyr Ile Thr His
        35                  40                  45

Pro Val Ala Val Ala Cys Ile Leu Ala Glu Met Lys Leu Asp Tyr Glu

50                  55                  60

Thr Leu Met Ala Ala Leu Leu His Asp Val Ile Glu Asp Thr Pro Ala
65                  70                  75                  80

Thr Tyr Gln Asp His Asp Met Glu Gln Leu Phe Gly Lys Ser Val Ala
                85                  90                  95

Glu Leu Val Glu Gly Val Ser Lys Leu Asp Lys Leu Lys Phe Arg Asp
            100                 105                 110

Lys Lys Glu Ala Gln Ala Glu Asn Phe Arg Lys Met Ile Met Ala Met
        115                 120                 125

Val Gln Asp Ile Arg Val Ile Leu Ile Lys Leu Ala Asp Arg Thr His
    130                 135                 140

Asn Met Arg Thr Leu Gly Ser Leu Arg Pro Asp Lys Arg Arg Arg Ile
145                 150                 155                 160

Ala Arg Glu Thr Leu Glu Ile Tyr Ser Pro Leu Ala His Arg Leu Cys
                165                 170                 175

Ile His His Ile Lys Thr Glu Leu Glu Glu Leu Gly Phe Glu Ala Leu
            180                 185                 190

Tyr Pro Asn Arg Tyr Arg Val Ile Lys Glu Val Val Lys Ala Ala Arg
        195                 200                 205

Gly Asn Arg Lys Glu Met Ile Gln Lys Ile Leu Ser Glu Ile Glu Gly
    210                 215                 220

Arg Leu Gln Glu Ala Gly Ile Pro Cys Arg Val Ser Gly Arg Glu Lys
225                 230                 235                 240

His Leu Tyr Ser Ile Tyr Cys Lys Met Val Leu Lys Glu Gln Arg Phe
                245                 250                 255

His Ser Ile Met Asp Ile Tyr Ala Phe Arg Val Ile Val Asn Asp Ser
            260                 265                 270

Asp Thr Cys Tyr Arg Val Leu Gly Gln Met His Ser Leu Tyr Lys Pro
        275                 280                 285

Arg Pro Gly Arg Val Lys Asp Tyr Ile Ala Ile Pro Lys Ala Asn Gly
    290                 295                 300

Tyr Gln Ser Leu His Thr Ser Met Ile Gly Pro His Gly Val Pro Val
305                 310                 315                 320

Glu Val Gln Ile Arg Thr Glu Asp Met Asp Gln Met Ala Glu Met Gly
                325                 330                 335

Val Ala Ala His Trp Ala Tyr Lys Glu His Gly Glu Thr Ser Thr Thr
            340                 345                 350

Ala Gln Ile Arg Ala Gln Arg Trp Met Gln Ser Leu Leu Glu Leu Gln
        355                 360                 365

Gln Ser Ala Gly Ser Ser Phe Glu Phe Ile Glu Ser Val Lys Ser Asp
    370                 375                 380

Leu Phe Pro Asp Glu Ile Tyr Val Phe Thr Pro Glu Gly Arg Ile Val
385                 390                 395                 400

Glu Leu Pro Ala Gly Ala Thr Pro Val Asp Phe Ala Tyr Ala Val His
                405                 410                 415

Thr Asp Ile Gly His Ala Cys Val Gly Ala Arg Val Asp Arg Gln Pro
            420                 425                 430

Tyr Pro Leu Ser Gln Pro Leu Thr Ser Gly Gln Thr Val Glu Ile Ile
        435                 440                 445

Thr Ala Pro Gly Ala Arg Pro Asn Ala Ala Trp Leu Asn Phe Val Val
    450                 455                 460

Ser Ser Lys Ala Arg Ala Lys Ile Arg Gln Leu Leu Lys Asn Leu Lys
465                 470                 475                 480

Arg Asp Asp Ser Val Ser Leu Gly Arg Arg Leu Leu Asn His Ala Leu
                485                 490                 495

Gly Gly Ser Arg Lys Leu Asn Glu Ile Pro Gln Glu Asn Ile Gln Arg
            500                 505                 510

Glu Leu Asp Arg Met Lys Leu Ala Thr Leu Asp Asp Leu Leu Ala Glu
        515                 520                 525

Ile Gly Phe Gly Asn Ala Met Ser Val Val Val Ala Lys Asn Leu Gln
    530                 535                 540

His Gly Asp Ala Ser Ile Pro Pro Ala Thr Gln Ser His Gly His Leu
545                 550                 555                 560

Pro Ile Lys Gly Ala Asp Gly Val Leu Ile Thr Phe Ala Lys Cys Cys
                565                 570                 575

Arg Pro Ile Pro Gly Asp Pro Ile Ile Ala His Val Ser Pro Gly Lys
            580                 585                 590

Gly Leu Val Ile His His Glu Ser Cys Arg Asn Ile Arg Gly Tyr Gln
        595                 600                 605

Lys Glu Pro Glu Lys Phe Met Ala Val Glu Trp Asp Lys Glu Thr Ala
    610                 615                 620

Gln Glu Phe Ile Thr Glu Ile Lys Val Glu Met Phe Asn His Gln Gly
625                 630                 635                 640

Ala Leu Ala Asn Leu Thr Ala Ala Ile Asn Thr Thr Thr Ser Asn Ile
                645                 650                 655

Gln Ser Leu Asn Thr Glu Glu Lys Asp Gly Arg Val Tyr Ser Ala Phe
            660                 665                 670

Ile Arg Leu Thr Ala Arg Asp Arg Val His Leu Ala Asn Ile Met Arg
        675                 680                 685

Lys Ile Arg Val Met Pro Asp Val Ile Lys Val Thr Arg Asn Arg Asn
    690                 695                 700

<210>  9
<211>  32
<212>  DNA
<213>  Escherichia coli

<220>
<221>  misc_feature
<222>  (1)..(32)
<223>  Primer ligB-up

<400>  9
cagtactcta gaagccacga aggacactaa gg                                32

<210>  10
<211>  32
<212>  DNA
<213>  Escherichia coli

<220>
<221>  misc_feature
<222>  (1)..(32)
<223>  Primer ligB-down

<400>  10
ttagttggta cccggatgga ccgcagttaa tg                                32

<210>  11
<211>  1045

<212>  DNA
<213>  Escherichia coli


<220>
<221>  misc_feature
<222>  (1)..(1045)
<223>  PCR-Produkt ligB

<400>  11
cagtactcta gaagccacga aggacactaa ggttcaaaac ctgtgatctg ctgggcagcc      60

agccaactgc ccagcttctt gatttgcgca ttttccttcc attcaataac ctgtctggcg     120

cgtcccgatc cagtccccgg cagctgctgc cagaactgct ccgtgctaaa taaaagttgc     180

gaccaggacc gttcatcact ggcattaagc gccgcccggg ttagcggtat tcccattgcc     240

atcacccagc gagtaaaagg ctgcttacga gccagattaa actgatgcca tagctgcgca     300

cttttacttt tcgcgatccc cggcgtgttc tgtaattgct ctggcgttaa taaaagccag     360

gaaaagatat gttcaaagcg atgagtctga tgcagcgcgc gccaaccggc ctcaccaatg     420

ccatccagcc caagaacctg ttttgccccc agccagacta agcgtgaaat gaactgttcc     480

tgacaaacat cagaagcaaa gtagcaggtc aacgagttaa agcggttttc tggcggtgtc     540

ggttttgtac gttctgcacc gcgccacacc acatcatcaa tgcgaggaat accctgaccg     600

gcaaggctga cgagaatctg atcaccaggc gcaatatccc actcctgcca gcgcctgacg     660

gaaccaatat tcacccgctg gactttttta tcatccagca tgacaggtgc gagtgacgca     720

accaccgata ttttaccgct cttacccacc gcaaactgaa ttgccttcac ttcggcaacc     780

tgagctacag gttgatattt ccaggccacc agccactctg cctggcccgg tagccaatgg     840

cgggattctg gctctttcgc cgctcgtaca actacgccat cggtgacgaa gggtaattcc     900

gctttccacc actcattgcg tacgcgcgca acttcatcag catttttcac cgcacgggta     960

tacgtctgcg ttagagtaaa acctgcggta gccagctctt ttaaacgatc agacattaac    1020

tgcggtccat ccgggtacca actaa                                          1045


<210>  12
<211>  38
<212>  DNA
<213>  Escherichia coli


<220>
<221>  misc_feature
<222>  (1)..(38)
<223>  primer serCF(XbaI)

<400>  12
aggtgctcta gagtccgcgc tgtgcaaatc cagaatgg                              38


<210>  13
<211>  37
<212>  DNA

<213>  Escherichia coli

<220>
<221>  misc_feature
<222>  (1)..(37)
<223>  primer serCR(HindIII)

<400>  13
tacaccaagc ttaactctct acaacagaaa taaaaac                                37

<210>  14
<211>  33
<212>  DNA
<213>  Escherichia coli

<220>
<221>  misc_feature
<222>  (1)..(33)
<223>  primer serAF(XbaI)

<400>  14
ctgtagtcta gattagtaca gcagacgggc gcg                                    33

<210>  15
<211>  68
<212>  DNA
<213>  Escherichia coli

<220>
<221>  misc_feature
<222>  (1)..(68)
<223>  primer serAR(SHSSNB)

<400>  15
caagagctca agcttgcatg cgattcccgg gcggccgcaa taagatctcc gtcagggcgt       60

ggtgaccg                                                                68

<210>  16
<211>  34
<212>  DNA
<213>  Escherichia coli

<220>
<221>  misc_feature
<222>  (1)..(34)
<223>  primer serB(SphI)

<400>  16
ccatgcgcat gcccaccctt tgaaaatttg agac                                   34

<210>  17
<211>  75
<212>  DNA
<213>  Escherichia coli

<220>
<221>  misc_feature
<222>  (1)..(75)
<223>  primer serB(SmaI)

<400>  17
ccgcatgtcg acatcccggg gcagaaaggc ccacccgaag gtgagccagt gtgattactt        60

ctgattcagg ctgcc                                                          75


<210>  18
<211>  35
<212>  DNA
<213>  Escherichia coli


<220>
<221>  misc_feature
<222>  (1)..(35)
<223>  primer glyA-downstream

<400>  18
atctaaagat ctgttacgac agatttgatg gcgcg                                    35


<210>  19
<211>  33
<212>  DNA
<213>  Escherichia coli


<220>
<221>  misc_feature
<222>  (1)..(33)
<223>  primer glyA-upstream

<400>  19
ttcatcgcgg ccgcgaaaga atgtgatgaa gtg                                      33

## Beschreibung

Die vorliegende Erfindung betrifft eine Zelle, die ihrem Wildtyp gegenüber derartig genetisch verändert ist, dass sie eine relativ zu ihrem Wildtyp verringerte ppGppase-Aktvität aufweist und bevorzugt eine essentielle Aminosäure, noch bevorzugter eine von Serin abgeleitete essentielle Aminosäure, am bevorzugtesten Methionin oder Tryptophan, überproduziert, ein Futtermitteladditiv umfassend eine solche Zelle, ein Verfahren zur Herstellung einer eine von Serin abgeleitete essentielle Aminosäure, bevorzugt Methionin oder eine aromatischen essentiellen Aminosäure, insbesondere Tryptophan, überproduzierenden Zelle, umfassend Herstellen einer Zelle mit einem Knock out eines für eine ppGppase kodierenden Gens, sowie ein Verfahren zur Herstellung einer von Serin abgeleiteten essentiellen Aminosäure davon umfassend die Schritte a) Kultivieren der Zelle nach dem ersten Aspekt der vorliegenden Erfindung oder einer seiner Ausführungsformen und b) optional: Aufreinigen der Aminosäure.

Schwefelhaltige und aromatische L-Aminosäuren haben eine große wirtschaftliche Bedeutung. L-Cystein wird als Lebensmittelzusatz, als Ausgangsstoff für pharmakologische Wirkstoffe (z.B. N-Acetylcystein) und für Kosmetika verwendet. Die Aminosäuren L-Methionin und L-Tryptophan spielen eine herausragende Rolle in der Tierernährung. Sie gehören zu den essentiellen Aminosäuren, die im Stoffwechsel der Wirbeltiere nicht durch Biosynthese hergestellt werden können. Demzufolge muss bei der Tierzucht sichergestellt werden, dass die jeweilige Aminosäure in ausreichenden Mengen mit der Nahrung aufgenommen wird. Da zum Beispiel L-Methionin aber in klassischen Futtermittelpflanzen (wie Soja oder Getreide) insbesondere für Schweine und Geflügel häufig in zu geringen Mengen vorhanden ist, um eine optimale Tierernährung zu gewährleisten, ist es vorteilhaft, Methionin dem Tierfutter als Zusatzstoff beizumischen. D-Methionin kann von Wirbeltieren in biologisch aktives L-Methionin überführt werden. Daher wird dem Tierfutter meist ein Racemat aus D- und L-Methionin zugesetzt. L-Homocystein kann von Tieren durch Transmethylierung zu L-Methionin umgesetzt werden und dieses somit ersetzen.

Im Stand der Technik werden Aminosäuren wie Methionin durch chemische Synthese hergestellt. Dabei wird zunächst Acrolein und Methylmercaptan zu 3-Methylthiopropionaldehyd umgesetzt, das wiederum mit Cyanid, Ammoniak und Kohlenmonoxid zum Hydantoin führt. Dieses kann letztendlich zum Racemat, einem äquimolaren Gemisch der beiden Stereoisomeren D- bzw. L-Methionin hydrolysiert werden. Da die biologisch aktive Form des Moleküls ausschließlich die L-Form darstellt, muss die im Futter enthaltene D-Form im Stoffwechsel erst durch Des- und Transaminierung in die aktive L-Form überführt werden.

Im Gegensatz zu Methionin werden die meisten anderen natürlichen proteinogenen Aminosäuren wie zum Beispiel L-Tryptophan vorwiegend durch Fermentation von Mikroorganismen hergestellt. Dabei wird ausgenutzt, dass Mikroorganismen über entsprechende Biosynthesewege zur Synthese der natürlichen Aminosäuren verfügen. Zudem erreichen viele Fermentationsverfahren mit kostengünstigen Edukten wie Glucose und Mineralsalzen sehr günstige Herstellungskosten und liefern zudem die biologisch aktive L-Form der jeweiligen Aminosäure.

Es ist bekannt, dass derartige Aminosäuren durch Fermentation von Stämmen der *Enterobacteriaceae*, insbesondere *Escherichia coli* (*E. coli*) und *Serratia marcescens*, hergestellt werden. Wegen der großen Bedeutung wird ständig an der Verbesserung der Herstellverfahren gearbeitet. Verfahrensverbesserungen können fermentationstechnische Maßnahmen, wie z. B. Rührung und Versorgung mit Sauerstoff, oder die Zusammensetzung der Nährmedien wie zum Beispiel die Auswahl des verwendeten Zuckers oder die Zuckerkonzentration während der Fermentation, oder die Aufarbeitung zur Produktform, durch z. B. Ionenaustauschchromatographie, oder die intrinsischen Leistungseigenschaften des Mikroorganismus selbst betreffen.

Biosynthesewege von Aminosäuren unterliegen in Wildtyp-Stämmen einer strengen metabolischen Kontrolle, die gewährleistet, dass die Aminosäuren nur in dem für den Eigenbedarf der Zelle erforderlichen Maß hergestellt werden. Eine wichtige Voraussetzung für effiziente Produktionsprozesse ist es deshalb, dass geeignete Mikroorganismen verfügbar sind, die im Gegensatz zu Wildtyp-Organismen eine drastisch über den Eigenbedarf hinaus gesteigerte Produktionsleistung (Überproduktion) für die Herstellung der gewünschten Aminosäure aufweisen.

Solche Aminosäure-überproduzierenden Mikroorganismen können durch klassische Mutations-/Selektionsverfahren und/oder durch moderne, gezielte, rekombinante Techniken ("metabolic engineering") erzeugt werden. Bei letzterem werden zunächst Gene oder Allele identifiziert, die durch ihre Veränderung, Aktivierung oder Inaktivierung eine Aminosäure-Überproduktion bewirken. Diese Gene/Allele werden dann durch molekularbiologische Techniken in einen Mikroorganismenstamm eingebracht oder inaktiviert, so dass eine optimale Überproduktion erzielt wird. Häufig führt jedoch erst die Kombination mehrerer, verschiedener Maßnahmen zu einer wirklich effizienten Produktion. Die Gene oder Allele können für Enzyme, Transkriptionsfaktoren, Transporter und weitere Polypeptide, Cofaktoren oder Cofaktoren synthetisierende Polypeptide oder Komponenten kodieren, die Einfluß auf die Synthese der interessierenden Aminosäure nehmen können. In einer bevorzugten Ausführungsform wird unter dem Begriff "Komponenten", wie hierin verstanden, eine Untereinheit eines solchen, die Synthese der interessierenden Aminosäure beeinflussenden Polypeptides verstanden, die zwar kleiner ist als das gesamte vom Gen oder Operon kodierte System , beispielsweise eine Untereinheit oder eine Domäne eines Polypeptides, aber mit Hinblick auf die Aufgabe des Gesamtsystems funktionell ist.

L-Methionin leitet sich von Aspartat und Serin ab. Schwefel wird in E. coli in Form von L-Cystein (über Cystathionin als Zwischenprodukt) durch Transsulfurierung in L-Methionin eingebracht. Die CH₃-Gruppe des L-Methionins stammt aus dem C1-Stoffwechsel und sie wird von den Methionin-Synthasen MetE oder MetH auf L-Homocystein übertragen (Review: Greene RC (1996) in Neidhardt FC et al. (Hrsg.) "Escherichia coli and Salmonella", 2. Ausgabe, S. 542-560). Stämme und Verfahren zur fermentativen Produktion von L-Methionin wurden für *E*. *coli* z.B. in der WO2006/001616 oder WO2009/043803 beschrieben.

Der Shikimat-Stoffwechselweg zur Synthese von u.a. Chorismat, Enterobactin, Menaquinon, Tetrahydrofolat, Ubiquinon und den aromatischen Aminosäuren ist ebenfalls stark reguliert. Die Aromatenbiosynthese wird in *E*. *coli* sowohl auf genetischer Ebene durch Attenuation und Repression, wie auch durch Endprodukthemmung von Enzymen (Frost und Draths, Annual review of microbiology, 49:557-79 (1995); Pittard, Genes to Cells 1 (8):717-25 (1996)) reguliert. Über diesen Weg wird die Produktion aromatischer Metabolite genau an den Bedarf der Zelle angepasst. Der Shikimisäureweg wird vor allem durch die Steuerung der Eingangsreaktion, die durch drei verschiedene Isoenzyme der 3-Desoxy-D-Arabino-Heptulosonat-7-Phosphat-Synthase (DAHP-Synthase, kodiert durch die Gene aroF, aroG und aroH) katalysiert wird, reguliert. Das AroG-Genprodukt macht in *E*. *coli* 80% der Gesamtaktivität aller DAHP-Synthasen aus (Tribe et al., Journal of Bacteriology 127(3): 1085-1097 (1976); Pittard, Genes to Cells 1(8):717-25 (1996)); AroG wird durch die aromatische Aminosäure L-Phenylalanin zu 95% inhibiert. Auf genetischer Ebene reprimiert der Regulator TyrR in Anwesenheit von Phenylalanin und Tryptophan die Transkription von aroG (Baseggio et al., Journal of Bacteriology 172(5):2547-57 (1990); Davies et al., Gene 33(3):323-31 (1985)). In den Patentanmeldungen EP 1 270 721 und EP 2 147 972 wird die förderliche Wirkung der Verwendung eines aroG-Allels auf die Produktion und Herstellung der aromatischer Aminosäuren L-Phenylalanin und L-Tryptophan mit Stämmen der Gattung *Escherichia* beschrieben.

Vor diesem Hintergrund besteht die der vorliegenden Erfindung zu Grunde liegende Aufgabe darin, eine gegenüber den im Stand der Technik beschriebenen Zellen verbesserte Zelle, Verfahren zur Herstellung einer solchen Zelle und Verfahren unter Verwendung einer solchen Zelle zur Überproduktion einer von Serin abgeleiteten essentiellen Aminosäure, bevorzugt von Methionin oder einer aromatischen essentiellen Aminosäure, insbesondere Tryptophan, bereitzustellen, wobei die Verbesserung Faktoren wie die intrazellulär erreichte Konzentration der überproduzierten Aminosäure, die Ausbeute der überproduzierten Aminosäure nach Aufbereitung der Kultur, die Wachstumseigenschaften der Zelle sowie die für die Überproduktion und Aufbereitung der Aminosäure benötigte Zeit und Zahl von Verfahrensschritten sowie den Ressourcenbedarf des Prozesses, beispielsweise mit Hinblick auf Zeit, Energie und Menge der eingesetzten Stämme und Edukte, betrifft.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

In einem ersten Aspekt wird die der Anmeldung zu Grunde liegende Aufgabe gelöst durch eine Zelle, die ihrem Wildtyp gegenüber derartig genetisch verändert ist, dass sie eine relativ zu ihrem Wildtyp verringerte ppGppase-Aktvität aufweist und bevorzugt eine essentielle Aminosäure, noch bevorzugter eine von Serin abgeleitete essentielle Aminosäure, am bevorzugtesten Methionin oder Tryptophan, überproduziert.

In einer ersten Ausführungsform des ersten Aspekts wird das Problem gelöst durch eine Zelle, die eine relativ zu ihrem Wildtyp im Wesentlichen unveränderte (p)ppGpp-Synthetase-Aktivtität aufweist.

Im Rahmen der vorliegenden Erfindung bedeutet "eine Zelle, die eine relativ zu ihrem Wildtyp im _Wesentlichen unveränderte (p)ppGpp-Synthetase-Aktivtität aufweist", dass diese Zelle unter vergleichbaren Bedingungen wenigstens 90, 95, 99 , 101, 105, 110 % der Aktivität, gemessen als Zahl der pro Zeit synthetisierten (p)ppGpp-Moleküle, einer Wildtyp-Zelle aufweist.

In einer zweiten Ausführungsform des ersten Aspekts, die auch eine Ausführungsform der ersten Ausführungsform darstellt, wird das Problem gelöst durch eine Zelle, die ihrem Wildtyp gegenüber derartig genetisch verändert ist, dass sie eine relativ zu ihrem Wildtyp verringerte Aktivität wenigstens einer ppGppase aufweist, die aus der Gruppe ausgewählt ist, welche die Aminosäuresequenzen SEQ ID NO:2, SEQ ID NO:4 und SEQ ID NO:6 sowie Varianten davon umfasst, und es sich bevorzugt um SEQ ID NO:2 oder Varianten davon handelt.

In einer dritten Ausführungsform des ersten Aspekts, die auch eine Ausführungsform der ersten bis zweiten Ausführungsform darstellt, wird das Problem gelöst durch eine Zelle, wobei die Aktivität wenigstens einer ppGppase aus der Gruppe umfassend die Aminosäuresequenzen SEQ ID NO:2, SEQ ID NO:4 und SEQ ID NO:6 sowie Varianten davon, bevorzugt SEQ ID NO:2 oder Varianten davon, dadurch verringert ist, dass diese ppGppase wenigstens eine der folgenden Modifikationen aufweist:
a) das Gly174 oder eine dazu homologe Aminosäure ist gegen eine andere Aminosäure ausgetauscht, bevorzugt nicht konservativ,
b) das Leu529 oder eine dazu homologe Aminosäure ist gegen eine andere Aminosäure ausgetauscht, bevorzugt nicht konservativ,
c) zwischen Asp84 und Met85 oder dazu homologen Aminosäuren liegt eine Insertion von wenigstens einer Aminosäure, bevorzugt wenigstens zwei Aminosäuren, bevorzugt von His und Asn, vor,
wobei bevorzugt alle drei Mutationen vorliegen.

In einer vierten Ausführungsform des ersten Aspekts, die auch eine Ausführungsform der dritten Ausführungsform darstellt, wird das Problem gelöst durch eine Zelle, wobei die ppGppase zusätzlich wenigstens eine Modifikation aus der Gruppe aufweist, die Austausche, bevorzugt nicht konservative Austausche, an den Aminosäuren Gln9, Thr13, Tyr63, Arg109, Gln225, Cys245, Val248, Asn268, Ser270, Met280, His344, Pro436, Asn501, Gln505, His543, Ala546, Ser547, Ile548, His555, Gly556, His557, Pro559, Lys619, Thr621, Ala622, Thr627, Thr651, Ala669, Ala675, Thr698 und eine Insertion zwischen Glu343 und His344 oder einer dazu jeweils homologen Aminosäure umfasst.

In einer fünften Ausführungsform des ersten Aspekts, die auch eine Ausführungsform der dritten und vierten Ausführungsform darstellt, wird das Problem gelöst durch eine Zelle, wobei die ppGppase wenigstens eine Modifikation aus der Gruppe aufweist, die folgende Austausche oder Austausche von zur auszutauschenden Aminosäure homologen Aminosäuren gegen die gleichen Aminosäuren aufweist:
1). Austausch von Gln9 gegen eine Aminosäure ausgewählt aus der Gruppe Leu, Ile und Val, bevorzugt Leu,
2). Austausch von Thr13 gegen eine Aminosäure ausgewählt aus der Gruppe Lys, Arg, His, Gln und Asn, bevorzugt Arg oder Asn,
3). Austausch von Tyr63 gegen eine Aminosäure ausgewählt aus der Gruppe Lys, Arg und His, bevorzugt His,
4). Austausch des Arg109 gegen eine Aminosäure ausgewählt aus der Gruppe Gln und Asn, bevorzugt Gln,
5). Austausch von Gln225 gegen eine Aminosäure ausgewählt aus der Gruppe Ser, Ala und Thr, bevorzugt Thr,
6). Austausch des Cys245 gegen eine Aminosäure ausgewählt aus der Gruppe Leu, Ile und Val, bevorzugt Leu,
7). Austausch des Val248 gegen eine Aminosäure ausgewählt aus der Gruppe Lys, Arg und His, bevorzugt His,
8). Austausch des Asn268 gegen eine Aminosäure ausgewählt aus der Gruppe Lys, Arg und His, bevorzugt L-His oder Lys,
9). Austausch des Ser270 gegen eine Aminosäure ausgewählt aus der Gruppe Ala, Leu, Ile und Val, bevorzugt Ala oder Val,
10). Austausch des Met280 gegen eine Aminosäure ausgewählt aus der Gruppe Ser, Thr und Ala, bevorzugt Ala,
11). Insertion der Aminosäuren Lys und Glu zwischen Aminosäure Glu343 und His344,
12). Austausch des His344 gegen eine Aminosäure ausgewählt aus der Gruppe Gln und Asn, bevorzugt Gln,
13). Austausch des Pro436 gegen eine Aminosäure ausgewählt aus der Gruppe Ser, Ala und Thr, bevorzugt Ser,
14). Austausch des Asn501 gegen eine Aminosäure ausgewählt aus der Gruppe Ser, Ala und Thr, bevorzugt L-Alanin,
15). Austausch des Gln505 gegen eine Aminosäure ausgewählt aus der Gruppe Pro, Ser, Ala und Thr, bevorzugt Pro oder Ala,
16). Austausch des His543 gegen eine Aminosäure ausgewählt aus der Gruppe Asn und Gln, bevorzugt Gln,
17). Austausch des Ala546 gegen eine Aminosäure ausgewählt aus der Gruppe Asn und Gln, bevorzugt Gln,
18). Austausch des Ser547 gegen eine Aminosäure ausgewählt aus der Gruppe Ala, Leu, Ile und Val, bevorzugt Ala oder Val,
19). Austausch des Ile548 gegen eine Aminosäure ausgewählt aus der Gruppe Asn und Gln, bevorzugt Asn,
20). Austausch des His555 gegen eine Aminosäure ausgewählt aus der Gruppe Leu, Ile und Val, bevorzugt Val,
21). Austausch des Gly556 gegen Lys, Arg und His, bevorzugt Arg,
22). Austausch des His557 gegen eine Aminosäure ausgewählt aus der Gruppe Asn und Gln, bevorzugt Gln,
23). Austausch der Pro559 gegen eine Aminosäure ausgewählt aus der Gruppe Ser, Ala und Thr, bevorzugt Ala,
24). Austausch des Lys619 gegen eine Aminosäure ausgewählt aus der Gruppe Asn und Gln, bevorzugt Gln,
25). Austausch des Thr621 gegen eine Aminosäure ausgewählt aus der Gruppe Leu, Ile und Val, bevorzugt Ile,
26). Austausch des Ala622 gegen eine Aminosäure ausgewählt aus der Gruppe Glu und Asp, bevorzugt Asp,
27). Austausch des Thr627 gegen eine Aminosäure ausgewählt aus der Gruppe Ala und Gly, bevorzugt Ala,
28). Austausch des Thr651 gegen eine Aminosäure ausgewählt aus der Gruppe Glu und Asp, bevorzugt Glu,
29). Austausch des Ala669 und/oder Ala675 gegen Thr,
30). Austausch des Thr698 gegen eine Aminosäure ausgewählt aus der Gruppe Gln und Asn, bevorzugt Asn.

In einer sechsten Ausführungsform des ersten Aspekts, die auch eine Ausführungsform der ersten bis fünften Ausführungsform darstellt, wird das Problem gelöst durch eine Zelle, die ihrem Wildtyp gegenüber derartig gentechnisch verändert ist, dass sie relativ zu ihrem Wildtyp wenigstens eine Nukleinsäuresequenz oder eine Variante davon überexprimiert, die für eines der folgenden Enzyme, Komponenten davon oder für Varianten der Enzyme oder Komponenten davon kodiert:
1) Thiosulfat-/Sulfat-Transportsystems CysPUWA (EC 3.6.3.25),
2) 3'-Phosphoadenosin 5'-Phosphosulfat-Reduktase CysH (EC 1.8.4.8),
3) Sulfit-Reduktase CysJI (EC 1.8.1.2),
4) Cystein-Synthase A CysK (EC 2.5.1.47),
5) Cystein-Synthase B CysM (EC 2.5.1.47),
6) Serin-Acetyltransferase CysE (EC 2.3.1.30),
7) Glycin-Cleavage-System GcvTHP-Lpd (EC 2.1.2.10, EC 1.4.4.2, EC 1.8.1.4),
8) Lipoyl-Synthase LipA (EC 2.8.1.8),
9) Lipoyl-Protein-Ligase LipB (EC 2.3.1.181),
10) Phosphoglycerat-Dehydrogenase SerA (EC 1.1.1.95),
11) 3-Phosphoserin-Phosphatase SerB (EC 3.1.3.3),
12) 3-Phosphoserin/Phosphohydroxythreonin-Aminotransferase SerC (EC 2.6.1.52),
13) Serin-Hydroxymethyltransferase GlyA (EC 2.1.2.1),
14) Aspartokinase I- und Homoserin-Dehydrogenase I ThrA (EC 2.7.2.4, EC 1.1.1.3),
15) Aspartatkinase LysC (EC 2.7.2.4),
16) Homoserin-Dehydrogenase Hom (EC 1.1.1.3),
17) Homoserin O-Acetyltransferase MetX (EC 2.3.1.31),
18) Homoserin O-Succinyltransferase MetA (EC 2.3.1.46),
19) Cystathionin gamma-Synthase MetB (EC 2.5.1.48),
20) β-C-S-Lyase AecD (EC 4.4.1.8, auch als beta-Lyase bezeichnet),
21) Cystathionin beta-Lyase MetC (EC 4.4.1.8),
22) B12-unabhängige Homocystein S-Methyltransferase MetE (EC 2.1.1.14),
23) B12-abhängige Homocystein S-Methyltransferase MetH (EC 2.1.1.13),
24) Methylentetrahydrofolat-Reduktase MetF (EC 1.5.1.20),
25) L-Methionin-Exporter BrnFE aus *Corynebacterium glutamicum*,
26) Valinexporter YgaZH aus *Escherichia coli,* bevorzugt einer, der durch die Datenbankcodes b2682, b2683 dargestellt ist oder Varianten davon).
27) putativer Transporter YjeH aus *Escherichia coli,* bevorzugt einer, der durch den Datenbankcode b4141 dargestellt ist oder Varianten davon,
28) Pyridinnucleotid-Transhydrogenase PntAB (EC 1.6.1.2),
29) O-Succinylhomoserin-Sulfhydrylase MetZ (EC 2.5.1.48),
30) Phosphoenolpyruvat-Carboxylase Pyc (EC 4.1.1.31),
31) Thiosulfat-Sulfurtransferase RDL2 (EC 2.8.1.1),
32) Thiosulfat-Thiol-Sulfurtransferase (EC 2.8.1.3),
33) Thiosulfat-Dithiol-Sulfurtransferase (EC 2.8.1.5).

In einer siebten Ausführungsform des ersten Aspekts, die auch eine Ausführungsform der ersten bis sechsten Ausführungsform darstellt, wird das Problem gelöst durch eine Zelle, die ihrem Wildtyp gegenüber derartig gentechnisch verändert ist, dass sie relativ zu ihrem Wildtyp wenigstens eine Nukleinsäuresequenz oder eine Variante davon in einem verringerten Ausmaß exprimiert, die für eines der folgenden Enzyme, Komponenten davon oder für Varianten der Enzyme oder Komponenten davon kodiert:
1) Transkriptionsregulator der L-Methioninbiosynthese (MetJ), bevorzugt einer, der dargestellt ist durch die Datenbankcodes b3938, ECK3930 oder Varianten davon,
2) Glucose-6-Phosphat-Isomerase (Pgi, EC Nr. 5.3.1.9), bevorzugt eine, die dargestellt ist durch die Datenbankcodes b4025, ECK4017 oder Varianten davon,
3) Homoserinkinase (ThrB, EC 2.7.1.39), bevorzugt eine, die dargestellt ist durch die Datenbankcodes b0003, ECK0003 oder Varianten davon,
4) S-Adenosylmethionin-Synthase (MetK, EC Nr. 2.5.1.6) bevorzugt eine, die dargestellt ist durch die Datenbankcodes b2942, ECK2937 oder Varianten davon,
5) Dihydrodipicolinat-Synthase (DapA, EC Nr. 4.2.1.52) bevorzugt eine, die dargestellt ist durch die Datenbankcodes b2478, ECK2474 oder Varianten davon,
6) Phosphoenolpyruvat-Carboxykinase (Pck, EC Nr. 4.1.1.49), bevorzugt eine, die dargestellt ist durch die Datenbankcodes b3403, ECK3390 oder Varianten davon,
7) Formyltetrahydrofolat-Hydrolase (PurU, EC Nr. 3.5.1.10), bevorzugt eine, die dargestellt ist durch die Datenbankcodes b1232, ECK1227 oder Varianten davon,
8) Pyruvatkinase II (PykA, EC Nr. 2.7.1.40), bevorzugt eine, die dargestellt ist durch die Datenbankcodes b1854, ECK1855 oder Varianten davon,
9) Pyruvatkinase I (PykF, EC 2.7.1.40), bevorzugt eine, die dargestellt ist durch die Datenbankcodes b1676, ECK1672 oder Varianten davon,
10) Untereinheit des L-Methionintransporters (MetQNI), bevorzugt einer der dargestellt ist durch die Datenbankcodes b0197, ECK0197 oder Varianten davon,
11) Untereinheit des L-Methionintransporters (MetQNI), bevorzugt einer der dargestellt ist durch die Datenbankcodes b0198, ECK0198 oder Varianten davon,
12) Untereinheit des L-Methionintransporters (MetQNI), bevorzugt einer der dargestellt ist durch die Datenbankcodes b0199, ECK0199 oder Varianten davon,
13) Deoxycytidin 5'-Triphosphat Deaminase (Dcd, EC Nr. 3.5.4.13), bevorzugt eine, die dargestellt ist durch die Datenbankcodes b2065, ECK2059 oder Varianten davon,
14) putative N-Acyltransferase (YncA), bevorzugt eine, die dargestellt ist durch die Datenbankcodes b1448, ECK1442 oder Varianten davon,
15) regulatorische sRNA FnrS, bevorzugt eine, die dargestellt ist durch die Datenbankcodes b4699, ECK4511 oder Varianten davon,
16) Sigma-Faktor RpoS, bevorzugt einer, der dargestellt ist durch die Datenbankcodes b2741, ECK2736 oder Varianten davon.

In einer achten Ausführungsform des ersten Aspekts, die auch eine Ausführungsform der ersten bis fünften Ausführungsform darstellt, wird das Problem gelöst durch eine Zelle, die ihrem Wildtyp gegenüber derartig gentechnisch verändert ist, dass sie relativ zu ihrem Wildtyp wenigstens eine Nukleinsäuresequenz oder eine Variante davon überexprimiert, die für eines der folgenden Enzyme, Komponenten davon oder für Varianten der Enzyme oder Komponenten davon kodiert:
1) Anthranilat-Synthase (trpDE, EC Nr. 4.1.3.27), Anthranilat-Phosphoribosyltransferase (trpD, EC Nr. 2.4.2.18), Phosphoribo-sylanthranilat-Isomerase (trpC, EC Nr. 5.3.1.24), Indol-3-Glycerinphosphat-Synthase (trpC, EC Nr. 4.1.1.48) und die Tryptophan-Synthase (trpAB, EC Nr. 4.1.2.8 und 4.2.1.122),
2) Phosphoglycerat Dehydrogenase SerA (EC 1.1.1.95),
3) 3-Phosphoserin Phosphatase SerB (EC 3.1.3.3),
4) 3-Phosphoserin/Phosphohydroxythreonin-Aminotransferase SerC (EC 2.6.1.52),
5) L-Tyrosin-sensitive DHAP-Synthase (aroF, EC 2.5.1.54),
6) L-Phenylalanin-feedbackresistente DHAP-Synthase (aroG, EC 2.5.1.54),
7) L-Tryptophan-sensitive DHAP-Synthase (aroH, EC 2.5.1.54),
8) Phosphoenolpyruvat-Synthase ppsA (EC 2.7.9.2),
9) Phosphoenolpyruvat-Carboxykinase pck (EC 4.1.1.49),
10) Transketolase A tktA (EC 2.2.1.1),
11) Transketolase B tktB (EC 2.2.1.1),
12) das Genprodukt des offenen Leserahmens (ORF) yddG von *E*. *coli* , bevorzugt eines, das dargestellt ist durch die Datenbankcodes b1473, ECK1467 oder Varianten davon.

In einer neunten Ausführungsform des ersten Aspekts, die auch eine Ausführungsform der ersten bis fünften Ausführungsform darstellt, wird das Problem gelöst durch eine Zelle, die ihrem Wildtyp gegenüber derartig gentechnisch verändert ist, dass sie relativ zu ihrem Wildtyp wenigstens eine Nukleinsäuresequenz oder eine Variante davon in einem verringerten Ausmaß exprimiert, die für eines der folgenden Enzyme, Komponenten davon oder für Varianten der Enzyme oder Komponenten davon kodiert:
1) Tryptophanase (tnaA, EC 4.1.99.1),
2) Repressor des trp-Operons (trpR), bevorzugt einen, der dargestellt ist durch die Datenbankcodes b4393, ECK4385 oder Varianten davon,
3) Chorismat-Mutase T oder Prephenat-Dehydrogenase (tyrA, EC 1.3.1.12),
4) Chorismat-Mutase P oder Prephenat-Dehydrogenase (pheA, EC 4.2.1.51),
5) Tryptophan-spezifisches Transportprotein mtr, bevorzugt eines, das dargestellt ist durch die Datenbankcodes b3161, ECK3149 oder Varianten davon,
6) Tryptophan-Permease (tnaB), bevorzugt eine, die dargestellt ist durch die Datenbankcodes b3709, ECK3702 oder Varianten davon,
7) Transporter für aromatische Aminosäuren (aroP), bevorzugt einer, der dargestellt ist durch die Datenbankcodes b0112, ECK0111 oder Varianten davon,
8) L-Serin-Deaminase (sdaA, EC 4.3.1.17),
9) Glukose-6-Phosphat-Isomerase (pgi, EC 5.3.1.9),
10) Tyrosin-Aminotransferase (thrB), bevorzugt eine, die dargestellt ist durch die Datenbankcodes b4054, ECK4046 oder Varianten davon,
11) Repressor des glp-Regulons (glpR), bevorzugt einer, der dargstellt ist durch die Datenbankcodes b3423, ECK3409 oder Varianten davon,
12) Sigma-Faktor RpoS (rpoS), bevorzugt einer, der dargestellt ist durch die Datenbankcodes b2741, ECK2736 oder Varianten davon.

In einer zehnten Ausführungsform des ersten Aspekts, die auch eine Ausführungsform der ersten bis neunten Ausführungsform darstellt, wird das Problem gelöst durch eine Zelle, bei der es sich um eine Zelle zur Überproduktion einer von Serin abgeleiteten essentiellen Aminosäure, bevorzugt von Methionin oder einer aromatischen essentiellen Aminosäure, insbesondere Tryptophan, handelt.

Das der vorliegenden Erfindung zu Grunde liegende Problem wird in einem zweiten Aspekt gelöst durch ein Futtermitteladditiv umfassend eine Zelle nach dem ersten Aspekt der vorliegenden Erfindung oder einer Ausführungsform davon.

Das der vorliegenden Erfindung zu Grunde liegende Problem wird in einem dritten Aspekt gelöst durch ein Verfahren zur Herstellung einer essentiellen Aminosäure, noch bevorzugter einer von Serin abgeleitete essentielle Aminosäure, am bevorzugtesten Methionin oder Tryptophan, überproduzierenden Zelle, umfassend Herstellen einer Zelle mit einem Knock out eines für eine ppGppase kodierenden Gens, bevorzugt ohne Verringerung der (p)ppGpp-Synthetase-Aktivtität relativ zu ihrem Wildtyp, wobei die ppGppase bevorzugt aus der Gruppe ausgewählt ist, die die Aminosäuresequenzen SEQ ID NO:2, SEQ ID NO:4 und SEQ ID NO:6 sowie Varianten davon umfasst, und es sich noch bevorzugter um SEQ ID NO:2 handelt, und Kultivieren der Zelle, optional Aufreinigen der Aminosäure.

Das der vorliegenden Erfindung zu Grunde liegende Problem wird in einem vierten Aspekt gelöst durch ein Verfahren zur Herstellung einer von Serin abgeleiteten essentiellen Aminosäure umfassend die Schritte
a) Kultivieren der Zelle nach dem ersten Aspekt der vorliegenden Erfindung oder einer seiner Ausführungsformen,
b) optional: Aufreinigen der Aminosäure.

In einer ersten Ausführungsform des vierten Aspekts wird das Problem dadurch gelöst, dass eine Zelle nach dem ersten Aspekt oder einer seiner Ausführungsformen verwendet wird und das Verfahren ein Verfahren zur Herstellung von Methionin ist.

In einer zweiten Ausführungsform des vierten Aspekts, bei der es sich auch um eine Ausführungsform der ersten Ausführungsform handelt, wird das Problem durch ein Verfahren gelöst, wobei eine Zelle nach dem ersten Aspekt oder seiner zweiten bis sechsten oder neunten bis zehnten Ausführungsform verwendet wird und das Verfahren ein Verfahren zu Herstellung einer aromatischen essentiellen Aminosäure, bevorzugt Tryptophan, ist.

In einer dritten Ausführungsform des vierten Aspektes, bei der es sich auch um eine Ausführungsform der ersten bis zweiten Ausführungsform handelt, wird das Problem gelöst durch ein Verfahren, wobei die Mikroorganismen in einem batch-Verfahren, einem fed batch-Verfahren, einem repeated fed batch-Verfahren oder einem kontinuierlichen Verfahren kultiviert werden.

In einer weiteren Ausführungsform des ersten, zweiten, dritten und vierten Aspektes, bei der es sich auch um eine Ausführungsform sämtlicher Ausführungsformen des ersten bis vierten Aspektes handelt, wird das Problem gelöst durch eine Zelle/ein Futtermitteladditiv bzw. durch ein Verfahren, wobei es sich bei der Zelle um eine bakterielle Zelle aus der Gattung der *Enterobacteriaceae* handelt.

In einer weiteren Ausführungsform des ersten, zweiten, dritten und vierten Aspektes, bei der es sich auch um eine Ausführungsform sämtlicher Ausführungsformen des ersten bis vierten Aspektes handelt, wird das Problem gelöst durch eine Zelle/ein Futtermitteladditiv bzw. durch ein Verfahren, wobei die Zelle aus der Gruppe von Gattungen ausgewählt ist, die *Escherichia*, *Erwinia*, *Providencia* und *Serratia* umfasst, und es sich bei der Zelle bevorzugt um eine Zelle aus der Gattung *Escherichia,* noch bevorzugter um E. *coli* handelt.

Das der Erfindung zu Grunde liegende Problem wird in einem fünften Aspekt gelöst durch ein isoliertes oder rekombinantes Nukleinsäuremolekül umfassend einen für eine ppGppase kodierenden Teil, wobei die ppGppase ihrem Wildtyp gegenüber derartig genetisch verändert ist, dass sie eine relativ zu ihrem Wildtyp verringerte ppGppase-Aktvität aufweist.

In einer ersten Ausführungsform des fünften Aspekts weist die von dem isolierten oder rekombinanten Nukleinsäuremolekül kodierte ppGppase eine relativ zu ihrem Wildtyp im Wesentlichen unveränderte (p)ppGpp-Synthetase-Aktivtität auf.

In einer zweiten Ausführungsform des fünften Aspekts, die auch eine Ausführungsform der ersten Ausführungsform darstellt, ist die von dem isolierten oder rekombinanten Nukleinsäuremolekül kodierte ppGppase aus der Gruppe ausgewählt, die die Aminosäuresequenzen SEQ ID NO:2, SEQ ID NO:4 und SEQ ID NO:6 sowie Varianten davon umfasst, wobei es sich bevorzugt um SEQ ID NO:2 oder eine Variante davon handelt.

In einer dritten Ausführungsform des fünften Aspekts, die auch eine Ausführungsform der zweiten Ausführungsform darstellt, ist die Aktivität der von dem isolierten oder rekombinanten Nukleinsäuremolekül kodierten ppGppase dadurch verringert, dass das Nukleinsäuremolekül für eine ppGppase kodiert, die gegenüber der entsprechenden Wildtypsequenz wenigstens eine der folgenden Modifikationen aufweist:
a) das Gly174 oder eine dazu homologe Aminosäure ist gegen eine andere Aminosäure ausgetauscht, bevorzugt nicht konservativ,
b) das Leu529 oder eine dazu homologe Aminosäure ist gegen eine andere Aminosäure ausgetauscht, bevorzugt nicht konservativ,
c) zwischen Asp84 und Met85 oder dazu homologen Aminosäuren liegt eine Insertion von wenigstens einer Aminosäure, bevorzugt wenigstens zwei Aminosäuren, bevorzugt von His und Asn, vor,
wobei bevorzugt alle drei Mutationen vorliegen.

In einer vierten Ausführungsform des fünften Aspekts, die auch eine Ausführungsform der ersten bis dritten Ausführungsform darstellt, ist die Aktivität der von dem isolierten oder rekombinanten Nukleinsäuremolekül kodierten ppGppase dadurch verringert, dass das isolierte oder rekombinante Nukleinsäuremolekül für eine ppGppase kodiert, die gegenüber der entsprechenden Wildtypsequenz wenigstens eine der folgenden Modifikationen aus der Gruppe aufweist, die Austausche, bevorzugt nicht konservative Austausche, an den Aminosäuren Gln9, Thr13, Tyr63, Arg109, Gln225, Cys245, Val248, Asn268, Ser270, Met280, His344, Pro436, Asn501, Gln505, His543, Ala546, Ser547, Ile548, His555, Gly556, His557, Pro559, Lys619, Thr621, Ala622, Thr627, Thr651, Ala669, Ala675, Thr698 und eine Insertion zwischen Glu343 und His344 oder einer dazu jeweils homologen Aminosäure umfasst.

In einer fünften Ausführungsform des fünften Aspekts, die auch eine Ausführungsform der ersten bis vierten Ausführungsform darstellt, ist die Aktivität der von dem isolierten oder rekombinanten Nukleinsäuremolekül kodierten ppGppase dadurch verringert, dass das isolierte oder rekombinante Nukleinsäuremolekül für eine ppGppase kodiert, die gegenüber der entsprechenden Wildtypsequenz wenigstens einen der folgenden Austausche aufweist:
1.) Austausch von Gln9 gegen eine Aminosäure ausgewählt aus der Gruppe Leu, Ile und Val, bevorzugt Leu,
2.) Austausch von Thr13 gegen eine Aminosäure ausgewählt aus der Gruppe Lys, Arg, His, Gln und Asn, bevorzugt Arg oder Asn,
3.) Austausch von Tyr63 gegen eine Aminosäure ausgewählt aus der Gruppe Lys, Arg und His, bevorzugt His,
4.) Austausch des Arg109 gegen eine Aminosäure ausgewählt aus der Gruppe Gln und Asn, bevorzugt Gln,
5.) Austausch von Gln225 gegen eine Aminosäure ausgewählt aus der Gruppe Ser, Ala und Thr, bevorzugt Thr,
6.) Austausch des Cys245 gegen eine Aminosäure ausgewählt aus der Gruppe Leu, Ile und Val, bevorzugt Leu,
7.) Austausch des Val248 gegen eine Aminosäure ausgewählt aus der Gruppe Lys, Arg und His, bevorzugt His,
8.) Austausch des Asn268 gegen eine Aminosäure ausgewählt aus der Gruppe Lys, Arg und His, bevorzugt L-His oder Lys,
9.) Austausch des Ser270 gegen eine Aminosäure ausgewählt aus der Gruppe Ala, Leu, Ile und Val, bevorzugt Ala oder Val,
10.) Austausch des Met280 gegen eine Aminosäure ausgewählt aus der Gruppe Ser, Thr und Ala, bevorzugt Ala,
11.) Insertion der Aminosäuren Lys und Glu zwischen Aminosäure Glu343 und His344,
12.) Austausch des His344 gegen eine Aminosäure ausgewählt aus der Gruppe Gln und Asn, bevorzugt Gln,
13.) Austausch des Pro436 gegen eine Aminosäure ausgewählt aus der Gruppe Ser, Ala und Thr, bevorzugt Ser,
14.) Austausch des Asn501 gegen eine Aminosäure ausgewählt aus der Gruppe Ser, Ala und Thr, bevorzugt L-Alanin,
15.) Austausch des Gln505 gegen eine Aminosäure ausgewählt aus der Gruppe Pro, Ser, Ala und Thr, bevorzugt Pro oder Ala,
16.) Austausch des His543 gegen eine Aminosäure ausgewählt aus der Gruppe Asn und Gln, bevorzugt Gln,
17.) Austausch des Ala546 gegen eine Aminosäure ausgewählt aus der Gruppe Asn und Gln, bevorzugt Gln,
18.) Austausch des Ser547 gegen eine Aminosäure ausgewählt aus der Gruppe Ala, Leu, Ile und Val, bevorzugt Ala oder Val,
19.) Austausch des Ile548 gegen eine Aminosäure ausgewählt aus der Gruppe Asn und Gln, bevorzugt Asn,
20.) Austausch des His555 gegen eine Aminosäure ausgewählt aus der Gruppe Leu, Ile und Val, bevorzugt Val,
21.) Austausch des Glycin an Position 556 gegen Lys, Arg und His, bevorzugt Arg,
22.) Austausch des His557 gegen eine Aminosäure ausgewählt aus der Gruppe Asn und Gln, bevorzugt Gln,
23.) Austausch der Pro559 gegen eine Aminosäure ausgewählt aus der Gruppe Ser, Ala und Thr, bevorzugt Ala,
24.) Austausch des Lys619 gegen eine Aminosäure ausgewählt aus der Gruppe Asn und Gln, bevorzugt Gln,
25.) Austausch des Thr621 gegen eine Aminosäure ausgewählt aus der Gruppe Leu, Ile und Val, bevorzugt Ile,
26.) Austausch des Ala622 gegen eine Aminosäure ausgewählt aus der Gruppe Glu und Asp, bevorzugt Asp,
27.) Austausch des Thr627 gegen eine Aminosäure ausgewählt aus der Gruppe Ala und Gly, bevorzugt Ala,
28.) Austausch des Thr651 gegen eine Aminosäure ausgewählt aus der Gruppe Glu und Asp, bevorzugt Glu,
29.) Austausch des Ala669 und/oder Ala675 gegen Thr,
30.) Austausch des Thr698 gegen eine Aminosäure ausgewählt aus der Gruppe Gln und Asn, bevorzugt Asn.

In einer sechsten Ausführungsform des fünften Aspekts, die auch eine Ausführungsform der ersten bis fünften Ausführungsform darstellt, wird das Problem gelöst durch ein isoliertes oder rekombinantes Nukleinsäuremolekül kodierend für die Sequenz SEQ ID NO 8 oder eine Variante davon.

In einer siebten Ausführungsform des fünften Aspekts, die auch eine Ausführungsform der ersten bis fünften Ausführungsform darstellt, wird das Problem gelöst durch ein isoliertes oder rekombinantes Nukleinsäuremolekül umfassend die Sequenz SEQ ID NO 7 oder eine Variante davon.

Das der Erfindung zu Grunde liegende Problem wird in einem sechsten Aspekt gelöst durch einen Vektor umfassend ein Nukleinsäuremolekül nach dem fünften Aspekt oder einer seiner Ausführungsformen.

Die Erfinder der vorliegenden Erfindung haben festgestellt, dass die Produktionsleistung einer essentielle Aminosäure, bevorzugter einer von Serin abgeleiteten essentiellen Aminosäure, am bevorzugtesten Methionin oder Tryptophan, überproduzierenden Zelle überraschend dadurch gesteigert ist, wenn die Zelle eine relativ zu ihrem Wildtyp verringerte ppGppase-Aktivität aufweist. Dies ist überraschend besonders dann der Fall, wenn die (p)ppGpp-Synthetase-Aktivität der Zelle im Wesentlichen relativ zu ihrem Wildtyp unverändert ist.

Eine essentielle Eigenschaft der erfindungsgemäßen Zelle besteht darin, dass sie ihrem Wildtyp gegenüber derartig genetisch verändert ist, dass sie eine relativ zu ihrem Wildtyp verringerte ppGppase-Aktvität aufweist. In einer bevorzugten Ausführungsform wird unter dem Begriff "ppGppase-Aktvität", wie hierin verwendet, die enzymatische Aktivität einer Guanosin-3',5'-bis(Diphosphat) 3'-Diphosphatase (EC-Nr. 3.1.7.2) verstanden, d.h. die Fähigkeit, den Abbau von Guanosin-3',5'-bis(Diphosphat) (=ppGpp) zu GDP und Pyrophosphat zu katalysieren. In einer bevorzugten Ausführungsform wird unter dem Begriff "ppGppase", wie hierin verwendet, ein Enzym mit ppGppase-Aktivität verstanden, wobei das Enzym ausschließlich ppGppase-Aktivität oder mehrere Aktivitäten, darunter ppGppase-Aktivität, aufweisen kann. ppGpp entsteht unter Einwirkung einer Guanosin-5'-Triphosphat, 3'-Diphosphat Pyrophosphatase (EC-Nr. 3.6.1.40) aus Guanosine 3'-Diphosphat 5'-Triphosphat (=pppGpp), einer Verbindung, die wiederum durch GTP-Diphosphokinasen (EC-Nr. 2.7.6.5, in der Literatur häufig mit PSI und PSII abgekürzt) aus ATP und GTP hergestellt wird. In einer bevorzugten Ausführungsform umfasst der Begriff "(p)ppGpp-Synthetase-Aktivität" die Fähigkeit, wie hierin verwendet, wenigstens eine, bevorzugt beide der beiden Verbindungen pppGpp oder ppGpp herzustellen, bevorzugt unter Hydrolyse von ATP. Im Stand der Technik sind zahlreiche ppGppasen und für ppGppasen kodierende Gene beschrieben, beispielsweise in Blattner *et al.* (Science 277: 1453-1462 (1997)) und in Datenbanken, beispielsweise NC_003197 (REGION: 3934070-3936181), NC_009832 (REGION: (5391421-5393532), NC_007613, NC_004741, NC_013971 und NC_009648. In einer bevorzugten Ausführungsform bedeutet die Formulierung, dass eine Zelle, die ihrem Wildtyp gegenüber derartig genetisch verändert ist, dass sie eine relativ zu ihrem Wildtyp verringerte ppGppase-Aktvität aufweist und "bevorzugt eine essentielle Aminosäure, noch bevorzugter eine von Serin abgeleitete essentielle Aminosäure, am bevorzugtesten Methionin oder Tryptophan, überproduziert", wie hierin verwendet, dass der der Zelle zu Grunde liegende Ausgangsstamm bereits eine seinem ursprünglichen Wildtypstamm gegenüber erhöhte Menge der entsprechenden Aminosäure produziert, noch vor und unabhängig von der Verringerung der ppGppase-Aktivität.

Die Aktivität eines Enzyms, beispielsweise einer ppGppase oder einer (p)ppGpp-Synthetase, kann der Fachmann anhand von Aktivitätstests bestimmen, wie sie zusammen mit Verfahren zur fachmännischen Auswertung und Interpretation der bei solchen Bestimmungen erhaltenen kinetischen Parameter im Stand der Technik beschrieben sind, beispielsweise Cornish-Bowden, Fundamentals of Enzym Kinetics, Portland Press Limited, 1995. Im Stand der Technik sind weiterhin spezifische Tests für die Bestimmung der Aktivität von (p)ppGpp-Synthetasen (Mechold et al., 1996, J. Bact. 178, 1401-1411) und ppGppase (Gallant et al., 1970, Cold Spring Harbor Symp. Quant. Biol. 35, 397) beschrieben. Kurz gesagt werden die Zellen mit der zu bestimmenden Aktivität dazu mit radioaktiv markierten Nukleotiden markiert, durch die Hinzugabe von 400 µg/ml Valin mit Hinblick auf Isoleucin ausgehungert, gefolgt von Extraktion des Nukleotidpools der Zelle nach 15 Minuten Valininhibition in 0,66 N Ameisensäure und anschließender Auftrennung von pppGpp, ppGpp, pppG und pppA durch Dünnschichtchromatographie auf PEI-Cellulose-Platten in 1,5 M Kaliumdihydrogenphosphat und Visualisierung durch Autoradiographie. In einer bevorzugten Ausführungsform bedeutet "eine Zelle, die ihrem Wildtyp gegenüber derartig genetisch verändert ist, dass sie eine relativ zu ihrem Wildtyp verringerte ppGppase-Aktvität aufweist", dass eine derartige Zelle unter vergleichbaren Bedingungen weniger ppGppase-Aktivität, gemessen als Zahl der pro Zeit hydrolysierten ppGpp-Moleküle, als eine Wildtypzelle aufweist, und zwar in der Reihenfolge zunehmender Bevorzugung weniger 70, 60, 40, 20, 10 oder 5 % der Aktivität einer Wildtyp-Zelle. In einer weiteren bevorzugten Ausführungsform bedeutet "eine Zelle, die eine relativ zu ihrem Wildtyp im _Wesentlichen unveränderte (p)ppGpp-Synthetase-Aktivtität aufweist", dass diese Zelle unter vergleichbaren Bedingungen wenigstens 90, 95, 99 , 101, 105, 110 % der Aktivität, gemessen als Zahl der pro Zeit synthetisierten (p)ppGpp-Moleküle, einer Wildtyp-Zelle aufweist. In einer besonders bevorzugten Ausführungsform handelt es sich bei der Zelle um einen Stamm von *E*. *coli,* dessen ppGppase-Aktivität der Sequenz SEQ ID NO. 2 oder einer Variante verringert ist, während die Aktivität der (p)ppGpp-Synthetase der Zelle im Wesentlichen unverändert ist.

Die der Lehre der vorliegenden Erfindung ist nicht auf Ausführungsformen beschränkt, bei denen die Aminosäure- oder Nukleinsäuresequenzen der dabei verwendeten biologischen Makromoleküle identisch zu den in dieser Anmeldung beschriebenen Sequenzen oder zu den hierin angegebenen oder zitierten Sequenzen des Standes der Technik sind, sondern die erfindungsgemäße Lehre umfasst auch Varianten derartiger Makromoleküle, die durch Deletion, Addition oder Substitution einer oder mehr als einer Aminosäuren oder Nukleinsäuren erhalten werden können sowie Fusionen umfassend derartige Makromoleküle oder Varianten davon. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die mit Hinblick auf die entsprechende ursprüngliche Wildtyp-Nukleinsäure- oder Aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder letztere lediglich konservativ substituiert sind. Der Stand der Technik lehrt, wie konservative und nichtkonservative Substitutionen von Aminosäuren beschaffen sind und herbeigeführt werden können, siehe z. B. Berg, Jeremy M., Tymoczko, John L., and Stryer, Lubert. Biochemistry. 6th ed. New York, N.Y.: W.H. Freeman and Company, 2007, und beschreibt Algorithmen, die verwendet werden können, um das Ausmaß von Homologie von zwei Sequenzen zu berechnen, z. B. Arthur Lesk (2008), Introduction to bioinformatics, 3rd edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatische Aktivität wie das Wildtypmolekül bzw. das ursprüngliche Molekül auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer weiteren bevorzugten Ausführungsform umfasst der Begriff "Variante" einer Nukleinsäure- oder Aminosäuresequenz wenigstens einen aktiven Teil / oder ein Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als (p)ppGpp-Synthetase. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Sonden mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktionen und entsprechende Bedingungen sind ausführlicher in F. M. Ausubel (1995), Current Protocols in Molecular Biology. John Wiley & Sons, Inc., beschrieben. Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet in einer bevorzugten Ausführungsform unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von in der Reihenfolge zunehmender Bevorzugung ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2 x SSC oder 0,1 x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise in der Reihenfolge zunehmender Bevorzugung mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1, SEQ ID No. 3 oder SEQ ID No. 5 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

Die Einführung von gezielten Mutationen in biologische Makromoleküle, gleichermaßen auf der Ebene der Nuklein- und Aminosäuren, gehört heutzutage zu den routinemäßig angewandten Standardmethoden der Molekularbiologie, Mikrobiologie und Biochemie. Beispielsweise können Verfahren der ortsgerichteten Mutagenese unter Verwendung mutagener Oligonukleotide (T. A. Brown: Gentechnologie für Einsteiger, Spektrum Akademischer Verlag, Heidelberg, 1993) oder der Polymerase-Kettenreaktion (PCR), wie sie im Handbuch von Gait: Oligonukleotide synthesis: A Practical Approach (IRL Press, Oxford, UK, 1984) oder von Newton und Graham (PCR, Spektrum Akademischer Verlag, Heidelberg, 1994) beschrieben sind, verwendet werden. Zur Konstruktion von Mutationen kann zum Beispiel das Quick Change Site-Directed Mutagenesis Kit der Firma Stratagene (Amsterdam, Niederlande) verwendet werden. Bei Verwendung dieser Methoden wird die Ausgangssequenz, beispielsweise eine im Stand der Technik beschriebene für eine ppGppase codierende Sequenz ausgehend von isolierter Gesamt-DNA eines Wildtypstammes mit Hilfe der Polymerase-Kettenreaktion (PCR) amplifiziert, in geeignete Plasmidvektoren kloniert, und die DNA anschließend dem Mutageneseverfahren unterworfen. Mittels "GeneSOEing" (Gene Splicing by Overlap Extension, Horton, Molecular Biotechnology 3: 93-98 (1995)) können die Punktmutationen schon per PCR erhalten werden. Auch eine *de novo* Gensynthese (z.B. durch die Firma GENEART AG, Regensburg, Deutschland) der Nukleotidsequenzen kann zur Herstellung von Mutationen im spoT-Gen verwendet werden. Die erzeugten Mutationen können durch DNA-Sequenzierung beispielsweise nach der Methode von Sanger et al. (Proc. Natl. Acad. Sci. 74 (12): 5463-5467, 1977) bestimmt und überprüft werden. Die erzeugten Allele können beispielsweise durch Transformation und das Verfahren des Gen- bzw. Allelaustausches in das Chromosom geeigneter Stämme eingebaut werden.

Eine gebräuchliche Methode ist die von Hamilton et al. (Journal of Bacteriology 174, 4617 - 4622 (1989)) beschriebene Methode des Genaustauschs mit Hilfe eines konditional replizierenden pSC101-Derivates pMAK705 oder mit pKO3 (Link et al., Journal of Bacteriology 179: 6228-6237). Andere im Stand der Technik beschriebene Methoden wie beispielsweise die von Martinez-Morales et al. (Journal of Bacteriology 1999, 7143-7148 (1999)) oder die von Boyd et al. (Journal of Bacteriology 182, 842-847 (2000)) können gleichfalls benutzt werden.

Eine weitere gebräuchliche Methode besteht darin, ein durch PCR oder Gensynthese erzeugtes DNA-Fragment über kurze, homologe Flankensequenzen direkt mit Hilfe der Lambda Red-Rekombinase in das Chromosom einzubauen, bzw. einen Austausch vorzunehmen (Proc. Natl. Acad. Sci. 97(12), 6640-6645 (2000); Nature Genetics 20, 123 - 128, 1998).

Es ist ebenfalls möglich, die erzeugten Allele durch Konjugation oder Transduktion in verschiedene Stämme zu überführen.

Zwar ist technisch die Einführung zahlreicher, nicht natürlich in Nukleinsäure- oder Aminosäuresequenzen vorkommender künstlicher Nukleotide bzw. Aminosäuren in biologische Makromoleküle denkbar, beispielsweise im Rahmen einer chemischen Komplettsynthese oder durch Veränderung des zellulären Translationsapparates, im Rahmen der vorliegenden Erfindung sind in einer bevorzugten Ausführungsform jedoch ausschließlich proteinogene L-Aminosäuren für Substitutionen zum Ersatz einer oder mehr als einer ursprünglich in dem entsprechenden Polypeptid vorkommenden Aminosäure vorgesehen. In einer bevorzugten Ausführungsform wird unter dem Begriff "proteinogene" Aminosäure eine jede Aminosäure aus der Gesamtheit der L-Formen der Aminosäuren Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin und Valin verstanden. In einer bevorzugten Ausführungsform wird unter dem Begriff "homologe" Aminosäure einer Aminosäure in der Wildtypsequenz eines Polypeptides, wie hierin verwendet, eine solche Aminosäure verstanden, die in einer anderen, aber mit der Wildtypsequenz mit Hinblick auf die Primärstruktur verwandten Aminosäuresequenz anhand eines Alignments der beiden Sequenzen als die Aminosäure identifiziert wird, die der Aminosäure in der Wildtypsequenz entspricht bzw. ihre entsprechenden Position in der verwandten Aminosäuresequenz einnimmt. Verfahren zur Durchführung eines Alignments zweier oder mehrerer Aminosäuresequenzen sind im Stand der Technik beschrieben, beispielsweise in Arthur Lesk (2008), Introduction to bioinformatics, 3rd edition, und umfassen die Verwendung von Software-Paketen wie Clustal W (Thompson et al., Nucleic Acids Research 22, 4637-4680, 1994) oder MAFFT (Katoh et al., Genome Information, 16(1), 22-33, 2005). Beispielsweise ist das Arg26 im von dem menschlichen Pax6-Gen kodierten Polypeptid homolog zum Arg59 des Drosophila-Gens eyless, vergleiche Lesk (2008), S. 36.

Die genetischen Methoden zur Herstellung der erfindungsgemäßen Mutanten umfasssen neben gentechnischen Methoden ebenso klassische genetische Methoden, wie in-vivo Mutageneseverfahren mit Zellpopulationen von Stämmen der *Enterobacteriaceae* unter Verwendung mutagener Stoffe wie beispielsweise N-Methyl-N'-Nitro-N-Nitrosoguanidin (MNNG), Ethylmethansulfonat (EMS), 5-Bromuracil oder ultraviolettes Licht. Mutagenesemethoden sind beispielsweise im Manual of Methods for General Bacteriology (Gerhard et al. (Eds.), American Society for Microbiology, Washington, DC, USA, 1981) oder bei Tosaka et al. (Agricultural and Biological Chemistry 42(4), 745-752 (1978)) oder bei Konicek et al. (Folia Microbiologica 33, 337-343 (1988)) beschrieben. Typische Mutagenesen unter Verwendung von MNNG umfassen Konzentrationen von 50 bis 500 mg/l oder auch höhere Konzentrationen bis zu maximal 1 g/l, eine Inkubationszeit von 1 bis 30 Minuten bei einem pH von 5,5 bis 7,5. Unter diesen Bedingungen wird die Zahl der lebensfähigen Zellen um einen Anteil von ca. 50% bis 90% oder ca. 50% bis 99% oder ca. 50% bis 99,9% oder mehr reduziert.

Aus der mutagenisierten Zellpopulation werden Mutanten bzw. Zellen entnommen und vermehrt. Vorzugsweise wird in einem weiteren Schritt deren Fähigkeit untersucht, in einer Satzkultur (batch) unter Verwendung eines geeigneten Nährmediums Aminosäuren, bevorzugt Methionin oder Tryptophan auszuscheiden. Bei Verwendungen von geeigneten Roboteranlagen, wie beispielsweise bei Zimmermann et al. (VDI Berichte Nr. 1841, VDI-Verlag, Düsseldorf, Deutschland 2004, 439-443) oder Zimmermann (Chemie Ingenenieur Technik 77 (4), 426-428 (2005)) beschrieben, können zahlreiche Mutanten in kurzer Zeit untersucht werden. Im Allgemeinen werden maximal 3.000, maximal 10.000, maximal 30.000 oder auch maximal 60.000 Mutanten gegebenenfalls auch mehr untersucht. Auf diese Weise werden Mutanten identifiziert, die im Vergleich zum Elternstamm bzw. nicht mutagenisierten Ausgangsstamm vermehrt Aminosäuren in das Nährmedium oder in das Zellinnere ausscheiden. Dazu gehören beispielsweise solche Mutanten, deren Aminosäuresekretion um mindestens 0,5% erhöht ist.

Anschließend wird aus den Mutanten DNA bereitgestellt, bzw. isoliert und mit Hilfe der Polymerase-Kettenreaktion unter Verwendung von Primerpaaren, die die Amplifizierung des spoT-Gens bzw. des erfindungsgemäßen spoT-Allels oder der erfindungsgemäßen Mutationen erlauben, das entsprechende Polynukleotid synthetisiert. Vorzugsweise wird die DNA aus solchen Mutanten isoliert, die in erhöhter Weise Aminosäuren ausscheiden.

In einer bevorzugten Ausführungsform bedeutet der Begriff "Gen", wie hierin verwendet, ein Abschnitt auf der Desoxyribonukleinsäure (DNA), der die Informationen zur Herstellung (Transkription) zunächst einer Ribonukleinsäure (RNA) und diese die Information zur Herstellung (Translation) eines Proteins (Polypeptids) enthält, hier ein Polypeptid mit der Aktivität einer (p)ppGpp Synthetase II. Die Tatsache, dass ein Gen oder ein Polynukleotid die Informationen zur Herstellung eines Proteins enthält, bezeichnet man auch als Kodierung eines Protein bzw. Polypeptides durch das Gen bzw. durch die RNA. Genexpression bezeichnet die Biosynthese von RNA und Proteinen aus den genetischen Informationen enthalten in DNA und Genen. Unter endogenen Genen beziehungsweise Polynukleotiden versteht man die in der Population einer Art vorhandenen offenen Leserahmen (ORF), Gene oder Allele beziehungsweise deren Polynukleotide. Die Begriffe "Gen" und "ORF" (offenen Leserahmen) werden in dieser Erfindung synonym verwendet.

In einer bevorzugten Ausführungsform wird unter dem Begriff "Polynukleotid", wie hierin verwendet, ein Polyribonukleotid und Polydeoxyribonukleotid verstanden, wobei es sich um nicht modifizierte RNA oder DNA oder modifizierte RNA oder DNA handeln kann und der Begriff Polynukleotid gleichbedeutend und austauschbar mit dem Begriff "Nukleinsäure" verwendet wird.

In einer bevorzugten Ausführungsform werden unter dem Begriff "Polypeptid", wie hierin verwendet, Peptide oder Proteine verstanden, die zwei oder mehr über Peptidbindungen verbundene Aminosäuren enthalten. Die Begriffe Polypeptid und Protein werden als Synonyme verwendet. Proteine gehören zu den Grundbausteinen aller Zellen. Sie verleihen der Zelle nicht nur Struktur, sondern sind die molekularen "Maschinen", die Stoffe transportieren, chemische Reaktionen katalysieren und Signalstoffe erkennen.

Dem Fachmann ist bekannt, dass der bei der Gestaltung einer Variante bzw. Mutante eines Polypeptides bzw. des kodierenden Gens mit hoher Wahrscheinlichkeit zu einem Polypeptid mit ähnlichen oder gar identischen Eigenschaften gelangt, wenn er eine Aminosäure in der Wildtypsequenz gegen eine Aminosäure konservativ austauscht. In einer bevorzugten Ausführungsform wird unter dem Begriff "konservativer" Austausch, wie hierin verwendet, verstanden, dass eine Aminosäure gegen eine proteinogene Aminosäure mit ähnlichen physikochemischen Eigenschaften ausgetauscht wird. In einer noch bevorzugteren Ausführungsform bedeutet dies, dass eine Aminosäure einer der folgenden Gruppen nur gegen eine andere Aminosäure aus der gleichen Gruppe ausgetauscht wird, wobei die Gruppen die Gruppe der kleinen Aminosäure umfassend Gly, Ala, Ser und Thr, die Gruppe der mittelgroßen unpolaren Aminosäuren umfassend Cys, Val, Ile und Leu, die Gruppe der großen unpolaren Aminosäuren umfassend Phe, Tyr, Met und Trp, die Gruppe der polaren Aminosäuren ohne Ladung an der Seitenkette umfassend His, Gln und Asn, die Gruppe der positiv geladenen Aminosäuren umfassend Lys und Arg und die Gruppe der negativ geladenen Aminosäuren umfassend Glu und Asp umfassen.

Die erfindungsgemäße Lehre kann grundsätzlich zur Herstellung einer jeden Aminosäure benutzt werden. In einer bevorzugten Ausführungsform ist unter dem Begriff "Aminosäure", wie hierin verwendet, eine organische Säure umfassend wenigstens eine Aminogruppe und eine Carboxygruppe, bevorzugt mit dem gleichen Kohlenstoffatom kovalent verbunden, zu verstehen, am bevorzugtesten die L-Form der Aminosäure. In einer bevorzugten Ausführungsform wird die erfindungsgemäße Zelle oder das erfindungsgemäße Verfahren zur Herstellung einer essentiellen und/oder von Serin abgeleiteten und/oder aromatischen Aminosäure eingesetzt. In einer bevorzugten Ausführungsform wird unter dem Begriff "essentielle" Aminosäure eine Aminosäure verstanden, die ein höherer Eukaryont, bevorzugt ein warmblütiger höherer Eukaryont, noch bevorzugter ein Vogel oder Säugetier, nicht eigenständig herstellen kann. In einer bevorzugteren Ausführungsform umfasst dies auch Aminosäuren, die der Organismus selbst herstellen kann, jedoch nur, wenn er eine geeignete Vorstufe, am bevorzugtesten eine andere Aminosäure, mit der Nahrung aufnimmt. In einer bevorzugten Ausführungsform wird unter dem Begriff "eine von Serin abgeleitete Aminosäure", wie hierin verwendet, eine Aminosäure verstanden, die über einen Biosyntheseweg hergestellt wird, der wenigstens eine Reaktion umfasst, bei der Serin als Edukt verbraucht wird, wobei zum Biosyntheseweg bevorzugt nur solche Reaktionen gezählt werden, die zum Aufgabe des Gerüstes der herzustellenden Aminosäure direkt beitragen, nicht jedoch Reaktionen, die lediglich Cofaktoren regenerieren.

Bei der erfindungsgemäßen oder in einem erfindungsgemäßen Verfahren hergestellten oder eingesetzten Zelle kann es sich um eine Zelle zahlreicher biotechnologisch einsetzbarer Organismen handeln. In einer bevorzugten Ausführungsform handelt es sich um eine prokaryotische oder niedere eukaryotische Zelle. In einer bevorzugten Ausführungsform wird unter dem Begriff "niedere eukaryotische Zelle", wie hierein verwendet, eine unizelluläre eukaryotische Zelle, bevorzugt eine Hefezelle verstanden. Beispiele für niedere eukaryotische Zellen umfassen Hefen der Gattungen *Saccharomyces*, *Schizosaccharomyces*, *Candida*, *Picchia* und *Yarrowia*. In einer weiteren bevorzugten Ausführungsform handelt es sich bei der Zelle um eine prokaryotische Zelle, bevorzugter um ein Gram-negatives Bakterium, noch bevorzugter um eine Zelle, die aus der Gruppe von Gattungen ausgewählt ist, welche *Escherichia*, *Erwinia*, *Providencia* und *Serratia* umfasst, noch bevorzugter um eine Zelle aus der Gattung *Escherichia,* und am bevorzugtesten um *E*. *coli*. In einer bevorzugten Ausführungsform handelt es sich um eine isolierte, in Reinkultur gehaltene Zelle. In einer weiteren Ausführungsform kommt eine Mischung von Kulturen zum Einsatz. In einer bevorzugtesten Ausführungsform handelt es sich bei der Zelle um ein Bakterium des Stammes EMC1_spoT1849, siehe Beispiel 4.

Bevorzugt liegt die erfindungsgemäße Zelle für die Herstellung wenigstens einer Aminosäure in intakter, unbeeinträchtigt stoffwechselaktiver Form vor. Die erfindungsgemäße Lehre kann jedoch auch unter Verwendung teilweise lysierter und teilaufgereinigter Zellen verwirklicht werden, deren Stoffwechsel noch zur Herstellung der Aminosäure(n) von Interesse befähigt ist. Im Falle der Verwendung der erfindungsgemäßen Zelle als Bestandteil eines Futtermitteladditivs ist jede Aufreinigungsstufe der Zelle, von der intakten Zelle über teilweise oder vollständig lysierte Zellen bis zur reinen Aminosäure möglich.

Besonders bevorzugt sind erfindungsgemäß solche Zellen, die bereits vor der eine verringerte ppGppase-Aktivität bewirkenden genetischen Veränderung, d.h. in Form des entsprechenden Ausgangsstammes, eine von Serin abgeleitete essentielle Aminosäure, bevorzugt Methionin oder Tryptophan, oder eine aromatische Aminosäure, insbesondere Tryptophan, eine ihrem der Natur entnommenen Wildtyp gegenüber erhöhte Menge der betreffenden Aminosäure produzieren. In einer bevorzugten Ausführungsform versteht man unter dem Begriff "Ausgangsstamm", austauschbar verwendet mit dem Begriff "Elternstamm", wie hierin verwendet, den Mikroorganismenstamm, an dem Maßnahmen zur Erhöhung der Produktivität einer oder mehrerer Aminosäuren, Peptide oder Proteine, bzw. Maßnahmen zur Erhöhung der Aktivität eines oder mehrerer Enzyme, z. B. eine zur Überexpression führende Maßnahme, durchgeführt werden. Bei einem Ausgangsstamm kann es sich um einen Wildtyp-Stamm, aber auch um einen bereits vorher veränderten Stamm handeln, beispielsweise um einen als Produktionsstamm verwendbaren, wenigstens eine L-Aminosäure überproduzierenden Stamm.

Im Stand der Technik sind zahlreiche entsprechende Aminosäuren überproduzierende Stämme sowie Maßnahmen und Modifikationen bekannt, mit denen eine solche Überproduktion erreicht werden kann. In einer bevorzugten Ausführungsform weist der L-Methionin ausscheidende oder produzierende Stamm des *E*. *coli* bevorzugt eine verstärkte Enzymaktivität der Aspartatkinase (EC 2.7.2.4) auf, wobei feedbackresistente Allele bevorzugt sind. In *E*. *coli* gibt es drei verschiedene Aspartatkinasen, die von den Genen *thrA*, *metL* oder *lysC* kodiert werden. In einer weiteren bevorzugten Ausführungsform ist die L-MethioninBiosynthese durch Abschwächung oder Deletion des Regulatorproteins MetJ, das von dem Gen *metJ* kodiert wird, gesteigert. MetJ stellt den Hauptrepressor der L-Methionin-Biosynthese in *E*. *coli* dar.

In einer weiteren bevorzugten Ausführungsform ist der Ausgangsstamm der Zelle von einem Stamm aus der Gruppe umfassend *Escherichia coli* MG1655, *Escherichia coli* W3110, *Escherichia coli* DH5α, *Escherichia coli* DH10B, *Escherichia coli* BW2952, *Escherichia coli* REL606 abgeleitet.

Bei sämtlichen in dieser Anmeldung zum Zitieren von Nukleinsäure- und Aminosäuresequenzen aus dem Stand der Technik verwendeten Zugangsnummern und -codes handelt es sich um Zugangsnummern und -codes aus der BioCyc-Datenbank von ISR, CA, USA in der am 1. Dezember 2011 online verfügbaren Version.

In einer weiteren bevorzugten Ausführungsform ist die Zelle von dem Methionin überproduzierenden *E*. *coli*-Produktionsstamm MG1655 ΔmetJ metA*11 Ptrc-metH Ptrc-metF PtrcF-cysPUWAM PtrcF-cysJIH ΔpykF ΔpykA Ptrc09-gcvTHP ΔpurU Ptrc36-ARNmst17-metF umfassend die Produktionsplasmide pME101-thrA*1-cysE-Pgap-metA*11 und pCC1 BAC-serB-glyA-serA-serC (WO2009/043803) abgeleitet.

In einer weiteren bevorzugten Ausführungsform ist die Zelle von dem Methionin überproduzierenden *E*. *coli*-Produktionsstamm MG1655 ΔmetJ Ptrc-metH Ptrc-metF PtrcF-cysPUWAM PtrcF-cysJIH Ptrc09-gcvTHP umfassend das Produktionsplasmid pME101-thrA*1-cysE-Pgap-metA*11 abgeleitet. Die Klonierung diese Stammes kann, wie in der Patentanmeldung WO2009/043803 beschrieben ist, durch eine Reihe von P1-Transduktionen und Kurierungen erfolgen. Der Stamm basiert auf dem Wildtypstamm *E*. *coli* K12 MG1655. Im Genom dieses Stammes wurden die folgenden Veränderungen eingeführt: Deletion des Gens für den Repressor dem L-Methioninbiosynthese *metJ;* Insertion des starken trc-Promoters stromaufwärts/upstream des Gens *metH,* das für die Cobalamin-abhängige Methioninsynthase kodiert; Insertion des starken trc-Promoters stromaufwärts/upstream des Gens *metF;* das für die 5,10-Methylentetrahydrofolat-Reduktase kodiert; Insertion des starken trc-Promoters stromaufwärts/upstream des Operons *cysPUWAM,* wobei *cysPUWA* für einen Sulfat/Thiosulfat-Aufnahmetransporter und *cysM* kodiert für die Cystein-Synthase B kodiert; Insertion des starken trc-Promoters stromaufwärts/upstream des Operons *cysJIH,* wobei *cysJI* für die Sulfit-Reductase und *cysH* für die 3'-Phospho-Adenylylsulfat-Reduktase kodiert; Insertion des starken trc09-Promoters stromaufwärts/upstream des Operons *gcvTHP,* wobei *gcvT, gcvH* und *gcv P* für drei Komponenten des Glycin-Cleavage-Systems kodieren.

Die Klonierung des *E*. *coli* Produktionsplasmides pME101-thrA*1-cysE-PgapmetA*11 wird in den Patentanmeldungen WO2007/077041 und WO2009/043803 beschrieben. Es handelt sich um ein Plasmid niedriger Kopienzahl (*low copy* plasmid) auf Basis des Vektors pCL1920 (Lerner, C.G. and Inouye, M., Nucl. Acids Res. (1990) 18:4631 [PMID: 2201955]). Das Leerplasmid pME101 besitzt das *lacI^{q}*-Gen, welches für ein stark exprimiertes Allel des lac-Repressors kodiert. Downstream eines starken, durch den Lac-Repressor reprimierbaren trc-Promoters wurde das Gen *thrA*1* kloniert. Es kodiert für eine *feedback*-resistente Variante der Aspartatkinase / Homoserindehydrogenase ThrA aus *E*. *coli.* In der gleichen Orientierung dahinter liegt das Gen *cysE* mitsamt seinem natürlichen Promoter. Es kodiert für die Serin-Acetyltransferase aus *E*. *coli.* Downstream von *cysE* folgt der starke *gap*A-Promoter aus *E*. *coli,* welcher die Expression des Gens *metA*11* kontrolliert. *metA*11* kodiert für eine *feedback*-resistente Variante der Homoserin O-Succinyltransferase aus *E*. *coli.*

Als Beispiele für weitere L-Methionin ausscheidende bzw. produzierende Mikroorganismen können folgende Stämme genannt werden: *E*. *coli* TF4076BJF metA#10 + metYX(Lm) (WO2008/127240); *E*. *coli* W3110ΔJ/pKP451 (EP 1 445 310 B1, Seite 7 Bsp. 4); *E*. *coli* WΔthrBCΔmetJmetK32 pMWPthrmetA4Δ5Δ9 (Yoshihiro Usuda and Osamu Kurahashi, 2005, Applied and Environmental Microbiology, Vol. 71, No. 6, p. 3228-3234); und W3110/pHC34 (WO01/27307 Seite 13, Bsp. 3). Weitere Beispiele verschiedener geeigneter Mikroorganismen sind bei Gomes et al. beschrieben (Enzyme and Microbial Technology 37, 2005, 3-18).

Auch Tryptophan überproduzierende Stämme sowie Maßnahmen und Modifikationen, mit denen eine Überproduktion dieser Aminosäure erreicht werden kann, sind im Stand der Technik beschrieben, beispielsweise *E*. *coli* JP4735/pMU3028 (US5,756,345), *E*. *coli* JP6015/pMU91 (US5,756,345), *E*. *coli* SV164(pGH5) (WO94/08031), *E*. *coli* AGX17(pGX44) (NRRL B-12263) (US4,371,614), *E*. *coli* AGX6(pGX50)aroP (NRRL B-12264) (US4,371,614), *E*. *coli* AGX17/pGX50,pACKG4-pps (WO97/08333), *E*. *coli* ATCC 31743 (CA1182409), *E*. *coli* C534/pD2310,pDM136 (ATCC 39795) (WO87/01130), *E*. *coli* JB102/p5LRPS2 (US5,939,295).

In einer bevorzugten Ausführungsform weisen L-Tryptophan überproduzierende Stämme aus der Familie der *Enterobacteriaceae* ein oder mehr als ein genetisches phänotypisches Merkmal ausgewählt aus der Gruppe umfassend Resistenz gegen 5-Methyl-DL-Tryptophan, Resistenz gegen 5-Fluoro-Tryptophan, Resistenz gegen 4-Methyl-DL-Tryptophan, Resistenz gegen 6-Methyl-DL-Tryptophan, Resistenz gegen 4-Fluoro-Tryptophan, Resistenz gegen 6-Fluoro-Tryptophan, Resistenz gegen Anthranilat, Resistenz gegen Tryptazan, Resistenz gegen Indol, Resistenz gegen Indol-Acrylsäure, Bedürftigkeit für Phenylalanin, Bedürftigkeit für Tyrosin, gegebenenfalls Fähigkeit zur Saccharose-Verwertung, Verstärkung des Tryptophan-Operons, bevorzugt der Anthranilat-Synthase bevorzugt der feed back resistenten Form, eine teilweise defekte Tryptophanyl-tRNA-Synthase, eine abgeschwächte Tryptophan-Aufnahme, eine defekte Tryptophanase, inaktivierte Repressorproteine, Verstärkung der Serin-Biosynthese, Verstärkung der Phosphoenolpyruvat-Synthese, Verstärkung der D-Erythrose-4-Phosphat-Synthese, Verstärkung der 3-deoxy-D-arabino-heptulosonat-7-Phosphat(DHAP)-Synthese und Verstärkung der Chorismat-Biosynthese auf.

Neben wenigstens einer eine Verringerung der ppGppase-Aktivität bewirkenden genetischen Veränderungen kann die erfindungsgemäße Zelle weitere Modifikationen gegenüber dem Ausgangsstamm aufweisen, die eine gesteigerte Produktion der interessierenden Aminosäure(n) bewirken, insbesondere durch Veränderung der Genexpression. In einer bevorzugten Ausführungsform bedeutet die Formulierung, die Zelle ist ihrem Wildtyp gegenüber derartig genetisch verändert, dass sie relativ zu ihrem Wildtyp "wenigstens eine Nukleinsäuresequenz oder eine Variante davon in einem verringerten Ausmaß exprimiert bzw. überexprimiert", wie hierin verwendet, dass die genetisch veränderte Zelle unter vergleichbaren Bedingungen, beispielsweise Temperatur, Nährstoffangebot und Zelldichte, eine geringere bzw. eine größere Menge Transkriptions- und/oder Translationsprodukt der Nukleinsäuresequenz herstellt als die nicht genetisch veränderte Zelle es tun würde.

Die Erhöhung der Expression einer Nukleinsäure lässt sich beispielsweise dadurch erzielen, dass man die Kopienzahl der entsprechenden Polynukleotide chromosomal oder extrachromosomal um mindestens eine Kopie erhöht. Eine weit verbreitete Methode zur Erhöhung der Kopienzahl besteht darin, dass man das entsprechende Polynukleotid in einen Vektor, bevorzugt ein Plasmid, einbaut, der von einem Bakterium repliziert wird. Geeignete Plasmidvektoren für Enterobacteriaceae sind z.B. von pACYC184 abgeleitete Kloniervektoren (Bartolomé et al.; Gene 102: 75-78 (1991)), pTrc99A (Amann et al.; Gene 69: 301-315, 1988) oder pSC101-Derivate (Vocke und Bastia; Proc. Natl. Acad. Sci. 80(21): 6557-6561, 1983). Besonders geeignet sind zudem von pCL1920 (Lerner, C.G. and Inouye, M., Nucl. Acids Res., 1990, 18:4631 [PMID: 2201955]) abgeleitete Plasmide. Von "Bacteriol Artificial Chromosomes" (BAC) abgeleitete Plasmidvektoren, wie z.B. pCC1BAC (EPICENTRE Biotechnologies, Madison, U.S.A.) sind ebenfalls geeignet, die Kopienzahl der entsprechenden Polynukleotide in *E*. *coli* zu erhöhen.

Weiterhin kann man als Vektoren Transposons, Insertionselemente (IS-Elemente) oder Phagen einsetzen. Derartige genetische Systeme sind beispielsweise in den Patentschriften US 4,822,738, US 5,804,414 und US 5,804414 dargestellt. In gleicher Weise kann das in der WO 92/02627 beschriebene IS-Element ISaBI oder das Transposon Tn 45 des Plasmids pXZ10142 (zitiert im "Handbook of *Corynebacterium glutamicum*" (Herausgeber: L. Eggeling und M. Bott)) verwendet werden.

Eine andere verbreitete Methode zur Erhöhung der Expression ist das Verfahren der chromosomalen Genamplifikation. Bei dieser Methode wird mindestens eine zusätzliche Kopie des interessierenden Polynukleotids in das Chromosom einer Zelle eingefügt. Derartige Amplifikationsverfahren sind beispielsweise in der WO 03/014330 oder WO 03/040373 beschrieben.

Eine weitere Methode zur Erhöhung der Expression besteht darin, das entsprechende Gen beziehungsweise Allel in funktioneller Weise (operably linked) mit einem Promotor beziehungsweise einer Expressionskassette zu verknüpfen. Geeignete Promotoren für *E*. *coli* sind z. B. die von Amann et al. (Gene 69(2), 301-315, 1988) und Amann und Brosius (Gene 40(2-3), 183-190, 1985) beschriebenen Promotoren T3, T7, SP6, M13, lac, tac und trc. Ein derartiger Promotor kann beispielsweise stromaufwärts des betreffenden Gens, typischerweise im Abstand von ungefähr 1 - 500 Nukleotidbasen vom Startkodon eingefügt werden. In der US 5,939,307 wird beschrieben, dass durch Einbau von Expressionskassetten oder Promotoren wie beispielsweise tac-Promoter, trp-Promoter, Ipp-Promoter oder PL-Promoter und PR-Promoter des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren, der nar-Promotor oder die Promotoren der Gene rrsG, rnpB, csrA, csrB, ompA, fusA, pepQ, rpIX oder rpsG verwendet werden. Derartige Expressionskassetten oder Promotoren können auch verwendet werden um, wie in der EP 0 593 792 beschrieben, plasmidgebundene Gene zu überexprimieren. Durch Verwendung des lacIQ-Allels lässt sich wiederum die Expression von Genen kontrollieren (Glascock und Weickert, Gene 223, 221-231, 1998). Es ist dem Fachmann weiterhin möglich, die Aktivität der Promotoren durch Modifikation ihrer Sequenz mittels einem oder mehreren Nukleotidaustauschen, durch Insertion (en)und/oder Deletion(en) zu erhöhen.

Das Ausmaß der Expression einer Nukleinsäure oder einer Variante davon kann durch geeignete Kulturführung, durch genetische Veränderung (Mutation) der Signalstrukturen der Genexpression oder auch durch Antisense-RNA Technik verringert werden. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195, 1998), bei Carrier und Keasling (Biotechnology Progress 15: 58-64, 1999), Franch und Gerdes (Current Opinion in Microbiology 3: 159-164, 2000), Kawano et al. (Nucleic Acids Research 33(19), 6268-6276, 2005) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990). In einer bevorzugten Ausführungsform bedeutet der Begriff "Knock out" eines Gens, wie hierin verwendet, dass das Gen oder die für seine Expression benötigte zelluläre Maschinerie oder benötigte Nukleinsäuren oder Teile davon, beispielsweise Promoter, derart genetisch verändert sind, z.B. durch Mutation, Deletion oder Insertion, dass die Expression in Reihenfolge zunehmender Bevorzugung um 50, 75, 80, 85, 90, 95 oder 99 % verringert ist. Beispielsweise besteht die Möglichkeit, die Expression des Gens unter die Kontrolle eines Promotors zu stellen, der in dem für das Verfahren verwendeten Mikroorganismus zu einer gegenüber dem Ausgangsstamm verringerten Expression führt. Weiterhin kann die Expression durch Deletion des Gens oder Teilen davon in dem für das Verfahren verwendeten Mikroorganismus verringert werden. Ebenso können Transitionen, Transversionen oder Insertionen, die zu Fehlsinnmutationen oder Nichtsinnmutationen führen, verwendet werden. Weiterhin kann die Expression durch Verwendung einer Ribosomen-Bindestelle, die in dem für das Verfahren verwendeten Mikroorganismus zu einer gegenüber dem Ausgangsstamm verringerten Translation führt, verringert werden. Weiterhin kann der Fachmann die Expression des Gens durch eine Verschlechterung der Kodon-Verwendung (codon usage) bezogen auf den für das Verfahren verwendeten Mikroorganismus verringern. Schließlich kann die Genexpression wird durch Suppression einer Stop-Kodon-Mutation im Kodierbereich des Gens durch geeignete t-RNA-Suppressoren verringert werden.

Die erfindungsgemäße bzw. erfindungsgemäß verwendete Zelle zeigt eine im Vergleich zum eingesetzten Ausgangsstamm beziehungsweise Elternstamm erhöhte Ausscheidung oder Produktion der gewünschten Aminosäure in einem Fermentationsprozess. Dabei werden die Aminosäuren in das sie umgebende Medium abgegeben oder im Zellinneren akkumuliert.

Die Leistung der erfindungsgemäßen Zelle oder des Fermentationsprozesses unter Verwendung der erfindungsgemäßen Zelle bezüglich eines oder mehrerer der Parameter ausgewählt aus der Gruppe der Produkt-Konzentration (Produkt pro Volumen), der Produkt-Ausbeute (gebildetes Produkt pro verbrauchter KohlenstoffQuelle) und der Produkt-Bildung (gebildetes Produkt pro Volumen und Zeit) oder auch anderer Prozess-Parameter und Kombinationen davon, wird um mindestens 0,5 %, mindestens 1 %, mindestens 1,5 % oder mindestens 2 % bezogen auf den Ausgangstamm bzw. Elternstamm bzw. den Fermentationsprozess unter Verwendung derselben verbessert.

Im erfindungsgemäßen Verfahren kann die Zelle kontinuierlich - wie beispielsweise in der PCT/EP2004/008882 beschrieben - oder diskontinuierlich im batch-Verfahren (Satzkultivierung) oder im fed batch (Zulaufverfahren)- oder repeated fed batch (repetitives Zulaufverfahren)-Verfahren zum Zwecke der Produktion von L-Aminosäuren kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium oder Fermentationsmedium muss in geeigneter Weise den Ansprüchen der jeweiligen Stämme genügen. Beispielsweise sind Beschreibungen von Kulturmedien für verschiedene Mikroorganismen im Handbuch "Manual of Methods for General Bacteriology" der American Society for Bacteriology (Washington D.C., USA, 1981) enthalten. Die Begriffe Kulturmedium, Fermentationsmedium und Medium sind gegeneinander austauschbar.

Als Kohlenstoffquelle können Zucker und Kohlehydrate wie z. B. Glucose, Saccharose, Laktose, Fructose, Maltose, Melasse, Saccharose-haltige Lösungen aus der Zuckerrüben- oder Zuckerrohrherstellung, Stärke, Stärkehydrolysat und Cellulose, Öle und Fette, wie zum Beispiel Sojaöl, Sonnenblumenöl, Erdnussöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure verwendet werden. Diese Stoffe können einzeln oder als Mischung verwendet werden.

Als Stickstoffquelle können organische stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat, Ammoniumnitrat, Ammoniak und Ammoniumthiosulfat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden.

Als Schwefelquelle kann ein Salz der Dithioschwefelsäure (Thiosulfat) allein oder zusammen mit anderen Schwefelquellen wie zum Beispiel Sulfat, Sulfit, Sulfid, Schwefelwasserstoff, Methanthiolat oder Dithionit, siehe auch EP-Anmeldung 11151526.8, verwendet werden.

Das Kulturmedium muss weiterhin Salze beispielsweise in Form von Chloriden, von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können Wuchsstoffe wie Aminosäuren beispielsweise Homoserin und Vitamine beispielsweise Cobalamin, Thiamin, Biotin oder Pantothensäure zusätzlich zu den oben genannten Stoffen eingesetzt werden.

Dem Kulturmedium können überdies geeignete Vorstufen der jeweiligen Aminosäure zugesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete selektiv wirkende Stoffe, wie zum Beispiel Antibiotika hinzugefügt werden. Um aerobe Bedingungen aufrechtzuerhalten, werden Sauerstoff oder Sauerstoff umfassende Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, durchgeführt. Die Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Geeignete Fermentationsmedien sind unter anderem in der US 6,221,636, in der US 5,840,551, in der US 5,770,409, in der US 5,605,818, in der US 5,275,940 und in der US 4,224,409 beschrieben.

Die auf diese Weise hergestellte Fermentationsbrühe wird anschließend zu einem festen oder flüssigen Produkt weiterverarbeitet.

In einer bevorzugten Ausführungsform ist unter einer "Fermentationsbrühe", wie hierin verwendet, Fermentationsmedium zu verstehen, in dem ein Mikroorganismus für eine gewisse Zeit und bei einer gewissen Temperatur kultiviert wurde. Das Fermentationsmedium oder das während der Fermentation eingesetzten Medium umfasst sämtliche Substanzen oder Komponenten, die für eine Vermehrung des Mikroorganismus und eine Bildung der gewünschten Aminosäure erforderlich sind.

Bei Abschluss der Fermentation enthält die entstandene Fermentationsbrühe dementsprechend a) die infolge der Vermehrung der Zellen des Mikroorganismus entstandene Biomasse des Mikroorganismus, b) die im Laufe der Fermentation gebildete gewünschte Aminosäure, c) die im Laufe der Fermentation gebildeten Nebenprodukte und d) die durch die Fermentation nicht verbrauchten Bestandteile des/der eingesetzten Fermentationsmediums/ Fermentationsmedien beziehungsweise der Einsatzstoffe wie beispielsweise Vitamine wie Biotin, Aminosäuren wie Homoserin oder Salze wie Magnesiumsulfat.

Zu den Nebenprodukten gehören Stoffe, die von den bei der Fermentation eingesetzten Mikroorganismen neben der jeweiligen erwünschten L-Aminosäure erzeugt und möglicherweise ausgeschieden werden. Hierzu zählen L-Aminosäuren, die im Vergleich zur erwünschten Aminosäure weniger als 30 %, 20 % oder 10 % ausmachen, weiterhin organische Säuren, die ein bis drei Carboxyl-Gruppen tragen, zum Beispiel Essigsäure, Milchsäure, Zitronensäure, Apfelsäure oder Fumarsäure, sowie Zucker, zum Beispiel Trehalose.

Typische für industrielle Zwecke geeignete Fermentationsbrühen haben einen Aminosäuregehalt von 40 g/kg bis 180 g/kg oder 50 g/kg bis 150 g/kg. Der Gehalt an Biomasse (als getrocknete Biomasse) beträgt im Allgemeinen 20 bis 50 g/kg.

Zum Aufreinigen der Aminosäure aus der hergestellten Kultur- oder Fermentationsbrühe sind im Stand der Technik zahlreiche Verfahren beschrieben und umfassen die Ionenaustauschchromatographie, die Kristallisation, Extraktionsverfahren und die Behandlung mit Aktivkohle. Hierdurch erhält man weitgehend reine L-Aminosäuren mit einem Gehalt von ≥ 90 Gew.-%, ≥ 95 Gew.-%, ≥ 96 Gew.-%, ≥ 97 Gew.-% ≥ 98 Gew.-% oder ≥ 99 Gew.-%.

Es ist ebenfalls möglich, aus der hergestellten Fermentationsbrühe eine Aminosäure aufzureinigen, indem man die in der Fermentationsbrühe enthaltene Biomasse des Bakteriums vollständig (100%) oder nahezu vollständig d.h. mehr als oder größer als (>) 90%, >95%, >97%, >99% entfernt und die übrigen Bestandteile der Fermentationsbrühe weitgehend d.h. zu 30% - 100%, 40% - 100%, 50% - 100%, 60% - 100%, 70% - 100%, 80% - 100%, oder 90% - 100%, bevorzugt größer gleich (≥) 50%, ≥60%, ≥70%, ≥80%, ≥90% oder ≥95% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder Granulier-Verfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10 Gew.-%, kleiner als Gew.-5%, kleiner als Gew.-4% oder kleiner 3 Gew.-% beträgt.

Die Analyse von L-Aminosäuren zur Bestimmung der Konzentration zu einem oder mehreren Zeitpunkt(en) im Verlauf der Fermentation kann durch Trennung der L-Aminosäuren mittels Ionenaustauschchromatographie vorzugsweise Kationenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtadialdehyd zur Nachsäulenderivatisierung eingesetzt werden. Einen Übersichtsartikel zur Ionenaustauschchromatographie findet man bei Pickering (LC·GC (Magazine of Chromatographic Science) 7(6), 484-487 (1989)).

Es ist ebenfalls möglich eine Vorsäulenderivatisierung beispielsweise unter Verwendung von ortho-Phtadialdehyd oder Phenylisothiocyanat vorzunehmen und die entstandenen Aminosäurederivate durch Reversed-Phase-Chromatographie (RP) vorzugsweise in Form der Hochleistungsflüssigkeitschromatographie (HPLC) aufzutrennen. Eine derartige Methode ist beispielsweise bei Lindroth et al. (Analytical Chemistry 51: 1167-1174 (1979)) beschrieben.

Die Detektion erfolgt photometrisch (Absorption, Fluoreszenz).

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Die vorliegende Erfindung wird weiterhin durch die folgenden Figuren und nicht beschränkenden Beispiele veranschaulicht, denen weitere Merkmale, Ausführungsformen, Aspekte und Vorteile der vorliegenden Erfindung entnommen werden können.

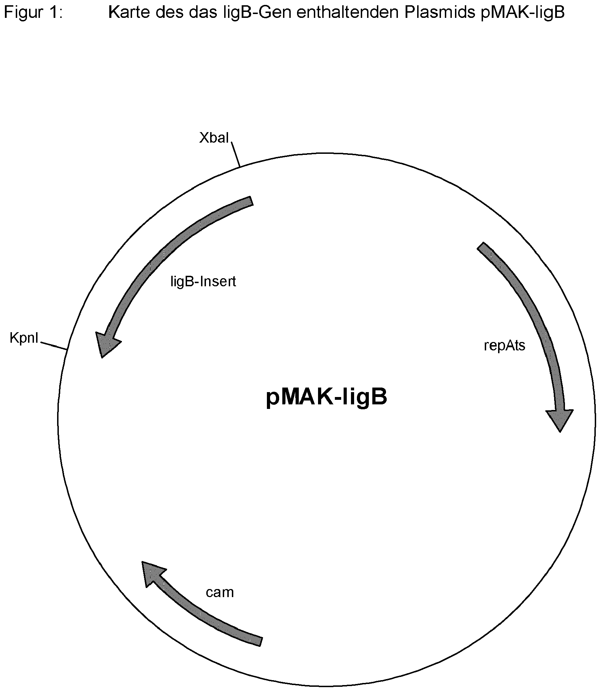

**Fig. 1** zeigt eine Karte des Plasmids pMAK-ligB, welches einen Abschnitt des ligB-Gens enthält.

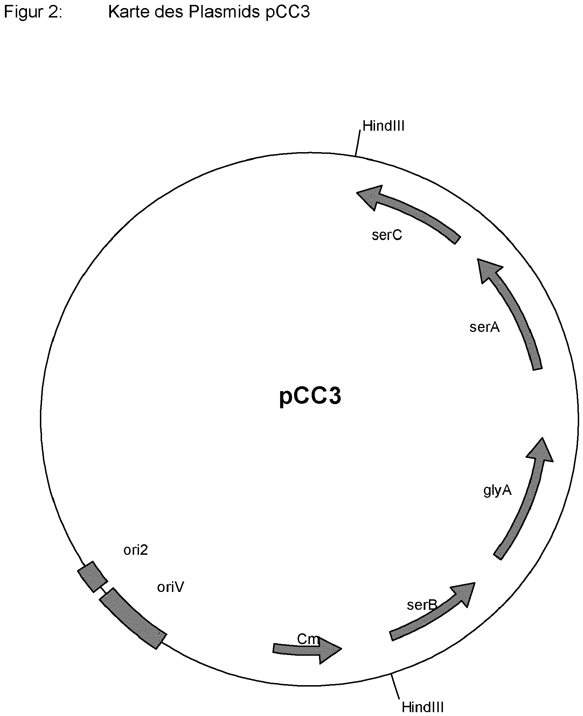

**Fig. 2** zeigt eine Karte des Plasmids pCC3

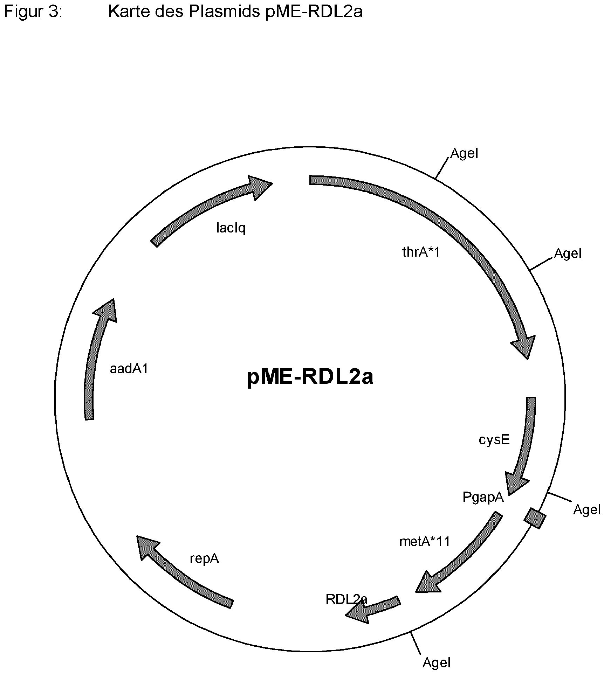

**Fig. 3** zeigt eine Karte des Plasmids pME-RDL2a

Längenangaben sind als ungefähre Angaben aufzufassen. Die verwendeten Abkürzungen und Bezeichnungen haben folgende Bedeutung:
- aadA1: Streptomycin-/Spectinomycinresistenzgen
- lacIq: Gen für das Repressorprotein des trc-Promotors
- repAts: plasmid replication protein (ts) ; auch repA
- oriV: origin of replication
- ori2: replication origin
- cam/Cm: Chloramphenicolresistenzgen
- ligB Insert: Teil der Kodierregion des ligB-Gens
- serC: Kodierregion des serC-Gens
- serA: Kodierregion des serA-Gens
- glyA: Kodierregion des glyA-Gens
- serB: Kodierregion des serB-Gens
- thrA*1: Kodierregion des thrA-Gens (feedbackresistentes Allel)
- cysE: Kodierregion des cysE-Gens
- PgapA: gap-Promotor
- metA*11: Kodierregion des metA-Gens (feedbackresistentes Allel)
- RDL2a: Kodierregion des RDL2-Allels kodierend für Thiosulfat-Sulfurtransferase

Die Abkürzungen für die Restriktionsenzyme haben folgende Bedeutung:
- Agel: Restriktionsendonuklease aus *Ruegeria gelatinovora*
- HindIII: Restriktionsendonuklease aus *Haemophilus influenzae* Rd
- XbaI: Restriktionsendonuklease aus *Xanthomonas campestris*
- KpnI: Restriktionsendonuklease aus *Klebsiella pneumoniae*

Die vorliegende Erfindung wird im Folgenden anhand von Ausführungsbeispielen näher erläutert.

Verwendete Minimal- (M9) und Vollmedien (LB) für *E. coli* sind von J.H. Miller (A Short Course in Bacteriol Genetics (1992), Cold Spring Harbor Laboratory Press) beschrieben. Die Isolierung von Plasmid-DNA aus *E. coli* sowie alle Techniken zur Restriktion, Ligation, Klenow- und alkalische Phosphatasebehandlung werden, wenn nicht anders beschrieben, nach Sambrook et al. (Molecular Cloning - A Laboratory Manual (1989) Cold Spring Harbor Laboratory Press) durchgeführt. Die Transformation von *E. coli* wird, wenn nicht anders beschrieben, nach Chung et al. (Proc. Natl. Acad. Sci. 86: 2172-2175, 1989) durchgeführt.

Wenn nicht anders beschrieben beträgt die Bebrütungstemperatur bei der Herstellung von Stämmen und Transformanten 37°C.

### Beispiel 1

### Klonierung von pMAK-ligB

Als Selektionsmarker für die Transduktion von spoT-Allelen wurde das Plasmid pMAK-ligB kloniert und im Chromosom von *E. coli* DM1849 in das Gen ligB inseriert. Das Gen ligB befindet sich im Chromosom etwa 1,2 kbp upstream von spoT. Der Stamm *E. coli* DM1849 besitzt ein erfindungsgemäßes spoT-Allel.

Die PCR-Primer ligb-up und ligb-down besitzen an den 5'-Enden jeweils 6 nach dem Zufallsprinzip ausgewählte Nukleotide gefolgt von Erkennungssequenzen für die Restriktionsendonucleasen XbaI (tctaga) bzw. KpnI (ggtacc). Die Nukleotide 13 bis 32 von ligb-up binden im *E. coli* MG1655-Genom von Pos. 3817496 bis 3817516. Die Nukleotide 13 bis 32 von ligb-down binden im *E. coli* MG1655-Genom von Pos. 3818519 bis 3818498.
ligB-up (SEQ ID NO:9) 5' CAGTACtctagaAGCCACGAAGGACACTAAGG 3'
ligB-down (SEQ ID NO:10) 5' TTAGTTggtaccCGGATGGACCGCAGTTAATG 3'

Mit diesen beiden Primern und genomischer DNA von *E. coli* MG1655 als Template wurde eine PCR durchgeführt. Das PCR-Produkt hatte eine Länge von 1045 bp und enthielt die Sequenz von Pos. 3817496 bis 3818519 des MG1655-Genoms (SEQ ID NO:11).

Das PCR-Produkt wurde über ein QIAquick PCR Purification Kit (Fa. Qiagen, Hilden) aufgereinigt, mit den Restriktionsenzymen Xbal und Kpnl gespalten und erneut aufgereinigt. Das Plasmid pMAK705 (Hamilton CM, Aldea M, Washburn BK, Babitzke P, Kushner SR (1989); J Bacteriol.; 171 (9): 4617-4622) wurde mit den Restriktionsenzymen Xbal und Kpnl gespalten, mit Alkalischer Phosphatase dephosphoryliert (Alkaline Phosphatase, Calf Intestinal, Fa. New England Biolabs, Frankfurt a.M.) und über ein QIAquick PCR Purification Kit (Fa. Qiagen, Hilden) aufgereinigt. Anschließend wurde das PCR-Produkt mit pMAK705 ligiert und der Ligationsansatz in *E. coli* DH5α transformiert. Korrekte Plasmidklone wurden durch 20 mg/l Chloramphenicol selektioniert und per Restriktionsspaltung sowie anschließender Sequenzierung der Inserts identifiziert. Das so erhaltene Plasmid wurde mit pMAK-ligB bezeichnet.

### Beispiel 2

### Integration von pMAK-ligB in den Donor-Stamm DM1849

Der *E. coli*-Stamm DM1849 trägt drei Mutationen im für die ppGppase kodierenden Gen spoT: eine Insertion der sechs Nucleotide CATGAT hinter der Nucleotidposition 252 (entspr. Pos. 3820674 im MG1655 Genom), den Austausch g520t (bezogen aus das wildtypische spoT-Gen, entspr. Pos. 3820942 im MG1655 Genom) und den Austausch c1585t (bezogen auf das wildtypische spoT-Gen, entspr. Pos. 3822007 im MG1655 Genom).

Der Stamm DM1849 wurde per Elektroporation mit dem Plasmid pMAK-ligB transformiert. pMAK-ligB besitzt ein Chloramphenicol-Resistenzgen und einen temperatursensisiven Replikationsursprung. Das Plasmid wird bei 30°C von *E. coli* repliziert, bei 44°C jedoch nicht. Der Transformationsansatz wurde auf LB-Agar mit 20 mg/l Chloramphenicol ausplattiert und 40 Stunden bei 30°C inkubiert. Anschließend wurden mit einer Impföse Zellen aufgenommen, in LB-Medium resuspendiert und mit LB-Medium 10000-fach verdünnt. 100 µl der Verdünnung wurden auf LB-Agar mit 20 mg/l Chloramphenicol ausplattiert und weitere 24 Stunden bei 44°C inkubiert. Dadurch wurden Kolonien selektioniert, bei denen das Plasmid pMAK-ligB chromosomal in das Gen ligB integriert war. Eine dieser Kolonien wurde auf LB-Agar mit 20 mg/l Chloramphenicol mit einer Impföse vereinzelt und für 24 Stunden bei 44°C inkubiert. Der resultierende Stamm wurde mit DM1849::pMAK-ligB bezeichnet.

### Beispiel 3

### Erstellung einer P1-Phagenbank aus DM1849::pMAK-ligB

Zunächst wurden 10 ml LB-Medium mit dem Stamm DM1849::pMAK-ligB beimpft und 18 Stunden bei 44°C kultiviert. Anschließend wurden 100 µl der Kultur in 10 LB-Medium überimpft und bei 44°C bis zu einer optischen Dichte (bei 600 nm) von 0,5 kultiviert. Nun wurde Calciumchlorid bis zu einer Endkonzentration von 5 mM und Glucose bis zu einer Endkonzentration von 2 g/l zugegeben. Anschließend wurden 100 µl einer P1-Phagensuspension zugegeben und die Zellen bei 37°C inkubiert. Nach 4 Stunden wurde die Kultur zentrifugiert und der Überstand zweimal steril filtriert.

### Beispiel 4

### Transduktion des Methionin-Produzenten E. coli ECM1 mit der P1-Phagenbank aus DM1849::pMAK-ligB zur Übertragung der drei Mutationen aus DM1849

Der L-Methionin produzierende *E. coli* Stamm ECM1 trägt ein feedbackresistentes metA-Allel, eine Deletion des Gens metJ sowie jeweils eine Kopie des starken trc-Promoters vor den Genen metH, metF, cysP und cysJ. Er basiert auf dem Wildtyp-K12-Stamm MG1655. 10 ml LB-Medium wurden mit dem Empfängerstamm ECM1 beimpft und 18 Stunden bei 37°C kultiviert. Anschließend wurden 2 ml der Kultur abzentrifugiert und das Zellpellet in 1 ml LB-Medium mit 10 mM MgSO₄ und 5 mM CaCl₂ resuspendiert. 300 µl der Zellsuspension wurden mit 100 µl der P1 Phagenbank aus DM1849::pMAK-ligB versetzt und für 30 Min bei 37°C inkubiert. Anschließend wurden 200 µl 1 M Tri-Natriumcitrat zugegeben und kurz gevortext. Nun wurde 1 ml LB-Medium zugegeben und die Zellen eine Stunde bei 44°C kultiviert. Anschließend wurden die Zellen zwei mal mit je 1 ml LB mit 100 mM Tri-Natriumcitrat gewaschen und schließlich in 100 µl LB mit 100 mM Tri-Natriumcitrat resuspendiert. Sie wurden auf LB-Agar mit 20 mg/l Chloramphenicol ausgestrichen und 48 Stunden bei 44°C inkubiert. Zehn Kolonien wurden auf LB-Agar mit 20 mg/l Chloramphenicol vereinzelt und erneut 48 Stunden bei 44°C inkubiert. Bei diesen Klonen war ein genomisches Fragment transduziert worden, welches die ligB-Region mitsamt dem integrierten pMAK-ligB-Plasmides umfasste. Durch anschließende DNA-Sequenzierung der relevanten Abschnitte des Gens spoT wurden fünf Klone identifiziert, bei denen das spoT-Allel aus DM1849 cotransduziert worden war.

Die Excision des Plasmides pMAK-ligB aus dem Chromosom und die Kurierung erfolgte wie bei Hamilton *et al.* beschrieben (CM Hamilton, Aldea M, Washburn BK, Babitzke P, Kushner SR (1989); J Bacteriol.; 171 (9): 4617-4622). Einer der so erhaltenen plasmidfreien Klone wurde mit ECM1_spoT1849 bezeichnet.

Die Stämme ECM1 und ECM1_spoT1849 wurden gemäß dem Budapester Vertrag am 3. August 2011 bei der DSMZ (Deutsche Sammlung von Mikroorganismen und Zellkulturen GmbH, Inhoffenstraße 7 B, 38124 Braunschweig, Deutschland) unter den DSM-Nummern DSM 25066 (= ECM1) und DSM 25067 (= EMC1_spoT1849) hinterlegt.

### Beispiel 5

### Klonierung des serC-Gens in das Plasmid pUC18

Das Gen serC aus *E. coli* MG1655 wurde mit Hilfe der Polymerase-Kettenreaktion (PCR) amplifiziert und anschließend in das Plasmid pUC18 (Fermentas GmbH, St. Leon-Rot, Deutschland) kloniert.

Die PCR-Primer serCF(XbaI) und serCR(HindIII) besitzen an den 5'-Enden jeweils nach dem Zufallsprinzip ausgewählte Nukleotide gefolgt von Erkennungssequenzen für die Restriktionsendonucleasen Xbal (TCTAGA) bzw. HindIII (AAGCTT). Die Nukleotide 13 bis 38 von serCF(XbaI) binden im *E. coli* MG1655-Genom von Pos. 956619 bis 956644. Die Nukleotide 13 bis 37 von serCR(HindIII) binden im E. coli MG1655-Genom von Pos. 958028 bis 958004.
serCF(XbaI) (SEQ ID NO: 12) 5' AGGTGCTCTAGAGTCCGCGCTGTGCAAATCCAGAATGG 3'
serCR(Hindlll) (SEQ ID NO:13) 5' TACACCAAGCTTAACTCTCTACAACAGAAATAAAAAC 3'

Das Gen serC wurde mit den Primern serCF(XbaI) und serCR(HindIII) unter Anwendung der Polymerase-Kettenreaktion (PCR) mit der Phusion DNA-Polymerase (Finnzymes Oy, Espoo, Finnland) amplifiziert. Als Template diente genomische DNA von E. coli MG1655. Das resultierende DNA-Fragment hatte eine Größe von 1434 bp. Es wurde mit den Restriktionsendonucleasen Xbal und HindIII gespalten und mit Hilfe eines QIAquick PCR Purification Kit (Qiagen, Hilden, Deutschland) aufgereinigt. Nicht methylierte DNA des Plasmides pUC18 wurde mit den Restriktionsendonucleasen Xbal und HindIII gespalten und mit Hilfe eines QIAquick PCR Purification Kit (Qiagen, Hilden, Deutschland) aufgereinigt. Anschließend wurde das gespaltene Plasmid mit dem PCR-Produkt ligiert und in *E. coli* DH5α transformiert. Plasmidklone, die das serC-Gen enthielten wurden durch Restriktionsspaltung und DNA-Sequenzierung identifiziert. Das resultierende Plasmid wurde mit pUC18-serC bezeichnet.

### Beispiel 6

### Klonierung des serA-Gens in das Plasmid pUC18-serC

Das Gen serA aus *E. coli* MG1655 wurde mit Hilfe der Polymerase-Kettenreaktion (PCR) amplifiziert und anschließend in das Plasmid pUC18-serC kloniert.

Der PCR-Primer serAF(XbaI) besitzt am 5'-Ende 6 nach dem Zufallsprinzip ausgewählte Nukleotide Nucleotide gefolgt von einer Erkennungssequenz für die Restriktionsendonuclease Xbal (TCTAGA). Die Nukleotide 12 bis 33 binden im *E. coli* MG1655-Genom von Pos. 3055199 bis 3055220. Der PCR-Primer serAR(SHSSNB) besitzt am 5'-Ende 6 nach dem Zufallsprinzip ausgewählte Nukleotide gefolgt von Erkennungssequenzen für die Restriktionsendonucleasen SacI, HindIII, SphI, SmaI, NotI und BgIII. Die Nukleotide 49 bis 58 binden im *E. coli* MG1655-Genom von Pos. 3056880 bis 3056861.
serAF(XbaI) (SEQ ID NO: 14) 5' CTGTAGTCTAGATTAGTACAGCAGACGGGCGCG 3'
serAR(SHSSNB) (SEQ ID NO: 15)

5'
CAAGAGCTCAAGCTTGCATGCGATTCCCGGGCGGCCGCAATAAGATCTCCGTC
AGGGCGTGGTGACCG 3'

Das Gen serA wurde mit den Primern serAF(XbaI) und serAR(SHSSNB) unter Anwendung der Polymerase-Kettenreaktion (PCR) mit der Phusion DNA-Polymerase (Finnzymes Oy, Espoo, Finnland) amplifiziert. Als Template diente genomische DNA von *E. coli* MG1655. Das resultierende DNA-Fragment hatte eine Größe von 1731 bp.

Es wurde mit den Restriktionsendonucleasen Xbal und Sacl gespalten und mit Hilfe eines QIAquick PCR Purification Kit (Qiagen, Hilden, Deutschland) aufgereinigt. Das Plasmid pUC18-serC wurde ebenfalls mit den Restriktionsendonucleasen Xbal und Sacl gespalten und mit Hilfe eines QIAquick PCR Purification Kit (Qiagen, Hilden, Deutschland) aufgereinigt. Anschließend wurde das gespaltene Plasmid mit dem PCR-Produkt ligiert und in *E. coli* DH5α transformiert. Plasmidklone, die das serA-Gen enthielten wurden durch Restriktionsspaltung und DNA-Sequenzierung identifiziert. Das resultierende Plasmid wurde mit pUC18-serAC bezeichnet.

### Beispiel 7

### Klonierung des serB-Gens in das Plasmid pUC18-serAC

Das Gen serB aus *E. coli* MG1655 wurde mit Hilfe der Polymerase-Kettenreaktion (PCR) amplifiziert und anschließend in das Plasmid pUC18-serAC kloniert.

Der PCR-Primer serB(Sphl) besitzt am 5'-Ende 6 nach dem Zufallsprinzip ausgewählte Nukleotide Nucleotide gefolgt von einer Erkennungssequenz für die Restriktionsendonuclease Sphl (GCATGC). Die Nukleotide 13 bis 34 binden im *E. coli* MG1655-Genom von Pos. 4622816 bis 4622837.

Der PCR-Primer serB(Smal) besitzt am 5'-Ende 6 nach dem Zufallsprinzip ausgewählte Nukleotide gefolgt von Erkennungssequenzen für die Restriktionsendonucleasen SalI (GTCGAC) und Smal (CCCGGG). Die Nukleotide 54 bis 75 binden im *E. coli* MG1655-Genom von Pos. 4623887 bis 4623866.
serB(Sphl) (SEQ ID NO: 16) 5' CCATGCGCATGCCCACCCTTTGAAAATTTGAGAC 3'
serB(Smal) (SEQ ID NO: 17)

5' CCGCATGTCGACATCCCGGGCAGAAAGGCCCACCCGAAGGTGAGCCAGTGTG
ATTACTTCTGATTCAGGCTGCC 3'

Das Gen serB wurde mit den Primern serB(Sphl) und serB(Smal) unter Anwendung der Polymerase-Kettenreaktion (PCR) mit der Phusion DNA-Polymerase (Finnzymes Oy, Espoo, Finnland) amplifiziert. Als Template diente genomische DNA von E. coli MG1655. Das resultierende DNA-Fragment hatte eine Größe von 1137 bp.

Es wurde mit den Restriktionsendonucleasen Sphl und Smal gespalten und mit Hilfe eines QIAquick PCR Purification Kit (Qiagen, Hilden, Deutschland) aufgereinigt. Das Plasmid pUC18-serAC wurde ebenfalls mit den Restriktionsendonucleasen Sphl und Smal gespalten, mit alkalischer Phosphatase dephosphoryliert und mit Hilfe eines QIAquick PCR Purification Kit (Qiagen, Hilden, Deutschland) aufgereinigt. Anschließend wurde das gespaltene Plasmid mit dem PCR-Produkt ligiert und in *E. coli* DH5α transformiert. Plasmidklone, die das serB-Gen enthielten wurden durch Restriktionsspaltung und DNA-Sequenzierung identifiziert. Das resultierende Plasmid wurde mit pUC18-serBAC bezeichnet.

### Beispiel 8

### Klonierung des glyA-Gens in das Plasmid pUC18-serBAC

Das Gen glyA aus *E. coli* MG1655 wurde mit Hilfe der Polymerase-Kettenreaktion (PCR) amplifiziert und anschließend in das Plasmid pUC18-serBAC kloniert.

Der PCR-Primer glyA-downstream besitzt am 5'-Ende 6 nach dem Zufallsprinzip ausgewählte Nukleotide gefolgt von einer Erkennungssequenz für die Restriktionsendonuclease BgIII (AGATCT). Die Nukleotide 13 bis 35 binden im *E. coli* MG1655-Genom von Pos. 2682063 bis 2682085.

Der PCR-Primer glyA-upstream besitzt am 5'-Ende nach dem Zufallsprinzip ausgewählte Nukleotide gefolgt von Erkennungssequenzen für die Restriktionsendonuclease Notl (GCGGCCGC). Die Nukleotide 15 bis 33 binden im E. coli MG1655-Genom von Pos. 2683762 bis 2683744.
glyA-downstream(SEQ ID NO:18) 5' ATCTAAAGATCTGTTACGACAGATTTGATGGCGCG 3'
glyA-upstream (SEQ ID NO:19) 5' TTCATCGCGGCCGCGAAAGAATGTGATGAAGTG 3'

Das Gen glyA wurde mit den Primern glyA-downstream und glyA-upstream unter Anwendung der Polymerase-Kettenreaktion (PCR) mit der Phusion DNA-Polymerase (Finnzymes Oy, Espoo, Finnland) amplifiziert. Als Template diente genomische DNA von E. coli MG1655. Das resultierende DNA-Fragment hatte eine Größe von 1726 bp.

Es wurde mit den Restriktionsendonucleasen BgIII und NotI gespalten und mit Hilfe eines QIAquick PCR Purification Kit (Qiagen, Hilden, Deutschland) aufgereinigt. Das Plasmid pUC18-serBAC wurde ebenfalls mit den Restriktionsendonucleasen BgIII und Notl gespalten und mit Hilfe eines QIAquick PCR Purification Kit (Qiagen, Hilden, Deutschland) aufgereinigt. Anschließend wurde das gespaltene Plasmid mit dem PCR-Produkt ligiert und in *E. coli* DH5α transformiert. Plasmidklone, die das glyA-Gen enthielten wurden durch Restriktionsspaltung und DNA-Sequenzierung identifiziert. Das resultierende Plasmid wurde mit pUC18-serB-glyA-serAC bezeichnet.

### Beispiel 9

### Klonierung der Gene serB-glyA-serAC aus pUC18-serB-glyA-serAC nach pCC1-BAC

Das Plasmid pUC18-serB-glyA-serAC wurde mit der Restriktionsendonuclease HindIII gespalten und die DNA-Fragmente durch eine Agarosegelelektrophorese aufgetrennt. Ein 5,9 kb großes DNA-Fragment wurde aus dem Gel isoliert. Es enthielt die Gene serB, glyA, serA und serC. Das Fragment wurde mit dem bereits mit HindIII gespaltenen Plasmid pCC1 BAC Cloning-Ready Vector (Hind III) der Firma Epicentre /Madison, USA) ligiert und nach *E. coli* EP1300 transformiert. Plasmidklone, die das DNA-Fragment aus serB, glyA, serA und serC enthielten wurden durch Restriktionsspaltung und DNA-Sequenzierung identifiziert. Das resultierende Produktionsplasmid wurde mit pCC3 bezeichnet.

### Beispiel 10

### Klonierung des Produktionsplasmides pME-RDL2a

Die Klonierung des Produktionsplasmides pME-RDL2a erfolgte, wie in der EP-Anmeldung 11151526.8 beschrieben. Es beinhaltet das Gen cysE aus *E. coli,* feedbackresistente Allele der Gene thrA und metA aus *E. coli* sowie das Gen RDL2a, welches für die Thiosulfat-Sulfurtransferase RDL2p aus Saccharomyces cerevisiae codiert. Zusätzlich beinhaltet es ein Streptomycin-Resistenzgen.

### Beispiel 11

### Transformation der Stämme ECM1 und ECM1 spoT1849 mit den Produktionsplasmiden

Die Stämme ECM1 und ECM1_spoT1849 wurden mit dem Produktionsvektor pCC3 aus Beispiel 9 transformiert und die Transformanden mit 20 mg/l Chloramphenicol selektioniert. Anschließend wurden die Zellen mit dem Plasmid pME-RDL2a aus Beispiel 10 transformiert und die resultierenden Transformanden mit 20 mg/l Chloramphenicol + 100 mg/l Streptomycin selektioniert. Die resultierenden Stämme wurden mit ECM1/pCC3/pME-RDL2a und ECM1_spoT1849/pCC3/pME-RDL2a bezeichnet.

### Beispiel 12

### Leistungstest im Schüttelkolbenversuch

Die Leistungsfähigkeit der *E.coli* L-Methionin-Produktionsstämme wurde durch Produktionsversuche in 100 ml Erlenmeyer-Kolben bewertet. Als Vorkulturen wurden jeweils 10 ml Vorkulturmedium (10% LB-Medium mit 2,5 g/l Glucose und 90% Minimalmedium PC1) mit 100µl Zellkultur beimpft und 10 Stunden bei 37°C kultiviert. Hiermit wurden anschließend je 10 ml PC1-Minimalmedium (Tabelle 1) auf eine OD 600 nm von 0,2 (Eppendorf Bio-Photometer; Eppendorf AG, Hamburg, Deutschland) beimpft und für 24 Stunden bei 37°C kultiviert. Die extrazelluläre L-Methionin-Konzentration wurde mit einem Aminosäureanalysator (Sykam GmbH, Eresing, Deutschland) durch Ionenaustauschchromatographie und Nachsäulenderivatisierung mit Ninhydrindetektion bestimmt. Die extrazelluläre GlucoseKonzentration wurde mit einem YSI 2700 Select Glucose Analyzer (YSI Life Sciences, Yellow Springs, Ohio, USA) bestimmt. Die Ergebnisse sind in Tabelle 2 dargestellt. Nach 24 Stunden war die Glucose in beiden Kulturen vollständig verbraucht. Die Methionin-Konzentration im Kulturüberstand von ECM1_spoT1849/pCC3/pME-RDL2a war mit 1,56 g/l deutlich höher, als im Vergleichstamm (1,01 g/I). Die Modifikation des für die ppGppase kodierenden Gens durch Einführung der Mutationen Gly174, Leu529 und einer Insertion zwischen Asp84 und Met85 führte somit zur Steigerung der Ausbeute an Methionin gegenüber dem Ausgangsstamm von 10,1 % (Gramm Methionin pro Gramm Glucose) auf 15,6 %.

**Tabelle 1: Minimal Medium PC1**

| Substanz | Konzentration |
|---|---|
| ZnSO4 * 7 H2O | 4 mg/l |
| CuCl2 * 2 H2O | 2 mg/l |
| MnSO4 * H2O | 20 mg/l |
| H3BO3 | 1 mg/l |
| Na2MoO4 * 2 H2O | 0,4 mg/l |
| MgSO4 * 7 H2O | 1 g/l |
| Citronensäure * 1 H2O | 6,56 g/l |
| CaCl2 * 2 H2O | 40 mg/l |
| K2HPO4 | 8,02 g/l |
| Na2HPO4 | 2 g/l |
| (NH4)2HPO4 | 8 g/l |
| NH4Cl | 0,13 g/l |
| (NH4)2SO3 | 5,6 g/l |
| MOPS | 5 g/l |
| NaOH 10M | auf pH 6,8 eingestellt |
| | |
| FeSO4 * 7 H2O | 40 mg/l |
| Thiaminhydrochlorid | 10 mg/l |
| Vitamin B12 | 10 mg/l |
| Glucose | 10 g/l |
| Isopropyl-thio-β-galaktosid (IPTG) | 2,4 mg/l |
| Spectinomycin | 50 mg/l |
| Chloramphenicol | 20 mg/l |

**Tabelle 2: L-Methioninkonzentrationen in den Fermentationsbrühen der E. coli Stämme ECM1/pCC3/pME-RDL2a und ECM1_spoT1849/pCC3/pME-RDL2a**

| Stamm | OD(600nm) | L-Methionin (g/l) |
|---|---|---|
| ECM1/pCC3/pME-RDL2a | 6,36 | 1,01 |
| ECM1_spoT1849/pCC3/pME-RDL2a | 7,46 | 1,56 |

Die in der vorangehenden Beschreibung und in den Ansprüchen offenbarten Merkmale der Erfindung können sowohl einzeln als auch in beliebiger Kombination zur Verwirklichung der Erfindung in ihren verschiedenen Ausführungsformen wesentlich sein.

## Patentansprüche

1. Zelle, die ihrem Wildtyp gegenüber derartig genetisch verändert ist, dass sie eine relativ zu ihrem Wildtyp verringerte ppGppase-Aktvität aufweist und bevorzugt eine essentielle Aminosäure, noch bevorzugter eine von Serin abgeleitete essentielle Aminosäure, am bevorzugtesten Methionin oder Tryptophan, überproduziert.

2. Zelle nach Anspruch 1, wobei die Zelle eine relativ zu ihrem Wildtyp im Wesentlichen unveränderte (p)ppGpp-Synthetase-Aktivtität aufweist.

3. Zelle nach einem der Ansprüche 1 oder 2, wobei die Zelle ihrem Wildtyp gegenüber derartig genetisch verändert ist, dass sie eine relativ zu ihrem Wildtyp verringerte Aktivität wenigstens einer ppGppase aufweist, die aus der Gruppe ausgewählt ist, welche die Aminosäuresequenzen SEQ ID NO:2, SEQ ID NO:4 und SEQ ID NO:6 sowie Varianten davon umfasst, und es sich bevorzugt um SEQ ID NO:2 oder Varianten davon handelt.

4. Zelle nach Anspruch 3, wobei die Aktivität wenigstens einer ppGppase aus der Gruppe umfassend die Aminosäuresequenzen SEQ ID NO:2, SEQ ID NO:4 und SEQ ID NO:6 sowie Varianten davon, bevorzugt SEQ ID NO:2 oder Varianten davon, dadurch verringert ist, dass diese ppGppase wenigstens eine der folgenden Modifikationen aufweist:
a) das Gly174 oder eine dazu homologe Aminosäure ist gegen eine andere Aminosäure ausgetauscht, bevorzugt nicht konservativ,
b) das Leu529 oder eine dazu homologe Aminosäure ist gegen eine andere Aminosäure ausgetauscht, bevorzugt nicht konservativ,
c) zwischen Asp84 und Met85 oder dazu homologen Aminosäuren liegt eine Insertion von wenigstens einer Aminosäure, bevorzugt wenigstens zwei Aminosäuren, bevorzugt von His und Asn, vor,
wobei bevorzugt alle drei Mutationen vorliegen.

5. Zelle nach Anspruch 4, wobei die ppGppase zusätzlich wenigstens eine Modifikation aus der Gruppe aufweist, die Austausche, bevorzugt nicht konservative Austausche, an den Aminosäuren Gln9, Thr13, Tyr63, Arg109, Gln225, Cys245, Val248, Asn268, Ser270, Met280, His344, Pro436, Asn501, Gln505, His543, Ala546, Ser547, Ile548, His555, Gly556, His557, Pro559, Lys619, Thr621, Ala622, Thr627, Thr651, Ala669, Ala675, Thr698 und eine Insertion zwischen Glu343 und His344 oder einer dazu jeweils homologen Aminosäure umfasst.

6. Zelle nach einem der Ansprüche 4 bis 5, wobei die ppGppase wenigstens eine Modifikation aus der Gruppe aufweist, die folgende Austausche oder Austausche von zur ausgetauschten Aminosäure homologen Aminosäuren gegen die gleichen Aminosäuren aufweist:
1.) Austausch von Gln9 gegen eine Aminosäure ausgewählt aus der Gruppe Leu, Ile und Val, bevorzugt Leu,
2.) Austausch von Thr13 gegen eine Aminosäure ausgewählt aus der Gruppe Lys, Arg, His, Gln und Asn, bevorzugt Arg oder Asn,
3.) Austausch von Tyr63 gegen eine Aminosäure ausgewählt aus der Gruppe Lys, Arg und His, bevorzugt His,
4.) Austausch des Arg109 gegen eine Aminosäure ausgewählt aus der Gruppe Gln und Asn, bevorzugt Gln,
5.) Austausch von Gln225 gegen eine Aminosäure ausgewählt aus der Gruppe Ser, Ala und Thr, bevorzugt Thr,
6.) Austausch des Cys245 gegen eine Aminosäure ausgewählt aus der Gruppe Leu, Ile und Val, bevorzugt Leu,
7.) Austausch des Val248 gegen eine Aminosäure ausgewählt aus der Gruppe Lys, Arg und His, bevorzugt His,
8.) Austausch des Asn268 gegen eine Aminosäure ausgewählt aus der Gruppe Lys, Arg und His, bevorzugt L-His oder Lys,
9.) Austausch des Ser270 gegen eine Aminosäure ausgewählt aus der Gruppe Ala, Leu, Ile und Val, bevorzugt Ala oder Val,
10.) Austausch des Met280 gegen eine Aminosäure ausgewählt aus der Gruppe Ser, Thr und Ala, bevorzugt Ala,
11.) Insertion der Aminosäuren Lys und Glu zwischen Aminosäure Glu343 und His344,
12.) Austausch des His344 gegen eine Aminosäure ausgewählt aus der Gruppe Gln und Asn, bevorzugt Gln,
13.) Austausch des Pro436 gegen eine Aminosäure ausgewählt aus der Gruppe Ser, Ala und Thr, bevorzugt Ser,
14.) Austausch des Asn501 gegen eine Aminosäure ausgewählt aus der Gruppe Ser, Ala und Thr, bevorzugt L-Alanin,
15.) Austausch des Gln505 gegen eine Aminosäure ausgewählt aus der Gruppe Pro, Ser, Ala und Thr, bevorzugt Pro oder Ala,
16.) Austausch des His543 gegen eine Aminosäure ausgewählt aus der Gruppe Asn und Gln, bevorzugt Gln,
17.) Austausch des Ala546 gegen eine Aminosäure ausgewählt aus der Gruppe Asn und Gln, bevorzugt Gln,
18.) Austausch des Ser547 gegen eine Aminosäure ausgewählt aus der Gruppe Ala, Leu, Ile und Val, bevorzugt Ala oder Val,
19.) Austausch des Ile548 gegen eine Aminosäure ausgewählt aus der Gruppe Asn und Gln, bevorzugt Asn,
20.) Austausch des His555 gegen eine Aminosäure ausgewählt aus der Gruppe Leu, Ile und Val, bevorzugt Val,
21.) Austausch des Glycin an Position 556 gegen Lys, Arg und His, bevorzugt Arg,
22.) Austausch des His557 gegen eine Aminosäure ausgewählt aus der Gruppe Asn und Gln, bevorzugt Gln,
23.) Austausch der Pro559 gegen eine Aminosäure ausgewählt aus der Gruppe Ser, Ala und Thr, bevorzugt Ala,
24.) Austausch des Lys619 gegen eine Aminosäure ausgewählt aus der Gruppe Asn und Gln, bevorzugt Gln,
25.) Austausch des Thr621 gegen eine Aminosäure ausgewählt aus der Gruppe Leu, Ile und Val, bevorzugt Ile,
26.) Austausch des Ala622 gegen eine Aminosäure ausgewählt aus der Gruppe Glu und Asp, bevorzugt Asp,
27.) Austausch des Thr627 gegen eine Aminosäure ausgewählt aus der Gruppe Ala und Gly, bevorzugt Ala,
28.) Austausch des Thr651 gegen eine Aminosäure ausgewählt aus der Gruppe Glu und Asp, bevorzugt Glu,
29.) Austausch des Ala669 und/oder Ala675 gegen Thr,
30.) Austausch des Thr698 gegen eine Aminosäure ausgewählt aus der Gruppe Gln und Asn, bevorzugt Asn.

7. Zelle nach einem der Ansprüche 1 bis 6, wobei die Zelle ihrem Wildtyp gegenüber derartig gentechnisch verändert ist, dass sie relativ zu ihrem Wildtyp wenigstens eine Nukleinsäuresequenz oder eine Variante davon überexprimiert, die für eines der folgenden Enzyme, Komponenten davon oder für Varianten der Enzyme oder Komponenten davon kodiert:
1.) Thiosulfat-/Sulfat-Transportsystems CysPUWA (EC 3.6.3.25),
2.) 3'-Phosphoadenosin 5'-Phosphosulfat Reduktase CysH (EC 1.8.4.8),
3.) Sulfit-Reduktase CysJI (EC 1.8.1.2),
4.) Cystein-Synthase A CysK (EC 2.5.1.47),
5.) Cystein-Synthase B CysM (EC 2.5.1.47),
6.) Serin-Acetyltransferase CysE (EC 2.3.1.30),
7.) Glycin-Cleavage-System GcvTHP-Lpd (EC 2.1.2.10, EC 1.4.4.2, EC 1.8.1.4),
8.) Lipoyl-Synthase LipA (EC 2.8.1.8),
9.) Lipoyl-Protein-Ligase LipB (EC 2.3.1.181),
10.) Phosphoglycerat-Dehydrogenase SerA (EC 1.1.1.95),
11.) 3-Phosphoserin-Phosphatase SerB (EC 3.1.3.3),
12.) 3-Phosphoserin/Phosphohydroxythreonin-Aminotransferase SerC (EC 2.6.1.52),
13.) Serin-Hydroxymethyltransferase GlyA (EC 2.1.2.1),
14.) Aspartokinase I und Homoserin Dehydrogenase I ThrA (EC 2.7.2.4, EC 1.1.1.3),
15.) Aspartatkinase LysC (EC 2.7.2.4),
16.) Homoserin-Dehydrogenase Hom (EC 1.1.1.3),
17.) Homoserin O-Acetyltransferase MetX (EC 2.3.1.31),
18.) Homoserin O-Succinyltransferase MetA (EC 2.3.1.46),
19.) Cystathionin gamma-Synthase MetB (EC 2.5.1.48),
20.) β-C-S-Lyase AecD (EC 4.4.1.8, auch als beta-Lyase bezeichnet),
21.) Cystathionin beta-Lyase MetC (EC 4.4.1.8),
22.) B12-unabhängige Homocystein S-Methyltransferase MetE (EC 2.1.1.14),
23.) B12-abhängige Homocystein S-Methyltransferase MetH (EC 2.1.1.13),
24.) Methylentetrahydrofolat-Reduktase MetF (EC 1.5.1.20),
25.) L-Methionin-Exporter BrnFE aus *Corynebacterium glutamicum*,
26.) Valinexporter YgaZH aus *Escherichia coli* (b2682, b2683),
27.) putativer Transporter YjeH aus *Escherichia coli* (b4141),
28.) Pyridinnucleotid-Transhydrogenase PntAB (EC 1.6.1.2),
29.) O-Succinylhomoserin-Sulfhydrylase MetZ (EC 2.5.1.48),
30.) Phosphoenolpyruvat-Carboxylase Pyc (EC 4.1.1.31),
31.) Thiosulfat-Sulfurtransferase RDL2p (EC 2.8.1.1),
32.) Thiosulfat-Thiol-Sulfurtransferase (EC 2.8.1.3),
33.) Thiosulfat-Dithiol-Sulfurtransferase (EC 2.8.1.5).

8. Zelle nach einem der Ansprüche 1 bis 7, wobei die Zelle ihrem Wildtyp gegenüber derartig gentechnisch verändert ist, dass sie relativ zu ihrem Wildtyp wenigstens eine Nukleinsäuresequenz oder eine Variante davon in einem verringerten Ausmaß exprimiert, die für eines der folgenden Enzyme, Komponenten davon oder für Varianten der Enzyme oder Komponenten davon kodiert:
1.) Transkriptionsregulator der L-Methioninbiosynthese (MetJ) (b3938, ECK3930,
2.) Glucose-6-Phosphat-Isomerase (Pgi, EC Nr. 5.3.1.9) (b4025, ECK4017),
3.) Homoserinkinase (ThrB, EC 2.7.1.39) (b0003, ECK0003),
4.) S-Adenosylmethionin-Synthase (MetK, EC Nr. 2.5.1.6) (b2942, ECK2937),
5.) Dihydrodipicolinat-Synthase (DapA, EC Nr. 4.2.1.52) (b2478, ECK2474),
6.) Phosphoenolpyruvat-Carboxykinase (Pck, EC Nr. 4.1.1.49) (b3403, ECK3390),
7.) Formyltetrahydrofolat-Hydrolase (PurU, EC Nr. 3.5.1.10) (b1232, ECK1227),
8.) Pyruvatkinase II (PykA, EC Nr. 2.7.1.40) (b1854, ECK1855),
9.) Pyruvatkinase I (PykF, EC 2.7.1.40) (b1676, ECK1672),
10.) Untereinheit des L-Methionintransporters (MetQNI) (b0197, ECK0197),
11.) Untereinheit des L-Methionintransporters (MetQNI) (b0198, ECK0198),
12.) Untereinheit des L-Methionintransporters (MetQNI) (b0199, ECK0199),
13.) Deoxycytidin 5'-Triphosphat Deaminase (Dcd, EC Nr. 3.5.4.13) (b2065, ECK2059),
14.) putative N-Acyltransferase (YncA),
15.) regulatorische sRNA FnrS,
16.) Sigma-Faktor RpoS.

9. Zelle nach einem der Ansprüche 1 bis 6, wobei die Zelle ihrem Wildtyp gegenüber derartig gentechnisch verändert ist, dass sie relativ zu ihrem Wildtyp wenigstens eine Nukleinsäuresequenz oder eine Variante davon überexprimiert, die für eines der folgenden Enzyme, Komponenten davon oder für Varianten der Enzyme oder Komponenten davon kodiert:
1.) Anthranilat-Synthase (trpDE, EC Nr. 4.1.3.27), Anthranilat-Phosphoribosyltransferase (trpD, EC Nr. 2.4.2.18), Phosphoribosylanthranilat-Isomerase (trpC, EC Nr. 5.3.1.24), Indol-3-Glycerinphosphat-Synthase (trpC, EC Nr. 4.1.1.48) und die Tryptophan-Synthase (trpAB, EC Nr. 4.1.2.8 und 4.2.1.122),
2.) Phosphoglycerat-Dehydrogenase SerA (EC 1.1.1.95),
3.) 3-Phosphoserin-Phosphatase SerB (EC 3.1.3.3),
4.) 3-Phosphoserin/Phosphohydroxythreonin-Aminotransferase SerC (EC 2.6.1.52),
5.) L-Tyrosin-sensitive DHAP-Synthase (aroF, EC 2.5.1.54),
6.) L-Phenylalanin-feedbackresistente DHAP-Synthase (aroG, EC 2.5.1.54),
7.) L-Tryptophan-sensitive DHAP-Synthase (aroH, EC 2.5.1.54),
8.) Phosphoenolpyruvat-Synthase ppsA (EC 2.7.9.2),
9.) Phosphoenolpyruvat-Carboxykinase pck (EC 4.1.1.49),
10.) Transketolase A tktA (EC 2.2.1.1),
11.) Transketolase B tktB (EC 2.2.1.1),
12.) das Genprodukt des offenen Leserahmens (ORF) yddG von *E. coli.*

10. Zelle nach einem der Ansprüche 1 bis 6, wobei die Zelle ihrem Wildtyp gegenüber derartig gentechnisch verändert ist, dass sie relativ zu ihrem Wildtyp wenigstens eine Nukleinsäuresequenz oder eine Variante davon in einem verringerten Ausmaß exprimiert, die für eines der folgenden Enzyme, Komponenten davon oder für Varianten der Enzyme oder Komponenten davon kodiert:
1.) Tryptophanase (tnaA, EC 4.1.99.1),
2.) Repressor des trp-Operons (trpR),
3.) Chorismat-Mutase T oder Prephenat-Dehydrogenase (tyrA, EC 1.3.1.12),
4.) Chorismat-Mutase P oder Prephenat-Dehydrogenase (pheA, EC 4.2.1.51),
5.) Tryptophan-spezifisches Transportprotein (mtr),
6.) Tryptophan-Permease (tnaB),
7.) Transporter für aromatische Aminosäuren (aroP),
8.) L-Serin-Deaminase (sdaA, EC 4.3.1.17),
9.) Glukose-6-Phosphat-Isomerase (pgi, EC 5.3.1.9),
10.) Tyrosin-Aminotransferase (tyrB),
11.) Repressor des glp-Regulons (glpR);
12.) Sigma-Faktor RpoS (rpoS).

11. Zelle nach einem der Ansprüche 1 bis 10, wobei es sich bei der Zelle um eine Zelle zur Überproduktion einer essentielle Aminosäure, noch bevorzugter einer von Serin abgeleitete essentiellen Aminosäure, am bevorzugtesten Methionin oder Tryptophan, handelt.

12. Futtermitteladditiv umfassend eine Zelle nach Anspruch 1 bis 11.

13. Verfahren zur Herstellung einer eine essentielle Aminosäure, bevorzugter eine von Serin abgeleitete essentielle Aminosäure, am bevorzugtesten Methionin oder Tryptophan, überproduzierenden Zelle, umfassend Herstellen einer Zelle mit einem Knock out eines für eine ppGppase kodierenden Gens, bevorzugt ohne Verringerung der (p)ppGpp-Synthetase-Aktivtität relativ zu ihrem Wildtyp, wobei die ppGppase bevorzugt aus der Gruppe ausgewählt ist, die die Aminosäuresequenzen SEQ ID NO:2, SEQ ID NO:4 und SEQ ID NO:6 sowie Varianten davon umfasst, und es sich noch bevorzugter um SEQ ID NO:2 handelt, und Kultivieren der Zelle, optional Aufreinigen der Aminosäure

14. Verfahren zur Herstellung einer von Serin abgeleiteten essentiellen Aminosäure umfassend die Schritte
a.) Kultivieren der Zelle nach einem der Ansprüche 1 bis 11,
b.) optional: Aufreinigen der Aminosäure.

15. Verfahren nach Anspruch 14, wobei eine Zelle nach einem der Ansprüche 1 bis 11 verwendet wird und das Verfahren ein Verfahren zur Herstellung von Methionin ist.

16. Verfahren nach Anspruch 14, wobei eine Zelle nach einem der Ansprüche 1 bis 7 oder 10 bis 11 verwendet wird und das Verfahren ein Verfahren zu Herstellung einer aromatischen Aminosäure, bevorzugt Tryptophan, ist.

17. Verfahren nach einem der Ansprüche 13 bis 16, wobei die Mikroorganismen in einem batch-Verfahren, einem fed batch-Verfahren, einem repeated fed batch-Verfahren oder einem kontinuierlichen Verfahren kultiviert werden.

18. Zelle/Futtermitteladditiv nach einem der Ansprüche 1 bis 12 oder Verfahren nach einem der Ansprüche 13 bis 17, wobei es sich bei der Zelle um eine bakterielle Zelle aus der Gattung der *Enterobacteriaceae* handelt.

19. Zelle/Futtermitteladditiv nach einem der Ansprüche 1 bis 12 oder Verfahren nach einem der Ansprüche 13 bis 18, wobei die Zelle aus der Gruppe von Gattungen ausgewählt ist, die *Escherichia*, *Erwinia*, *Providencia* und *Serratia* umfasst, und es sich bei der Zelle bevorzugt um eine Zelle aus der Gattung *Escherichia*, noch bevorzugter um *E. coli* handelt.
